(19) Europäisches Patentamt
European Patent Office
Office européen des brevets

(11) Veröffentlichungsnummer : **0 366 040 B1**

(12) # EUROPÄISCHE PATENTSCHRIFT

(45) Veröffentlichungstag der Patentschrift :
**17.03.93 Patentblatt 93/11**

(51) Int. Cl.$^5$ : **C07C 69/732,** C07C 235/34, C08K 5/00, C09K 15/04, C10M 129/76, C10M 133/16

(21) Anmeldenummer : **89119641.2**

(22) Anmeldetag : **23.10.89**

(54) **Phenolgruppenhaltige Verbindungen als Antioxidantien in organischen Materialien.**

Die Akte enthält technische Angaben, die nach dem Eingang der Anmeldung eingereicht wurden und die nicht in dieser Patentschrift enthalten sind.

(30) Priorität : **25.10.88 CH 3959/88**

(43) Veröffentlichungstag der Anmeldung :
**02.05.90 Patentblatt 90/18**

(45) Bekanntmachung des Hinweises auf die Patenterteilung :
**17.03.93 Patentblatt 93/11**

(84) Benannte Vertragsstaaten :
**BE DE ES FR GB IT NL**

(56) Entgegenhaltungen :
**US-A- 3 644 482**
**US-A- 4 529 809**

(73) Patentinhaber : **CIBA-GEIGY AG**
**Klybeckstrasse 141**
**CH-4002 Basel (CH)**

(72) Erfinder : **Evans, Samuel, Dr.**
**Route des Charbonnières 17**
**CH-1723 Marly (CH)**

(74) Vertreter : **Zumstein, Fritz, Dr. et al**
**Bräuhausstrasse 4**
**W-8000 München 2 (DE)**

EP 0 366 040 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Zusammensetzungen, enthaltend dem oxidativen, thermischen oder actinischen Abbau unterworfene organische Materialien und Hydroxyphenylcarbonsäureester-Verbindungen, neue Hydroxyphenylcarbonsäure-Verbindungen und deren Verwendung als Antioxidantien.

Die Stabilisierung, beispielsweise von Schmierstoffen oder von Kunststoffen mit Antioxidantien aus der Reihe der Hydroxyphenylcarbonsäureester, ist bekannt aus US-A 3,247,240; US-A 3,644,482; US-A 3,962,123; US-A 3,984,460 und US-A 3,494,887.

Aus der DE-A 28 37 141 ist eine Schmierölformulierung bekannt geworden, bestehend aus einem Erdöldestillat, kleinen Anteilen an anderen Stoffen und einem Oxidationsschutzstoff, wobei der Oxidationsschutzstoff den $\alpha,\omega$-Alkandiol-bis-ester einer 2,4,6-Trialkylphenolcarbonsäure enthält.

In der US-A 4,529,809 ist ein Verfahren zur Herstellung von Hydroxyphenylcarbonsäureestern beschrieben, wobei Methyl-, Ethyl und Propylester der 2-Methyl-3-(3′,5′-di-t-butyl-4′-hydroxyphenyl)propionsäure als wichtige Zwischenprodukte für die Herstellung von Antioxidantien für Polypropylenharze des Typs Pentaerythrit-tetrakis[2-methyl-3-(3′,5′-di-t-butyl-4′-hydroxyphenyl)propionat dienen.

Es wurde nun überraschend eine Reihe von Verbindungen gefunden, die als Antioxidantien in organischen Materialien, insbesondere in funktionellen Flüssigkeiten und Kunststoffen, verbesserte Eigenschaften aufweisen.

Die vorliegende Erfindung betrifft Zusammensetzungen enthaltend

a) mindestens ein dem oxidativen, thermischen oder actinischen Abbau unterworfenes organisches Material und

b) mindestens eine Verbindung der allgemeinen Formel I

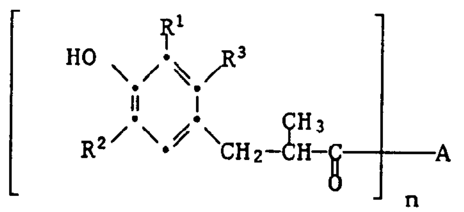

(I),

wobei

$R^1$ = Alkyl mit 1 bis 18 C-Atomen, Cycloalkyl mit 5 bis 12 C-Atomen, Phenyl oder Aralkyl mit 7 byl mit 1 bis 18 C-Atomen, Cycloalkyl mit 5 bis 12 C Phenyl oder Aralkyl mit 7 bis 9 C-Atomen und

$R^3$ = -H oder $CH_3$ bedeutet und

n eine Zahl von 1 bis 4 oder 6 bedeutet, wobei,

wenn n = 1 ist,

A = $-OR^4$ oder

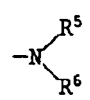

ist, und

$R^4$ die Bedeutung von -H, Alkyl mit 1 bis 45 C-Atomen, Cycloalkyl mit 5 bis 12 C-Atomen, Alkenyl mit 2 bis 18 C-Atomen,

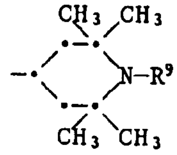

oder $-CH_2CH_2-XR^{5a}$ hat, und

$R^5$ und $R^6$, unabhängig voneinander, -H, Alkyl mit 1 bis 20 C-Atomen, Phenyl, Cycloalkyl mit 5 bis 12 C-Atomen, $C_1$-$C_4$ alkylsubstituiertes Cycloalkyl mit 5 bis 12 C-Atomen, Alkenyl mit 3 bis 8 C-Atomen, Aralkyl

mit 7 bis 10 C-Atomen oder

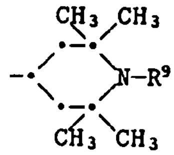

bedeuten, und zusätzlich

$R^5$ $-NH_2$ bedeutet,

$R^9$ = -H, Alkyl mit 1 bis 8 C-Atomen,

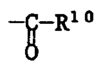,

-O· oder $-OR^{9'}$ ist, wobei

$R^{9'}$ = -H, Alkyl mit 1-25 C-Atomen oder

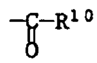

darstellt,

X = -O-, -S- oder

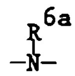

ist,

$R^{5a}$ =

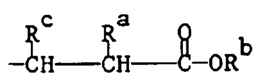, 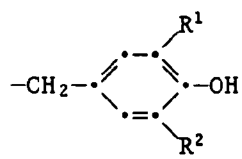,

-H, Alkyl mit 1-24 C-Atomen, Phenyl, Cycloalkyl mit 5 bis 12 C-Atomen oder

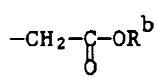

ist,

und

$R^{10}$ = Alkyl mit 1 bis 20 C-Atomen ist,

$R^a$ = -H oder $-CH_3$,

$R^b$ = -H oder Alkyl mit 1 bis 24 C-Atomen und

$R^c$ = -H oder $-CH_3$ sind, mit der Massgabe, dass $R^a$ und $R^c$ nicht gleichzeitig $-CH_3$ sind,

und

$R^{6a}$ = Alkyl mit 1 bis 18 C-Atomen, Phenyl, mit einer oder mehreren Alkylgruppen mit insgesamt 1 bis 24 C-Atomen substituiertes Phenyl oder $C_5$-$C_8$-Cycloalkyl ist, oder,

wenn n = 2 ist,

A =

$-O-C_xH_{2x}-O-$,

$-O-(CH_2CH_2O)_aCH_2CH_2O-$,

$-O-CH_2-CH_2-B-CH_2-CH_2-O-$,

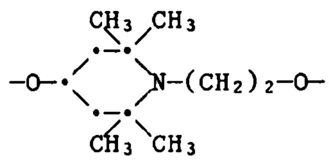 , 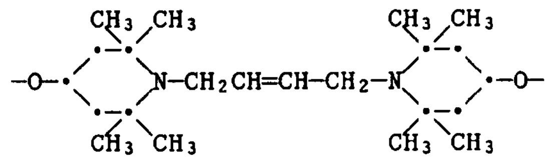 ,

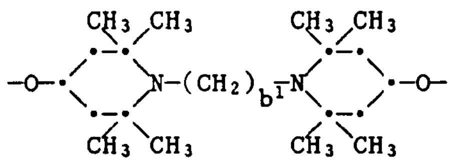 ,

$-O-CH_2CH=CHCH_2-O-$ , $-O-CH_2C\equiv CCH_2-O-$ , 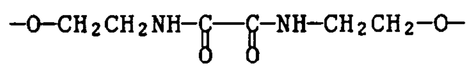 ,

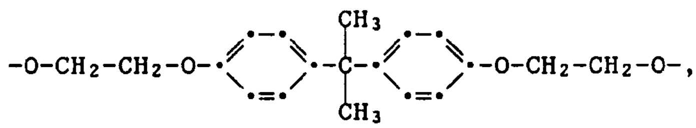

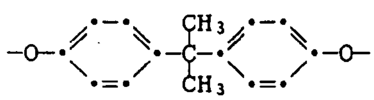 , 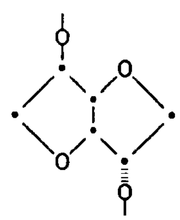 oder 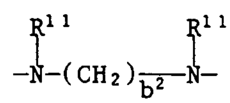

ist,

wobei

a eine Zahl von 1 bis 30 ist,

x eine Zahl von 2 bis 20 ist,

B = -S-,

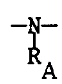 oder 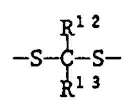

ist,

$R_A$ = Alkyl mit 1 bis 20 C-Atomen, Phenyl oder mit einer oder mehreren Alkylgruppen mit insgesamt 1 bis 20 C-Atomen substituiertes Phenyl, Cyclohexyl oder

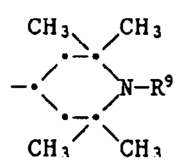

ist, und

$R^{11}$, $R^{12}$ und $R^{13}$, unabhängig voneinander, -H, Alkyl mit 1 bis 12 C-Atomen oder Phenyl sind oder

$R^{12}$ und $R^{13}$, mit dem C-Atom, an das sie gebunden sind, einen Cycloalkylring mit 5 bis 12 C-Atomen bilden,

$b^1$ eine Zahl von 2 bis 10 ist, und

$b^2$ eine Zahl von 0 bis 6 darstellt,

oder wenn n = 3 ist,

A =

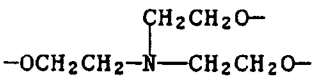 oder 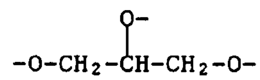

bedeutet,

oder wenn n = 4 ist

A =

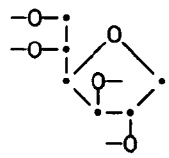 oder 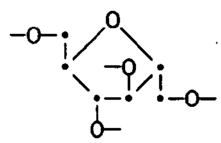

bedeutet,

oder wenn n = 6 ist,

A =

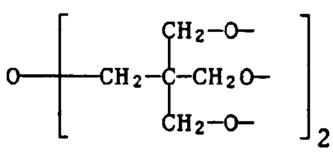

bedeutet.

Stellen $R^1$, $R^2$, und $R^{6a}$ beispielsweise Alkyl mit 1 bis 18 C-Atomen dar, so kann die Alkylgruppe geradkettig oder verzweigt sein und kann beispielsweise bedeuten: Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl oder 1-Methylundecyl. Bevorzugt sind Alkyl mit 1-12 C-Atomen, und besonders bevorzugt Alkyl mit 1-8 C-Atomen.

Für $R^1$ und $R^2$ sind die Alkylgruppen aus obiger beispielhafter Aufzählung mit 1 bis 8 C-Atomen als zweckmässig und mit 1 bis 4 C-Atomen als bevorzugt anzusehen. Ganz besonders bevorzugt für $R^1$ und $R^2$ wird die t-Butylgruppe.

$R^4$ stellt beispielsweise geradkettiges oder verzweigtes Alkyl mit 1 bis 45 C-Atomen dar. Beispiele dafür sind jene, die für $R^1$ und $R^2$ angegeben sind, zuzüglich Eicosyl, Hemicosyl, Docosyl, Triacontyl usw. Bevorzugt ist $R^4$ als Alkyl, $C_1$-$C_{20}$-, insbesondere $C_1$-$C_{18}$-Alkyl.

$R_A$, $R^5$, $R^6$ und $R^{10}$ können Alkyl mit 1 bis 20 C-Atomen bedeuten, wobei geradkettige oder verzweigte Alkylgruppen gemeint sind und auf die obengenannte Liste von Beispielen für $R^1$ und $R^2$, ergänzt durch das weitere Beispiel Eicosyl, hingewiesen wird. Bevorzugt werden Alkylgruppen mit 1 bis 12 C-Atomen.

$R^9$ oder $R^{9\prime}$ bedeuten beispielsweise Alkyl mit 1 bis 8 C-Atomen, wobei die Alkylgruppen geradkettig oder verzweigt sein können und Methyl, Ethyl, Propyl, Isopropyl, n-Butyl, Isobutyl, tert.-Butyl, Pentyl, Isopentyl, Hexyl, Heptyl, Octyl, 2-Ethylhexyl, 2, Ethylbutyl, Isoamyl, 1-Methylpentyl, 1,3-Dimethylbutyl, 1,1,3,3-Tetramethylbutyl, 1-Methylhexyl, Isoheptyl oder 1-Methylheptyl darstellen.

Ein zweckmässiger Rest für $R^9$ ist Alkyl mit 1 bis 4 C-Atomen, demnach beispielsweise Methyl, Ethyl, Propyl, Isopropyl, n-Butyl, Isobutyl oder tert.-Butyl.

Stellt $R^b$ oder $R^{5a}$ eine Alkylgruppe mit 1 bis 24 C-Atomen dar, so zählen dazu die geradkettigen oder verzweigten Alkylgruppen wie sie beispielsweise für $R^1$ genannt wurden, ergänzt beispielhaft durch Eicosyl oder Docosyl. Bevorzugt sind Alkylgruppen mit 1 bis 18 C-Atomen.

$R^{11}$, $R^{12}$ und $R^{13}$ stellen beispielsweise unabhängig voneinander Alkyl mit 1 bis 12 C-Atomen dar, wobei die Alkylgruppen geradkettig oder verzweigt sein können und in beispielhafter Erwähnung Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl oder 1-Methylundecyl sein können. Bevorzugt sind die entsprechenden Beispiele mit 1 bis 8 C-Atomen.

$R^{6a}$ sowie $R^5$ und $R^6$, unabhängig voneinander, können zweckmässig Alkyl mit 1 bis 12 C-Atomen sein. Die Alkylgruppe kann geradkettig oder verzweigt sein und Beispiele sind entsprechender obiger Aufzählung für $R^{11}$ und $R^{12}$ zu entnehmen.

In vorzugsweiser Form kann $R_A$ eine $C_4$-$C_8$-Alkylgruppe sein und Beispiele für eine solche Gruppe sind n-Butyl, Isobutyl, tert.-Butyl, Pentyl, Isopentyl, Hexyl, Heptyl, Octyl, Isooctyl, 2-Ethylhexyl, 2-Ethylbutyl, 1-Methylpentyl, 1,3-Dimethylbutyl, 1,1,3,3-Tetramethylbutyl, 1-Methylhexyl, Isoheptyl oder 1-Methylheptyl.

Soweit i-$C_8H_{17}$ genannt wird, kann darunter auch eine Mischung von Isomeren verstanden werden.

Bedeuten $R^1$, $R^2$, $R^4$, $R^5$, $R^6$ oder $R^{5a}$ Cycloalkyl mit 5 bis 12 C-Atomen, so zählen beispielsweise dazu Cyclopentyl, Cyclohexyl, Cycloheptyl, Cyclooctyl, Cyclodecyl und Cyclododecyl. Bevorzugt wird Cyclohexyl.

$R^{6a}$ kann als Cycloalkyl mit 5 bis 8 C-Atomen die Bedeutung von Cyclopentyl, Cyclohexyl, Cycloheptyl oder Cyclooctyl haben.

Darüber hinaus stellt $R^5$ und $R^6$ auch $C_1$- bis $C_4$-alkylsubstituiertes Cycloalkyl mit 5 bis 12 C-Atomen dar. Bevorzugt ist Cycloalkyl mit 5 bis 12 C-Atomen, das mit einer, zwei oder drei $C_1$-$C_4$-Alkylgruppen substituiert ist. Beispiele dafür sind 2- oder 4-Methylcyclohexyl, Dimethylcyclohexyl, Trimethylcyclohexyl oder t-Butylcyclohexyl.

Bedeutet $R^{6a}$ ein mit einer oder mehreren, vorzugsweise mit einer, zwei oder drei, Alkylgruppen mit insgesamt 1 bis 24 C-Atomen substituiertes Phenyl oder $R_A$ ein mit einer oder mehreren, vorzugsweise mit einer, zwei oder drei, Alkylgruppen mit insgesamt 1 bis 20 C-Atomen substituiertes Phenyl, so gehören zu den Beispielen, die den Substituenten $R^{6a}$ und $R_A$ zugeordnet werden können, Methylphenyl, Dimethylphenyl, Trimethylphenyl, Ethylphenyl, Diethylphenyl, Isopropylphenyl, t-Butylphenyl, Di-t-butylphenyl, Methyl-di-t-butylphenyl, tert.-Octylphenyl und Di-tert.-octylphenyl.

Schliesslich können $R^{12}$ und $R^{13}$ mit dem C-Atom an das sie gebunden sind, einen Cycloalkylring mit 5 bis 12 C-Atomen bilden. Dabei kann es sich in beispielhafter Nennung um einen Cyclopentyl-, Cyclohexyl-, Cycloheptyl-, Cyclooctyl-, Cyclodecyl- oder Cyclododecylring handeln. Bevorzugt ist ein Cyclohexylring.

Sind $R^1$, $R^2$, $R^5$ oder $R^6$ mit Aralkyl mit 7 bis 9 C-Atomen oder 7 bis 10 C-Atomen bezeichnet, so sind Beispiele dafür Benzyl, Phenethyl, $\alpha$-Methylbenzyl oder $\alpha,\alpha$-Dimethylbenzyl. Bevorzugt ist Benzyl.

Ist $R^4$ eine Alkenylgruppe mit 2 bis 18 C-Atomen, so sind Beispiele dafür Vinyl, Propenyl, Allyl, Butenyl, Methallyl, Hexenyl, Decenyl oder Heptadecenyl.

Der Rest

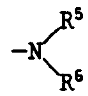

kann auch ein Hydrazinrest der Formel -NH-$NH_2$ darstellen, wobei auch der Hydrazinhydratrest mitumfasst sein kann.

Für $R^5$ oder $R^6$ als Alkenyl mit 3 bis 8 C-Atomen kommen beispielsweise Allyl, 2-Methallyl, 2-Butenyl oder 2-Hexenyl in Betracht.

Bevorzugt sind Verbindungen der Formel I, worin n 1 oder 2 ist.

Zweckmässige Zusammensetzungen nach der Erfindung enthalten mindestens eine Verbindung der Formel I, wobei

$R^1$ = Alkyl mit 1 bis 18 C-Atomen, Cycloalkyl mit 5 bis 12 C-Atomen, Phenyl oder Benzyl,

$R^2$ = -H, Alkyl mit 1 bis 18 C-Atomen, Cycloalkyl mit 5 bis 12 C-Atomen, Phenyl oder Benzyl und

$R^3$ = -H bedeuten und

n eine Zahl von 1 bis 4 oder 6 bedeutet, wobei,

wenn n = 1 ist,

A = -$OR^4$ oder

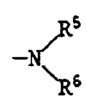

ist, und

$R^4$ die Bedeutung von -H, Alkyl mit 1 bis 20 C-Atomen, Cyclohexyl, Alkenyl mit 2 bis 18 C-Atomen oder -$CH_2CH_2$-$XR^{5a}$ hat, und

$R^5$ -H, Alkyl mit 1 bis 12 C-Atomen, Phenyl, Cyclohexyl, $C_1$-$C_4$ alkylsubstituiertes Cycloalkyl mit 5 bis 12 C-Atomen, Alkenyl mit 3 bis 8 C-Atomen, Benzyl oder -$NH_2$ bedeutet, und

$R^6$ -H oder Alkyl mit 1 bis 12 C-Atomen bedeutet,

X = -O-, -S- oder

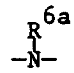

ist,

$R^{5a}$ = -H, Alkyl mit 1-12 C-Atomen, Phenyl, Cyclohexyl oder

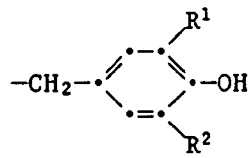

ist,

und

$R^{6a}$ = Alkyl mit 1 bis 12 C-Atomen, Phenyl oder mit einer oder mehreren Alkylgruppen mit insgesamt 1 bis 18 C-Atomen substituiertes Phenyl ist, oder,

wenn n = 2 ist,

A =

$$-O-C_xH_{2x}-O-,$$
$$-O-(CH_2CH_2O)_aCH_2CH_2O-,$$
$$-O-CH_2-CH_2-B-CH_2-CH_2-O-,$$

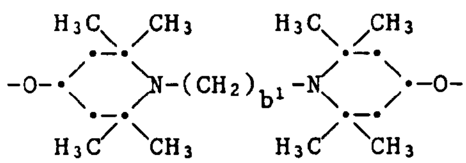

$-O-CH_2CH=CHCH_2-O-$ , 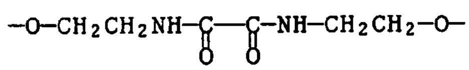 ,

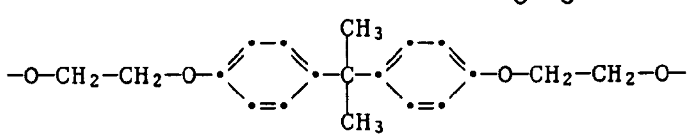 ,

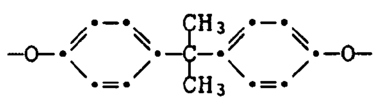 , 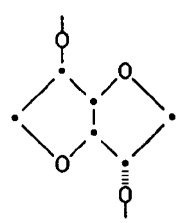 oder 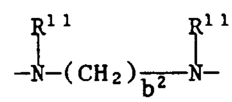

ist,

wobei

a eine Zahl von 1 bis 12 ist,

x eine Zahl von 2 bis 12 ist,

B = -S- oder

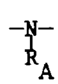

ist,

$R_A$ = Alkyl mit 1 bis 12 C-Atomen, Phenyl oder mit einer oder mehreren Alkylgruppen mit insgesamt 1 bis 18 C-Atomen substituiertes Phenyl, oder Cyclohexyl ist, und

$R^{11}$, -H, Alkyl mit 1 bis 12 C-Atomen oder Phenyl ist und

$b^1$ eine Zahl von 2 bis 6 ist, und
$b^2$ eine Zahl von 0 bis 6 darstellt,
oder, wenn n = 3 ist,
A =

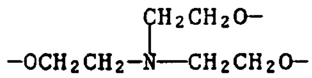 oder 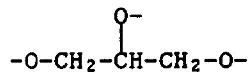

bedeutet,
oder, wenn n = 4 ist
A =

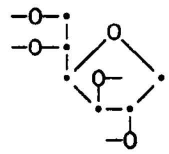 oder 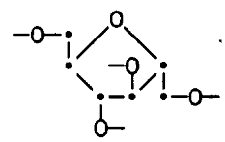

bedeutet,
oder, wenn n = 6 ist,
A =

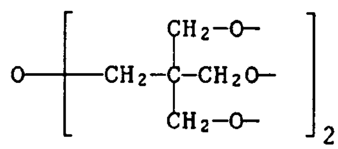

bedeutet.

Besonders zweckmässige Zusammensetzungen enthalten mindestens eine Verbindung der Formel I, wobei

$R^1$ = Alkyl mit 1 bis 8 C-Atomen, Cyclohexyl oder Phenyl,
$R^2$ = -H, Alkyl mit 1 bis 8 C-Atomen, Cyclohexyl oder Phenyl und
$R^3$ = -H darstellen und
n = 1 ist und
A = $-OR^4$ oder

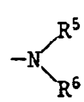

darstellt, wobei
$R^4$ = -H, Alkyl mit 1 bis 18 C-Atomen, Cyclohexyl, $-CH_2CH=CH_2$, $-(CH_2)_7-CH=CH-(CH_2)_7CH_3$,

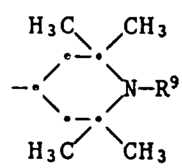

oder $-CH_2CH_2XR^{5a}$ bedeutet, wobei
$R^9$ = -H, Alkyl mit 1 bis 4 C-Atomen, -O·, -O-Alkyl mit 1 bis 4 C-Atomen oder Cyclohexyl ist,
X = -O-, -S- oder

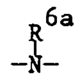

ist,

$R^{5a}$ = -H, Alkyl mit 1 bis 18 C-Atomen, Phenyl,

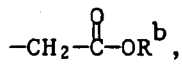

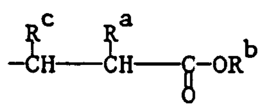

oder

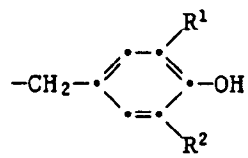

ist, wobei

$R^b$ = Alkyl mit 1 bis 24 C-Atomen ist,
$R^a$ = -H oder $-CH_3$ ist,
$R^c$ = -H ist und
$R^{6a}$ = Alkyl mit 1 bis 12 C-Atomen oder Phenyl ist, und
$R^5$ und $R^6$, unabhängig voneinander, -H, Alkyl mit 1 bis 12 C-Atomen, Phenyl oder

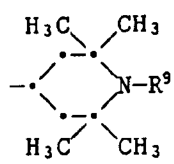

sind,

wobei $R^9$ die oben angegebene Bedeutung hat, oder
$R^5$ $-NH_2$ und $R^6$ -H bedeuten,
oder,
n gleich 2 ist, wobei
A = $-O-C_xH_{2x}-O-$ bedeutet und
x = 2 bis 8 ist.

Bevorzugte Zusammensetzungen enthalten mindestens eine Verbindung der Formel I, wobei

$R^1$ = tert.-Butyl
$R^2$ = -H, Methyl oder tert.-Butyl und
$R^3$ = -H darstellen und
n gleich 1 ist, wobei
A = $-OR^4$ oder

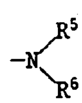

darstellt, und

$R^4$ = Alkyl mit 1 bis 18 C-Atomen, $-(CH_2)_7-CH=CH-(CH_2)_7CH_3$,

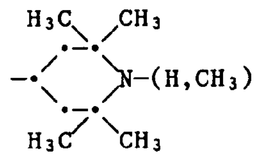

oder

$-CH_2CH_2-SR^{5a}$ ist, wobei

$R^{5a}$ = -H oder

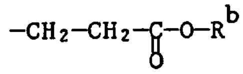

bedeutet, wobei

$R^b$ = n-$C_4H_9$ bis n-$C_8H_{17}$ oder tert.-$C_4H_9$ bis tert.-$C_8H_{17}$ ist,

oder

n gleich 2 ist, wobei

A = $-O-C_xH_{2x}-O-$ und

x = 2 bis 8 ist, oder

A = $-O-(CH_2-CH_2-O-)_a-CH_2-CH_2-O-$ und

a = 1 bis 4 ist, oder

A = $-O-CH_2-CH_2-B-CH_2-CH_2-O-$ und

B = -S-,

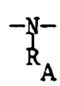 oder 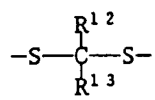

ist, wobei

$R_A$ = $C_4$-$C_8$-Alkyl oder Phenyl,

$R^{12}$ = H, $C_1$-$C_8$-Alkyl und

$R^{13}$ = H, $C_1$-$C_8$-Alkyl oder Phenyl darstellen, oder

A =

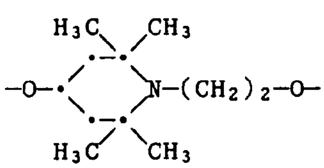 , 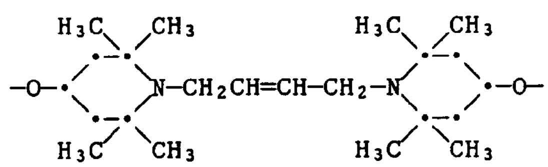 oder

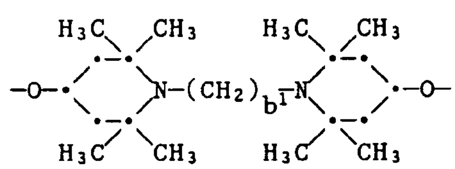 ist, wobei

$b^1$ = 2 bis 6 ist, oder

A =

$-O-CH_2-CH=CH-CH_2-O-$,

$-O-CH_2-C\equiv C-CH_2-O-$,

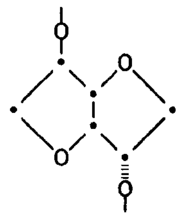 , 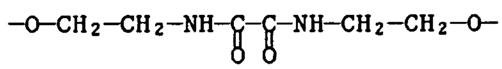 oder 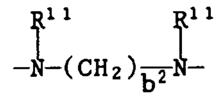

ist,

wobei

$b^2$ = 0 bis 6 und

$R^{11}$ = -H oder $C_1$-$C_8$-Alkyl darstellen.

Weitere bevorzugte Zusammensetzungen enthalten mindestens eine Verbindung der Formel I, wobei

$R^1$ = tert.-Butyl

$R^2$ = -H, $-CH_3$ oder tert.-Butyl und

$R^3$ = -H darstellen und

n = 1 ist und dabei

A = $-OR^4$ bedeutet und

$R^4$ = $C_1$-$C_{18}$-Alkyl ist.

Zu den weiteren bevorzugten Zusammensetzungen zählen solche, enthaltend mindestens eine Verbindung der Formel I, wobei

$R^1$ = tert.-Butyl

$R^2$ = -H, $-CH_3$ oder tert.-Butyl und

$R^3$ = -H darstellen und

n = 2 ist und dabei

A = $-O-C_xH_{2x}-O-$ und

x = 2 bis 6 ist, oder

A = $-O-(CH_2-CH_2-O)_a-CH_2-CH_2-O-$ und

a = 1, 2 oder 3 ist,

oder

A = $-O-CH_2-CH_2-B-CH_2-CH_2-O-$ und

B = -S- oder

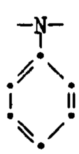

ist,

oder

A =

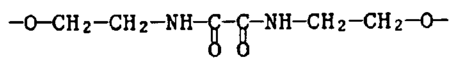

oder

A = -NH-NH- ist.

Auch bevorzugt ist eine Zusammensetzung enthaltend mindestens eine Verbindung der Formel I, wobei

$R^1$ = tert.-Butyl,

$R^2$ = -H, $-CH_3$ oder tert.-Butyl

$R^3$ = -H darstellen,

n = 1 ist und dabei

A = $-OR^4$ darstellt, wobei

$R^4$ = $C_1$-$C_{18}$-Alkyl ist, oder

n = 2 ist und dabei

A = $-O-C_xH_{2x}-O-$ und x = 2-6,

$-OCH_2CH_2-S-CH_2CH_2O-$ oder

$-O(CH_2CH_2O)_aCH_2CH_2-O-$ und

a = 1 bis 4 sind.

Besonders bevorzugt ist eine Zusammensetzung oben angegebener Art enthaltend mindestens eine Verbindung der Formel I, wobei a = 1 oder 2 ist.

Ebenfalls bevorzugt ist eine Zusammensetzung enthaltend mindestens eine Verbindung der Formel I, wobei

$R^1$ = tert.-Butyl

$R^2$ = -H, $-CH_3$, tert.-Butyl und

$R^3$ = -H darstellen und

n gleich 3 ist und dabei

A =

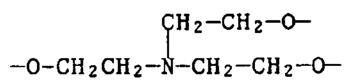

darstellt,

oder

n = 4 ist und dabei

A =

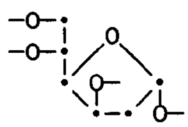

darstellt,

oder

n = 6 ist und dabei

A =

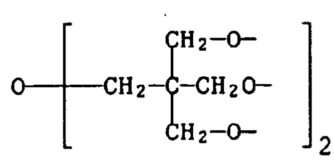

bedeutet.

Ganz besonders bevorzugt sind Zusammensetzungen, enthaltend mindestens eine Verbindung aus der Reihe der Formeln

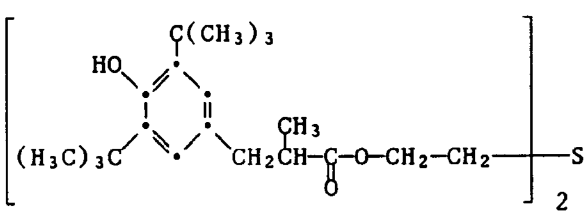

,

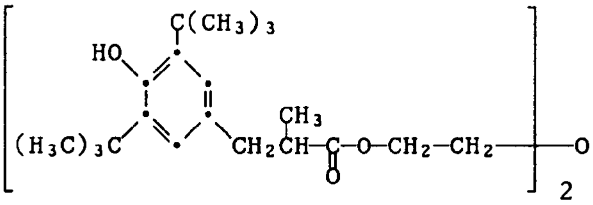

,

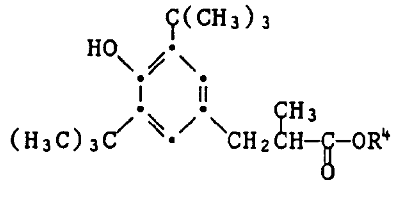

,

wobei

$R^4$ die Bedeutung von $C_1$-$C_{18}$-Alkyl und insbesondere von $-CH_3$, n-$C_4$-$C_9$, -i-$C_8H_{17}$, -n-$C_{12}H_{25}$ oder -n-$C_{18}H_{37}$ hat,

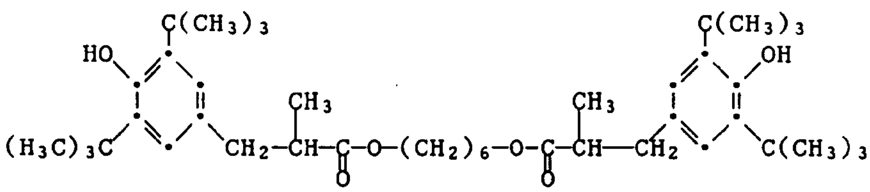

und

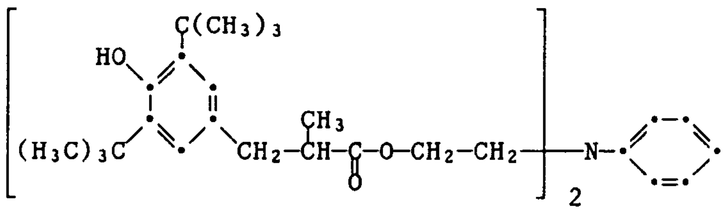

.

Die oben genannten Einzelverbindungen stellen in Schmierstoffen der Reihe der Mineralöle, der synthetischen Oele oder Mischungen davon ganz besonders wertvolle Zusammensetzungen dar.

Die Verbindungen der Formel I können beispielsweise durch eine Michael-Addition hergestellt werden, wobei die Reaktion der nachstehenden Gleichung folgt:

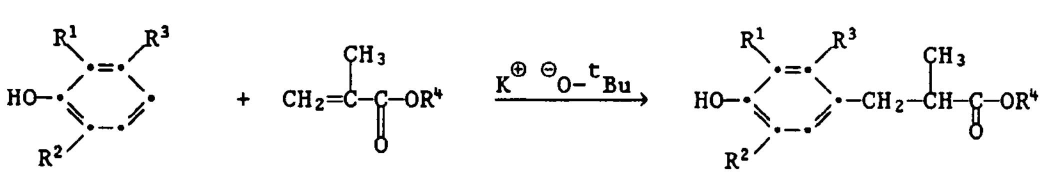


$R^1$, $R^2$, $R^3$ und $R^4$ können die obengenannte Bedeutung haben.

Das Verfahren wird derart ausgeführt, dass das alkylsubstituierte Phenol mit dem olefinischen Ester im stöchiometrischen Ueberschuss in Gegenwart einer katalytischen Menge einer Base, beispielsweise einer quaternären Ammoniumbase, eines Alkalimetallamides oder eines Alkalimetallalkoxides, bei einer Temperatur zwischen 150° und 220°C umgesetzt wird.

Eine detaillierte Verfahrensbeschreibung ist z.B. der eingangs erwähnten US-PS 4 529 809 zu entnehmen.

Ein weiteres Verfahren zur Herstellung erfindungsgemässer Verbindungen ist die Umesterung, die der allgemeinen Gleichung folgt:

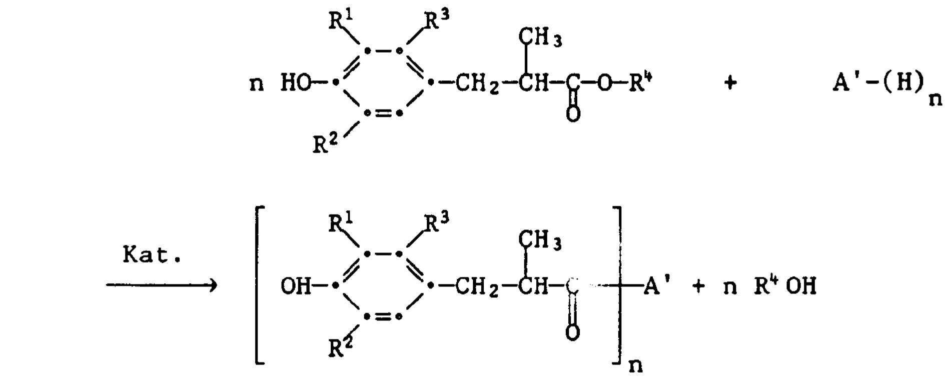


$R^1$, $R^2$ und $R^3$ haben obenstehende Bedeutung, während A′ die Bedeutung von A mit Ausnahme des Restes $-NR^5R^6$ hat. Zweckmässig setzt man Ausgangs-Ester mit $R^4$ = $C_1$-$C_4$-Alkyl, insbesondere Methyl, ein, die ebenfalls, wie oben beschrieben, durch Umsetzung eines entsprechenden Phenols mit einem Methacrylat der Formel

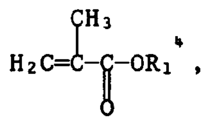

worin $R_1^4$ $C_1$-$C_4$-Alkyl ist, hergestellt werden können.

Geeignete Katalysatoren sind z.B. Dibutylzinnoxid, Ti(-O-i-$C_3H_7$)$_4$, $LiNH_2$, LiH, LiOH, KOR, NaOR, LiOR, wobei R = H oder $C_1$-$C_{10}$-Alkyl ist, oder

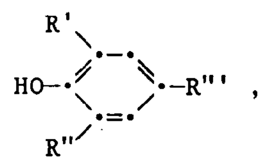

wobei R′, R″ und R‴ unabhängig voneinander H oder Alkyl mit 1 bis 10 C-Atomen sind.

Das Verfahren wird zweckmässig in einem Lösungsmittel, wie Benzol, Toluol, Xylol, Dimethylformamid usw. ausgeführt.

Ein weiteres Verfahren zur Herstellung von erfindungsgemässen Verbindungen folgt der allgemeinen Gleichung für die Veresterung:

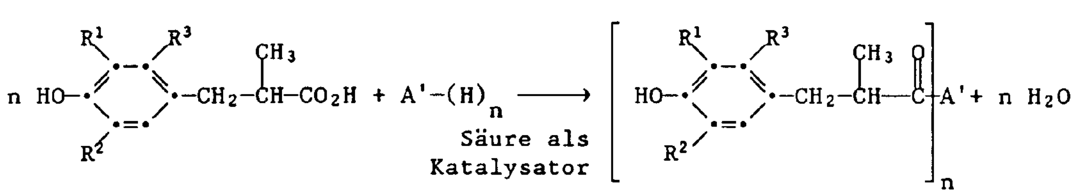


$R^1$, $R^2$, $R^3$ und A′ können dabei obengenannte Bedeutung haben. Die Reaktion wird in Gegenwart einer Säure als Katalysator durchgeführt. So kann als Katalysator beispielsweise eine Bleicherde, wie Tosil L80S, oder p-Toluolsulfonsäure zur Anwendung gelangen. Die Herstellung der Carbonsäure-Ausgangsverbindungen kann durch Verseifung von Verbindungen der Formel

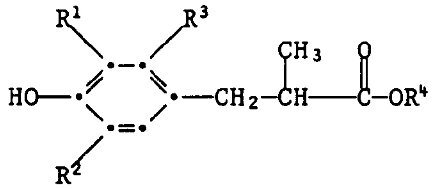

erfolgen.

Ein Verfahren, das ebenfalls geeignet ist, Verbindungen nach vorliegender Erfindung herzustellen, ist auch die Reaktion eines entsprechenden Säurechlorides mit einem Alkohol oder Amin. Das Verfahren kann mit nachfolgender Reaktionsgleichung näher umschrieben werden:

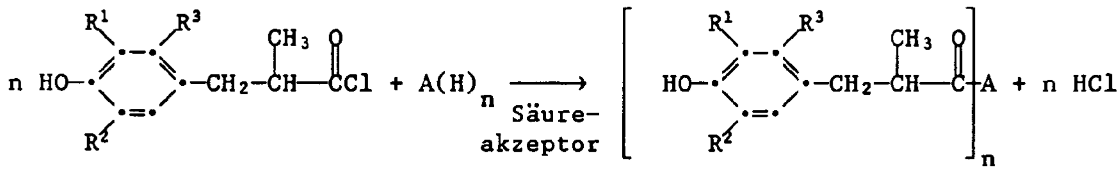


Die Bedeutung von $R^1$, $R^2$, $R^3$ und A ist oben angegeben. Als Säureakzeptor kann beispielsweise Pyridin, Triethylamin oder ein Alkalihydroxid, wie NaOH oder KOH eingesetzt werden.

Ein anderes Verfahren zur Herstellung von Verbindungen nach vorliegender Erfindung folgt der Gleichung:

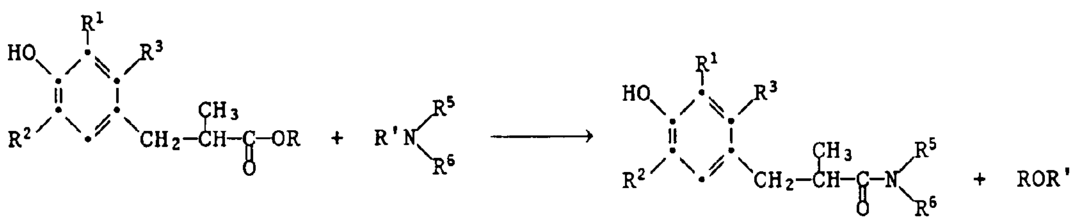

R und R′ haben z.B. unabhängig voneinander die Bedeutung von $R_1^4$ oder Wasserstoff. Dabei erfolgt die Umsetzung durch thermische Reaktion bei beispielsweise 80-190°C.

Die erfindungsgemässen Verbindungen der Formel I eignen sich hervorragend zum Stabilisieren von organischem Material, insbesondere solchem, das gegen oxidativen, thermischen oder actinischen Abbau empfindlich ist.

Die vorliegende Erfindung betrifft daher Zusammensetzungen enthaltend gegen oxidativen, thermischen oder actinischen Abbau empfindliche organische Materialien, und mindestens eine Verbindung der Formel I, sowie die Verwendung der Verbindungen der Formel I zum Stabilisieren von gegen oxidativen, thermischen oder actinischen Abbau empfindlichen organischen Materialien.

Zweckmässig enthalten die organischen Materialien 0,01-10, z.B. 0,05-5, vorzugsweise 0,05-3, insbesondere 0,1-2 Gew.-%, bezogen auf das organische Material, an Verbindungen der Formel I.

Ein weiterer Gegenstand der Erfindung sind Zusammensetzungen, die ein gegen oxidativen, thermischen oder actinischen Abbau empfindliches organisches Material und insbesondere ein synthetisches Polymer oder eine funtionelle Flüssigkeit und mindestens eine Verbindung der Formel I enthalten.

Als weitere organische Materialien, die erfindungsgemäss mit Hilfe der Verbindung der Formel I stabilisiert werden können, sind beispielsweise erwähnt:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit

Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit

EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten, wie z.B. von Epoxyacrylaten, Urethan-acrylaten oder Polyester-acrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z.B. von Bis-glycidylethern oder von cycloaliphatischen Diepoxiden.
26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als funktionelle Flüssigkeiten oder als Spinnpräparationen, einschliesslich der wässrigen Emulsionen von Spinnpräparationen, Anwendung finden.
29. Wässrige Emulsionen natürlicher oder synthetischer Kautschuke, wie z.B. Naturkautschuk-Latex oder Latices von carboxylierten Styrol-Butadien-Copolymeren.

Zweckmässige Gruppen sind die synthetischen Polymere und bevorzugte Gruppen von Kunststoffen sind diejenigen aus der Reihe der Poylolefine, der Elastomere, ABS, IPS, der Polycarbonate, der Polyimide, der Polyester, der Polyacetale und der carboxylierten SBR.

Die Einarbeitung der erfindungsgemässen Stabilisatorsubstanzen und allenfalls weiterer Zusätze in das organische Material erfolgt nach bekannten Methoden. Sie kann beispielsweise durch Einmischen der erfindungsgemässen Produkte und gegebenenfalls weiterer Additive nach den in der Technik üblichen Methoden, vor oder während der Formgebung, oder auch durch Aufbringen der gelösten oder dispergierten Verbindungen auf das Polymer, gegebenenfalls unter nachträglichem Verdunsten des Lösungsmittels, erfolgen. Die erfindungsgemässen Produkte können auch in Form eines Masterbatches, der diese beispielsweise in einer Konzentration von 2,5 bis 25 Gew.-% enthält, den zu stabilisierenden Materialien zugesetzt werden. Die erfindungsgemässen Produkte können auch vor oder während der Polymerisation oder vor der Vernetzung zugegeben werden.

Zweckmässig kann die Einarbeitung der Verbindungen der Formel I nach folgenden Methoden erfolgen:
- als Emulsion oder Dispersion (z.B. zu Latices oder Emulsionspolymeren)
- Als Trockenmischung während des Vermischens von Zusatzkomponenten oder Polymermischungen
- direktes Zugeben in die Verarbeitungsapparatur (.z.B. Extruder, Innenmischer usw.)
- als Lösung oder Schmelze.

Die so stabilisierten Materialien können in verschiedenster Form angewendet werden, z.B. als Folien, Fasern, Bändchen, Formmassen, Profile oder als Bindemittel für Lacke, Klebestoffe oder Kitte.

Beispielsweise in Polyolefinen haben die Verbindungen der Formel I aussergewöhnlich gute farbverbessernde Eigenschaften.

Zusätzlich zu den Verbindungen der Formel I können die obengenannten organischen Materialien und besonders die synthetischen Polymere andere Additive enthalten. Beispiele für zusätzliche Additive sind:

1. Antioxidantien

1.1. Alkylierte Monophenole, z.B. 2,6-Di-tert.-butylphenol 2,6-Di-tert.-butyl-4-methylphenol, 2-Tert.butyl-4,6-dimethylphenol, 2,6-Di-tert.-butyl-4-ethylphenol, 2,6-Di-tert.butyl-4-n-butylphenol, 2,6-Di-tert.-butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6(α,α-dimethylbenzyl)-4-nonylphenol], 4,4′-Methylen-bis-(2,6-di-tert.butylphenol), 4,4′-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3′-tert.butyl-4′-hydroxyphenyl)-butyrat], Bis-(3-tert.butyl-4-hydroxy-5-methylphenyl)-dicyclopentadien, Bis-[2-(3′-tert.butyl-2′-hydroxy-5′-methylbenzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N′-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.10. Amide der β-(3,5-Di-tert.butyl-4-hydroxyphenyl)-propionsäure, wie z.B. N,N′-Bis-(3,-di-tert.butyl-4-hydroxyphenylpropionyl)-hexamethylendiamin, N,N′-Bis-(3,5-di-tert.butyl-4-hydroxyphenylpropionyl)-trimethylendiamin, N,N′-Bis-(3,5-di-tert.butyl-4-hydroxyphenylpropionyl)-hydrazin.

2. UV-Absorber und Lichtschutzmittel

2.1. 2-(2′-Hydroxyphenyl)-benztriazole, wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl, 4′-Octoxy-, 3′,5′-Di-tert.amyl-, 3′,5′-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2′-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis( 1,2,2,6, 6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N′-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1′-(1,2-Ethandiyl)-bis-(3,3,5,5-tetramethyl-piperazinon).

2.7. Oxalsäurediamide, wie z.B. 4,4′-Di-octyloxy-oxanilid, 2,2′-Di-octyloxy-5,5′-di-tert.butyl-oxanilid, 2,2′-Di-dodecyloxy-5,5′di-tert.butyl-oxanilid, 2-Ethoxy-2′-ethyl-oxanilid, N,N′-Bis-(3-dimethylaminopropyl)-oxalamid, 2-Ethoxy-5-tert.butyl-2′-ethyloxanilid und dessen Gemisch mit 2-Ethoxy-2′-ethyl-5,4′-di-tert.butyl-oxanilid, Gemische von o-und p-Methoxy- sowie von o- und p-Ethoxy-di-substituierten Oxaniliden.

3. Metalldesaktivatoren, wie z.B. N,N′-Diphenyloxalsäurediamid, N-Salicylal-N′-salicyloylhydrazin, N,N′-Bis-(salicyloyl)-hydrazin, N,N′-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4′-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Zweckmässige Zusammensetzungen nach vorliegender Erfindung sind demnach beispielsweise solche, enthaltend

a) synthetische Polymere und

b) wenigstens eine Verbindung der Formel I, wie vorstehend beschrieben.

Das synthetische Polymer in genannter Zusammensetzung ist bevorzugt ein Polyolefin.

Andere zweckmässige Zusammensetzungen enthalten

a) eine funktionelle Flüssigkeit bevorzugt aus der Reihe der Schmierstoffe, der Hydraulikflüssigkeiten und der Metallbearbeitungsflüssigkeiten und

b) wenigstens eine Verbindung der Formel I, wie vorstehend beschrieben.

Besonders bevorzugt sind Schmierstoffe und dabei sind die Mineralöle, die synthetischen Oele oder Mischungen davon von besonderem Interesse.

Als funktionelle Flüssigkeiten aus der Reihe der Schmierstoffe, der Hydraulikflüssigkeiten und der Metallbearbeitungsflüssigkeiten kommen die an sich bekannten Produkte zum Einsatz.

Die in Frage kommenden Schmierstoffe und Hydraulikflüssigkeiten sind dem Fachmann geläufig und z.B. in Dieter Klamann "Schmierstoffe und verwandte Produkte", Verlag Chemie, Weinheim, 1982, in Schewe-Kobek, "Das Schmiermittel-Taschenbuch", Dr. Alfred Hüthig-Verlag, Heidelberg, 1974, oder in "Ullmanns Encyclopädie der technischen Chemie", Band 13, Seiten 85-94 (Verlag Chemie, Weinheim, 1977) beschrieben.

Beispiele hierfür sind Schmierstoffe und Hydraulikflüssigkeiten auf Mineralöl-Basis oder synthetische Schmierstoffe oder Hydraulikflüssigkeiten, insbesondere solche, die Carbonsäure-Esterderivate darstellen und bei Temperaturen von 200°C und höher verwendet werden.

Beispiele von synthetischen Schmierstoffen umfassen Schmierstoffe auf der Basis eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säure, wie z.B. Pentaerythrit-tetracaprylat, oder eines komplexen Esters von einwertigen und zweiwertigen Säuren mit mehrwertigen Alkoholen, wie z.B. ein komplexer Ester von Trimethylolpropan mit Capryl- und Sebacinsäure oder von einem Gemisch davon.

Besonders geeignet sind neben Mineralölen z.B. Poly-α-Olefine, Schmierstoffe auf Esterbasis, Phosphate, Glykole, Polyglykole und Polyalkylenglykole, sowie deren Mischungen mit Wasser.

Die Verbindungen der Formel I sind gut in Schmierstoffen löslich und sind deshalb als Zusätze zu Schmierstoffen besonders geeignet und es ist auf ihre überraschend gute antioxidative und antikorrosive Wirkung hinzuweisen.

Beispielsweise in Schmierstoffen für Verbrennungsmotoren, wie z.B. in Verbrennungsmotoren nach dem Otto-Prinzip, vermögen die Verbindungen der Formel I ihre überragenden Eigenschaften zu entfalten. So verhindern dabei die Verbindungen der Formel I in Schmierölen die Schlammbildung oder reduzieren diese in überraschendem Masse.

Es ist auch möglich, sogenannte Masterbatches herzustellen.

Die Verbindungen der Formel I wirken schon in sehr geringen Mengen als Additive in Schmierstoffen. Sie werden den Schmierstoffen zweckmässig in einer Menge von 0,01 bis 5 Gew.-%, vorzugsweise in einer Menge von 0,05 bis 3 Gew.-% und ganz besonders bevorzugt in einer Menge von 0,1 bis 2 Gew.-%, jeweils bezogen auf den Schmierstoff, beigemischt.

Die Schmierstoffe können zusatzlich andere Additive enthalten, die zugegeben werden, um die Grundeigenschaften von Schmierstoffen noch weiter zu verbessern; dazu gehören: Antioxidantien, Metallpassivatoren, Rostinhibitoren, Viskositätsindex-Verbesserer, Stockpunkterniedriger, Dispergiermittel, Detergentien, Hochdruck-Zusätze und Antiverschleiss-Additive.

Beispielsweise ist eine Reihe solcher Verbindungen obiger Auflistung "1. Antioxidantien", insbesondere Punkte 1.1 bis 1.10 zu entnehmen. Darüberhinaus sind weitere Additive beispielhaft zu nennen:

Beispiele für aminische Antioxidantien:

N,N′-Di-isopropyl-p-phenylendiamin, N,N′-Di-sec-butyl-p-phenylendiamin, N,N′-Bis(1,4-dimethyl-pentyl)-p-phenylendiamin, N,N′-Bis(1-ethyl-3-methyl-pentyl)-p-phenylendiamin, N,N′-Bis(1-methyl-heptyl)-p-phenylendiamin, N,N′-Dicyclohexyl-p-phenylendiamin, N,N′-Diphenyl-p-phenylendiamin, N,N′-Di-(naphthyl-2)-p-phenylendiamin, N-Isopropyl-N′-phenyl-p-phenylendiamin, N-1,3-(Dimethyl-butyl)-N′-phenyl-p-phenylendiamin, N-(1-Methyl-heptyl)-N′-phenyl-p-phenylendiamin, N-Cyclohexyl-N′-phenyl-p-phenylendiamin, 4-(p-Toluol-sulfonamido)-diphenylamin, N,N′-Dimethyl-N,N′-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p′-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4′-Diamino-diphenylmethan, 4,4′-Diamino-diphenylmethan, N,N,N′,N′-Tetramethyl-4,4′-diamino-diphenylmethan, 1,2-Di-[(2-methyl-phenyl)-amino]-ethan, 1,2-Di-(phenylamino)-propan, (o-Tolyl)-biguanid, Di-[4-(1′,3′-dimethyl-butyl)-phenyl]amin, tert-octyliertes N-Phenyl-1-naphthylamin, Gemisch aus mono- und dialkylierten tert-Butyl-/tert-Octyldiphenylaminen, 2,3-Dihydro-3,3-dimethyl-4H-1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Passivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5'-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbernsteinsäureanhydrid, z.B. Dodecenylbernsteinsäure-anhydrid, Alkenylbernsteinsäure-Teilester und -Teilamide, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside/Detergentien sind:

Polybutenylbernsteinsäureamide oder -imide, Polybutenylphosphonsäurederivate, basische Magnesium-, Calcium-, und Bariumsulfonate und -phenolate.

Beispiele für Hochdruckzusätze/Verschleissschutz-Additive sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Vorliegende Erfindung umfasst auch neue Verbindungen der allgemeinen Formel Ia

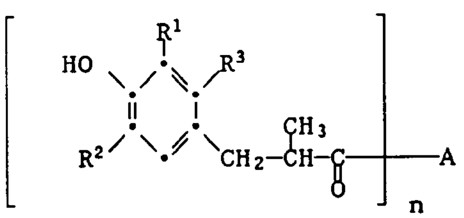

(Ia),

wobei

$R^1$ = Alkyl mit 1 bis 18 C-Atomen, Cycloalkyl mit 5 bis 12 C-Atomen, Phenyl oder Aralkyl mit 7 bis 9 C-Atomen,

$R^2$ = -H, Alkyl mit 1 bis 18 C-Atomen, Cycloalkyl mit 5 bis 12 C-Atomen, Phenyl oder Aralkyl mit 7 bis 9 C-Atomen und

$R^3$ = -H oder $CH_3$ bedeutet und

n eine Zahl von 1 bis 4 oder 6 bedeutet, wobei,
wenn n = 1 ist,
A = -$OR^4$ oder

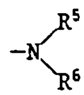

ist, und
$R^4$ die Bedeutung von -H, Alkyl mit 5 bis 45 C-Atomen, Cycloalkyl mit 5 bis 12 C-Atomen, Alkenyl mit 2 bis 18 C-Atomen,

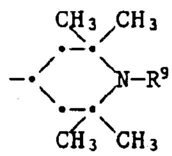

oder -$CH_2CH_2$-$XR^{5a}$ hat, und
$R^5$ und $R^6$, unabhängig voneinander, -H, Alkyl mit 1 bis 20 C-Atomen, Phenyl, Cycloalkyl mit 5 bis 12 C-Atomen, $C_1$-$C_4$ alkylsubstituiertes Cycloalkyl mit 5 bis 12 C-Atomen, Alkenyl mit 3 bis 8 C-Atomen, Aralkyl mit 7 bis 10 C-Atomen oder

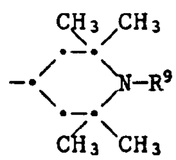

bedeuten, und zusätzlich
$R^5$ -$NH_2$ bedeutet,
$R^9$ = -H, Alkyl mit 1 bis 8 C-Atomen,

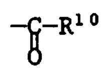 ,

-O· oder -$OR^{9}$'ist, wobei
$R^{9}$' = -H, Alkyl mit 1-25 C-Atomen oder

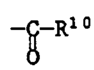

darstellt,
X = -O-, -S- oder

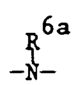

ist,
$R^{5a}$ =

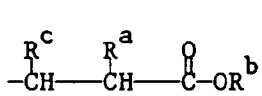 , 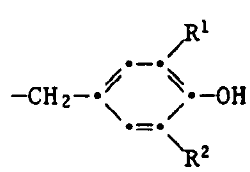 ,

-H, Alkyl mit 1-24 C-Atomen, Phenyl, Cycloalkyl mit 5 bis 12 C-Atomen oder

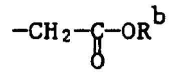

ist,

und

$R^{10}$ = Alkyl mit 1 bis 20 C-Atomen ist,

$R^a$ = -H oder $-CH_3$,

$R^b$ = -H oder Alkyl mit 1 bis 24 C-Atomen und

$R^c$ = -H oder $-CH_3$ sind, mit der Massgabe, dass $R^a$ und $R^c$ nicht gleichzeitig $-CH_3$ sind,

und

$R^{6a}$ = Alkyl mit 1 bis 18 C-Atomen, Phenyl, mit einer oder mehreren Alkylgruppen mit insgesamt 1 bis 24 C-Atomen substituiertes Phenyl oder $C_5$-$C_8$-Cycloalkyl ist, oder,

wenn n = 2 ist,

A =

$-O-C_xH_{2x}-O-$,

$-O-(CH_2CH_2O)_aCH_2CH_2O-$ ,

$-O-CH_2-CH_2-B-CH_2-CH_2-O-$ ,

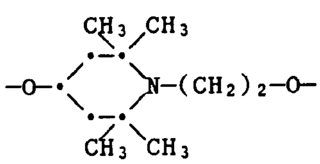 , 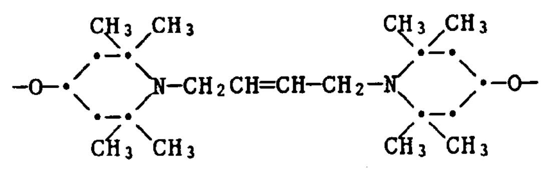 ,

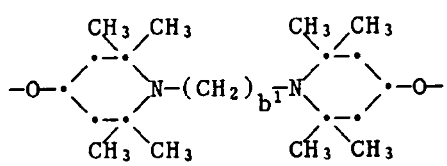 ,

$-O-CH_2CH=CHCH_2-O-$ , $-O-CH_2C\equiv CCH_2-O-$ , 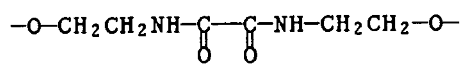 ,

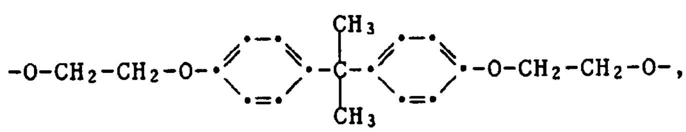,

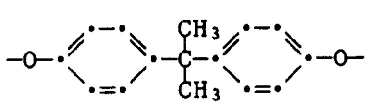 , 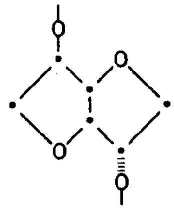 oder 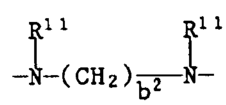 ist,

wobei

a eine Zahl von 1 bis 30 ist,

x eine Zahl von 2 bis 20 ist,

B = -S-,

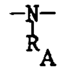 oder 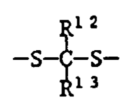

ist,

$R_A$ = Alkyl mit 1 bis 20 C-Atomen, Phenyl oder mit einer oder mehreren Alkylgruppen mit insgesamt 1 bis 20 C-Atomen substituiertes Phenyl, Cyclohexyl oder

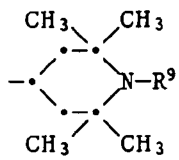

ist, und

$R^{11}$, $R^{12}$ und $R^{13}$, unabhängig voneinander, -H, Alkyl mit 1 bis 12 C-Atomen oder Phenyl sind oder

$R^{12}$ und $R^{13}$, mit dem C-Atom, an das sie gebunden sind, einen Cycloalkylring mit 5 bis 12 C-Atomen bilden,

$b^1$ eine Zahl von 2 bis 10 ist, und

$b^2$ eine Zahl von 0 bis 6 darstellt,

oder wenn n = 3 ist,

A =

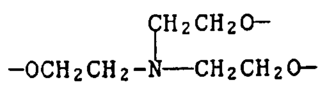 oder 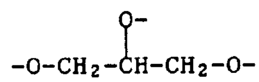

bedeutet,

oder wenn n = 4 ist

A =

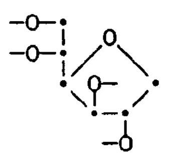 oder 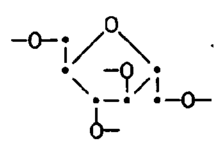

bedeutet,

oder wenn n = 6 ist,

A =

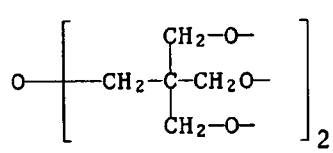

bedeutet.

Zweckmässige Verbindungen der Formel Ia sind solche, in denen

$R^1$ = Alkyl mit 1 bis 18 C-Atomen, Cycloalkyl mit 5 bis 12 C-Atomen, Phenyl oder Benzyl,

$R^2$ = -H, Alkyl mit 1 bis 18 C-Atomen, Cycloalkyl mit 5 bis 12 C-Atomen, Phenyl oder Benzyl und

$R^3$ = -H bedeuten und

n eine Zahl von 1 bis 4 oder 6 bedeutet, wobei,

wenn n = 1 ist,

A = $-OR^4$ oder

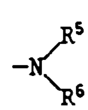

ist, und

$R^4$ die Bedeutung von -H, Alkyl mit 5 bis 20 C-Atomen, Cyclohexyl, Alkenyl mit 2 bis 18 C-Atomen oder $-CH_2CH_2-XR^{5a}$ hat, und

$R^5$ -H, Alkyl mit 1 bis 12 C-Atomen, Phenyl, Cyclohexyl, $C_1$-$C_4$ alkylsubstituiertes Cycloalkyl mit 5 bis 12 C-Atomen, Alkenyl mit 3 bis 8 C-Atomen, Benzyl oder $-NH_2$ bedeutet, und

$R^6$ -H oder Alkyl mit 1 bis 12 C-Atomen bedeutet,

X = -O-, -S- oder

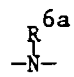

ist,

$R^{5a}$ = -H, Alkyl mit 1-12 C-Atomen, Phenyl, Cyclohexyl oder

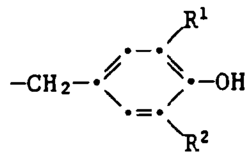

ist,

und

$R^{6a}$ = Alkyl mit 1 bis 12 C-Atomen, Phenyl oder mit einer oder mehreren Alkylgruppen mit insgesamt 1 bis 18 C-Atomen substituiertes Phenyl ist, oder,

wenn n = 2 ist,

A =

$$-O-C_xH_{2x}-O-,$$
$$-O-(CH_2CH_2O)_aCH_2CH_2O-,$$
$$-O-CH_2-CH_2-B-CH_2-CH_2-O-,$$

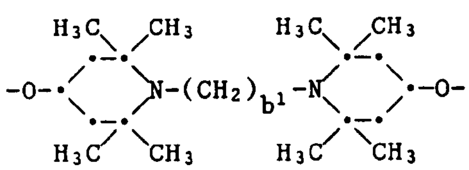

$-O-CH_2CH=CHCH_2-O-$ , 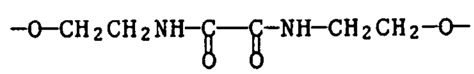 ,

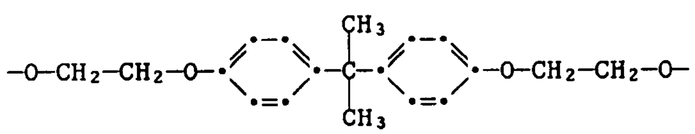 ,

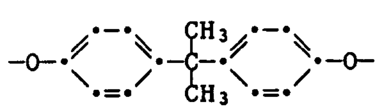 , 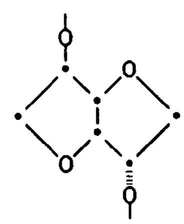 oder 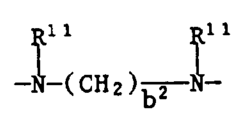

ist,

wobei

a eine Zahl von 1 bis 12 ist,

x eine Zahl von 2 bis 12 ist,
B = -S- oder

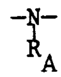

ist,

$R_A$ = Alkyl mit 1 bis 12 C-Atomen, Phenyl oder mit einer oder mehreren Alkylgruppen mit insgesamt 1 bis 18 C-Atomen substituiertes Phenyl, oder Cyclohexyl ist, und
$R^{11}$, -H, Alkyl mit 1 bis 12 C-Atomen oder Phenyl ist und
$b^1$ eine Zahl von 2 bis 6 ist, und
$b^2$ eine Zahl von 0 bis 6 darstellt,
oder wenn n = 3 ist,
A =

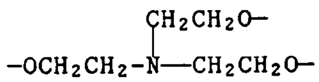 oder 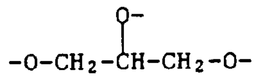

bedeutet,
oder wenn n = 4 ist
A =

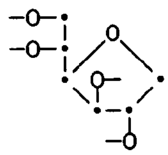 oder 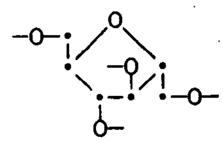

bedeutet,
oder wenn n = 6 ist,
A =

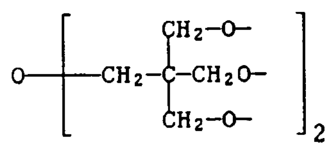

bedeutet.

Besonders zweckmässige Verbindungen der Formel Ia sind solche, in denen
$R^1$ = Alkyl mit 1 bis 8 C-Atomen, Cyclohexyl oder Phenyl,
$R^2$ = -H, Alkyl mit 1 bis 8 C-Atomen, Cyclohexyl oder Phenyl und
$R^3$ = -H darstellen und
n = 1 ist und
A = $-OR^4$ oder

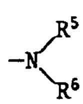

darstellt, wobei
$R^4$ = -H, Alkyl mit 5 bis 18 C-Atomen, Cyclohexyl, $-CH_2CH=CH_2$, $-(CH_2)_7-CH=CH-(CH_2)_7CH_3$,

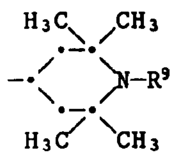

oder $-CH_2CH_2XR^{5a}$ bedeutet, wobei

$R^9$ = -H, Alkyl mit 1 bis 4 C-Atomen, -O·, -O-Alkyl mit 1 bis 4 C-Atomen oder Cyclohexyl ist,

X = -O-, -S- oder

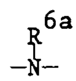

ist,

$R^{5a}$ = -H, Alkyl mit 1 bis 18 C-Atomen, Phenyl,

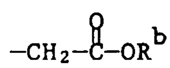,

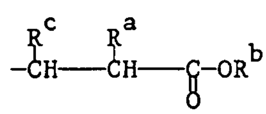 oder 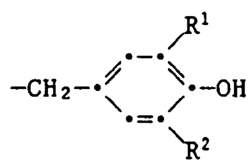

ist, wobei

$R^b$ = Alkyl mit 1 bis 24 C-Atomen ist,

$R^a$ = -H oder $-CH_3$ ist,

$R^c$ = -H ist und

$R^{6a}$ = Alkyl mit 1 bis 12 C-Atomen oder Phenyl ist, und

$R^5$ und $R^6$, unabhängig voneinander, -H, Alkyl mit 1 bis 12 C-Atomen, Phenyl oder

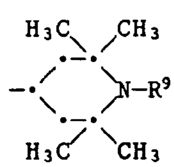

sind,

wobei $R^9$ die oben angegebene Bedeutung hat, oder

$R^5$ $-NH_2$ und $R^6$ -H bedeutet,

oder,

n gleich 2 ist, wobei

A = $-O-C_xH_{2x}-O-$ bedeutet und

x = 2 bis 8 ist.

Bevorzugt sind Verbindungen der Formel Ia, wobei

$R^1$ = tert.-Butyl

$R^2$ = -H, Methyl oder tert.-Butyl und

$R^3$ = -H darstellen und

n gleich 1 ist, wobei

A = $-OR^4$ oder

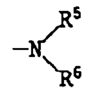

darstellt, und

$R^4$ = Alkyl mit 6 bis 18 C-Atomen, $-(CH_2)_7-CH=CH-(CH_2)_7CH_3$,

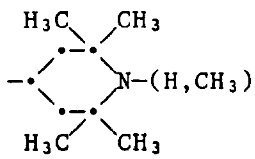

oder

$-CH_2CH_2-SR^{5a}$ ist, wobei

$R^{5a}$ = -H oder

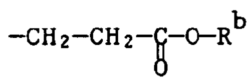

bedeutet, wobei

$R^b$ = $n-C_4H_9$ bis $n-C_8H_{17}$ oder tert-$C_4H_9$ bis tert.-$C_8H_{17}$ ist,

oder

n gleich 2 ist, wobei

A = $-O-C_xH_{2x}-O-$ und

x = 2 bis 8 ist, oder

A = $-O-(CH_2-CH_2-O-)_a-CH_2-CH_2-O-$ und

a = 1 bis 4 ist, oder

A = $-O-CH_2-CH_2-B-CH_2-CH_2-O-$ und

B = -S- ,

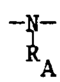 oder 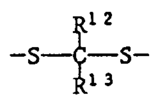

ist, wobei

$R_A$ = $C_4$-$C_8$-Alkyl oder Phenyl,

$R^{12}$ = H, $C_1$-$C_8$-Alkyl und

$R^{13}$ = H, $C_1$-$C_8$-Alkyl oder Phenyl darstellen, oder

A =

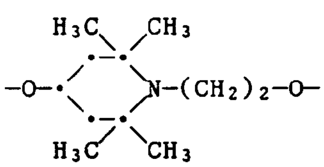 , 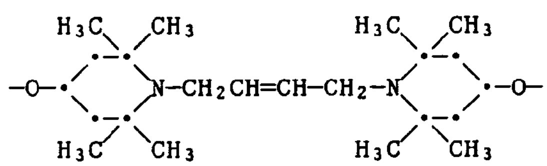 oder

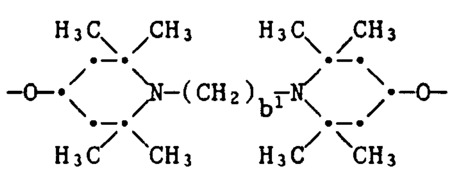 ist, wobei

$b^1$ = 2 bis 6 ist, oder

A =

-O-$CH_2$-CH=CH-$CH_2$-O- ,
-O-$CH_2$-C≡$CH_2$-O- ,

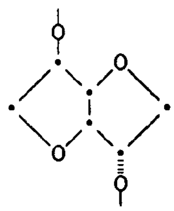 , 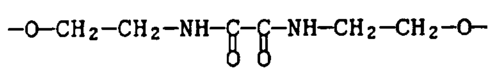 oder 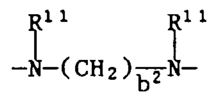

ist,

wobei
$b^2$ = 0 bis 6 und
$R^{11}$ = -H oder $C_1$-$C_8$-Alkyl darstellen.

Weitere bevorzugte Verbindungen der Formel Ia sind solche, in denen
$R^1$ = tert.-Butyl
$R^2$ = -H, -$CH_3$ oder tert.-Butyl und
$R^3$ = -H darstellen und
n = 1 ist und dabei
A = -$OR^4$ bedeutet und
$R^4$ = $C_6$-$C_8$-Alkyl ist.

Zu den weiteren bevorzugten Verbindungen der Formel Ia zählen auch diejenigen, in denen
$R^1$ = tert.-Butyl
$R^2$ = -H, -$CH_3$ oder tert.-Butyl und
$R^3$ = -H darstellen und
n = 2 ist und dabei
A = -O-$C_xH_{2x}$-O- und
x = 2 bis 6 ist, oder
A = -O-($CH_2$-$CH_2$-O)$_a$-$CH_2$-$CH_2$-O- und
a = 1, 2 oder 3 ist,
oder
A = -O-$CH_2$-$CH_2$-B-$CH_2$-$CH_2$-O- und
B = -S- oder

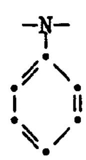

ist,

oder
A =

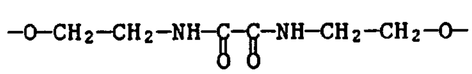

oder
A = -NH-NH- ist.

Auch bevorzugt sind Verbindungen der Formel Ia, wobei
$R^1$ = tert.-Butyl,
$R^2$ = -H, -$CH_3$ oder tert.-Butyl
$R^3$ = -H darstellen,
n = 1 ist und dabei
A = -$OR^4$ darstellt, wobei

$R^4$ = $C_6$-$C_{18}$-Alkyl ist, oder
n = 2 ist und dabei
A = $-O-C_xH_{2x}-O-$ und x = 2-6,
$-OCH_2CH_2-S-CH_2CH_2O-$ oder
$-O(CH_2CH_2O)_aCH_2CH_2-O-$ und
a = 1 bis 4 sind.

Besonders bevorzugt sind Verbindungen der Formel Ia oben angegebener Art, wobei a = 1 oder 2 ist.

Besonders bevorzugt sind Verbindungen der Formel Ia, wie vorbeschrieben, worin $R^4$ Alkyl mit 9 bis 18 C-Atomen ist.

Ebenfalls bevorzugt sind Verbindungen der Formel Ia, wobei
$R^1$ = tert.-Butyl
$R^2$ = -H, $-CH_3$, tert.-Butyl und
$R^3$ = -H darstellen und
n gleich 3 ist und dabei
A =

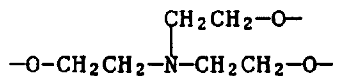

darstellt,
oder
n = 4 ist und dabei
A =

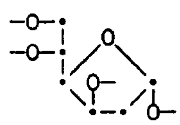

darstellt,
oder
n = 6 ist und dabei
A =

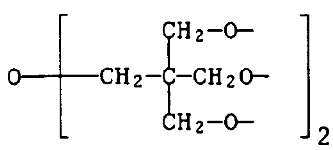

bedeutet.

Ganz besonders bevorzugt sind Verbindungen aus der Reihe mit den Formeln

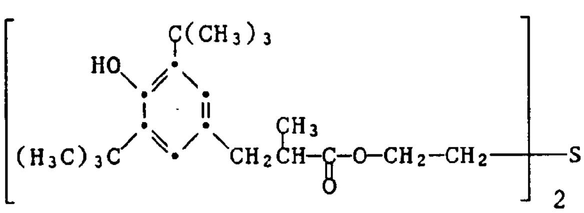

,

.

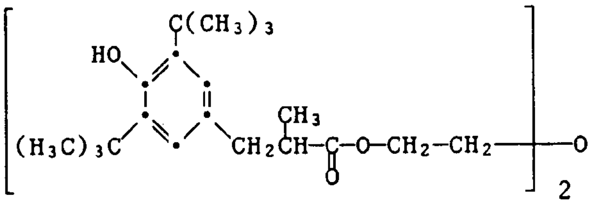

,

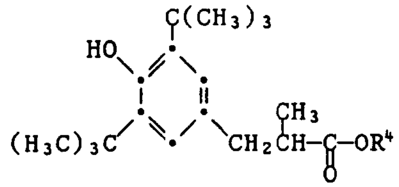

,

wobei

$R^4$ die Bedeutung von $C_8$-$C_{16}$-Alkyl und insbesondere von -i-$C_8H_{17}$, -n-$C_{12}H_{25}$ oder -n-$C_{18}H_{37}$ hat,

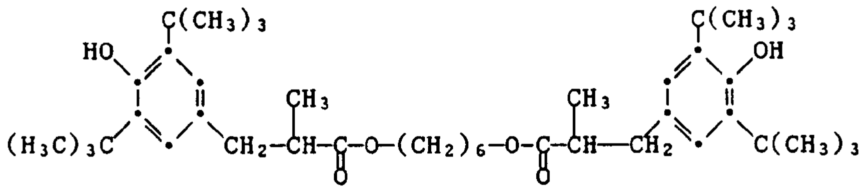

und

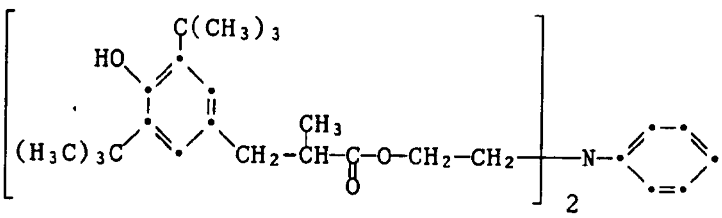

Im Umfang vorliegender Erfindung ist auch die Verwendung der Verbindungen der Formel I gemäss obenstehender Beschreibung als Antioxidantien in gegen oxidativen, thermischen oder actinischen Abbau empfindlichen organischen Materialien. Die Materialien an sich, als auch deren bevorzugte Gruppen und Verbindungen sind weiter oben definiert.

Besonders bevorzugt ist die Verwendung der Verbindungen der Formeln I und Ia in Schmierstoffen.

Die nachfolgenden Beispiele erläutern die vorliegende Erfindung weiter, ohne sie in ihrem Umfang zu beschränken. Die Angaben in Teilen oder Prozenten beziehen sich, soweit nicht anders angegeben, immer auf das Gewicht.

Beispiel 1: Octadecyl-2-methyl-3-(3′,5′-di-tert-butyl-4′-hydroxyphenyl)-propionat

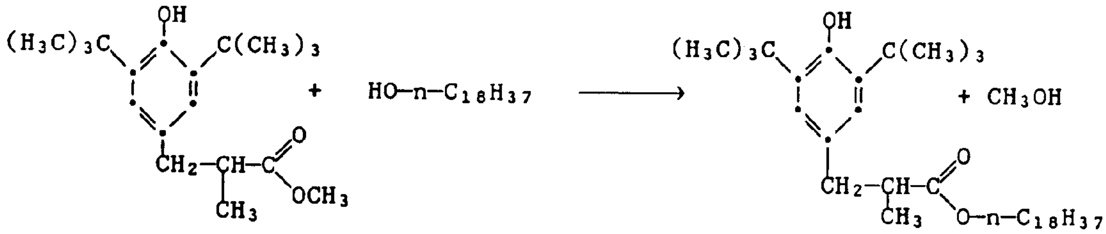

30,6 g (0,1 Mol) Methyl-2-methyl-3-(3′,5′-di-tert-butyl-4′-hydroxy phenyl)- propionat und 27 g (0,1 Mol) 1-n-Octadecanol werden in einem 4-Hals-Glaskolben vorgelegt und auf 100°C aufgeheizt. Dann gibt man 0,25 g (1 Mol-%) Dibutylzinnoxyd zu und erhitzt bis auf 180°C. Dabei spaltet sich Methanol ab. Die Temperatur wird während 6 Stunden auf 180°C gehalten.

Das Reaktionsgemisch wird mit HCl (20 %) neutralisiert am Hochvakuum getrocknet.

Man erhält 52,4 g ≙ 96,2 % d.Th. eines leicht gelblichen Oeles, das langsam kristallisiert. Der Schmelzpunkt liegt dann bei über 40°C.

| | | | |
|---|---|---|---|
| Analyse: | berechnet | 79,35 % C | 11,84 % H |
| | gefunden | 79,57 % C | 11,68 % H |

Beispiel 2: n-Nonyl-2-methyl-3-(3′,5′-di-tert-butyl-4′-hydroxyphenyl)-propionat

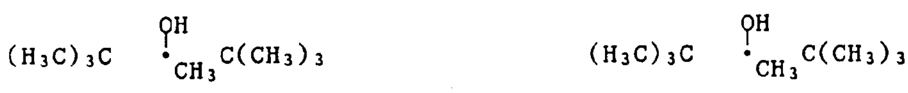

30,6 g Methyl-2-methyl-3-(3′,5′-di-tert-butyl-4′-hydroxyphenyl)-propionat und 14,4 g n-Nonylalkohol werden in einem 100 ml Sulfierkolben vorgelegt und auf 100°C aufgeheizt. Es werden 0,2 g Dibutylzinnoxyd zugegeben und bei Temperatur bis auf 150°C erhöht Ab einer Temperatur von 125°C spaltet sich Methylalkohol ab. Die Reaktion wird während 6 h bei 150°C gehalten.

Der Ansatz wird in 60 ml Toluol gelöst. Es wird am Rotationsverdampfer eingeengt und 2 h lang bei 80°C am Hochvakuum getrocknet.

Man erhält 39,4 g ≙ 94 % d.Th. eines viscosen leicht gelblichen Oels.

| | | | |
|---|---|---|---|
| Analyse: | berechnet | 77,46 % C | 11,08 % H |
| | gefunden | 77,35 % C | 10,93 % H |

Analog Beispiel 2 wird die Reaktion wiederholt, wobei gemäss:

Beispiel 3:

Durch Umsetzung von iso-Nonylalkohol nach einer Reaktionszeit von 7 Stunden bei 140°C ein gelblichen Oel enthaltend 96,8 % iso-Nonyl-2-methyl-3-(3′,5′-di-tert-butyl-4′-hydroxyphenyl)-propionat erhalten werden.

| | | | |
|---|---|---|---|
| Analyse: | berechnet | 77,46 % C | 11,08 % H |
| | gefunden | 77,33 % C | 11 % H |

Beispiel 4: Diethylenglykol bis[2-methyl-3-(3′,5′-di-t-butyl-4′-hydroxyphenyl)-propionat]

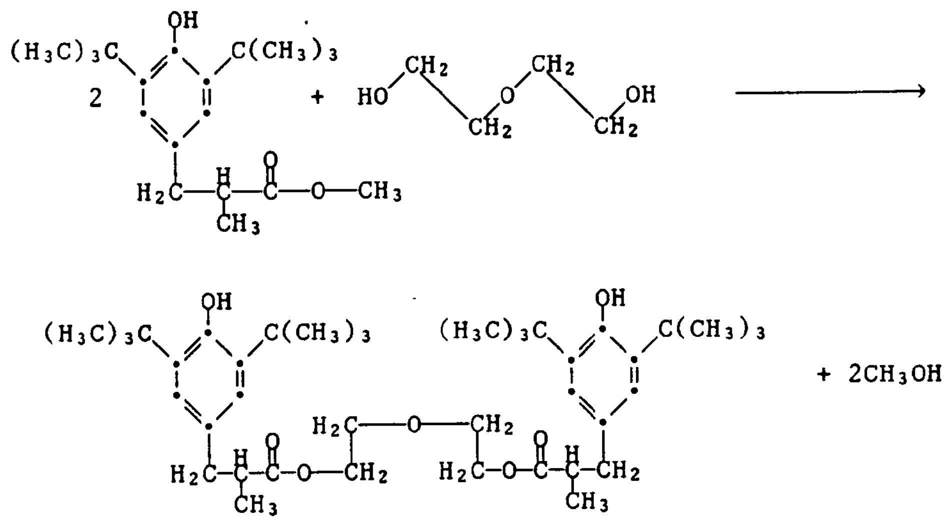

61,2 g (0,2 Mol) Methyl-2-methyl-3-(3′,5′-di-tert-butyl-4-hydroxyphenyl)-propionat und 10,6 g (0,1 Mol) Diethylenglykol werden in einem 4-Hals-Glaskolben vorgelegt und auf 100°C aufgeheizt. Dann werden 0,5 g ≙ 1 Mol-% Dibutylzinnoxyd zugegeben und das Reaktionsgemisch wird auf 180°C aufgeheizt, wobei sich Methanol abspaltet. Die Temperatur wird während 5 Stunden gehalten. Die Aufarbeitung erfolgt durch Hochvakuumtrocknung.

Man erhält 64,2 g ≙ 98 % d.Th. eines braunen zähflüssigen Oeles. Dieses Oel wird mittels Säulenchromatographie nachgereinigt, wobei ein leicht gelbliches zähflüssiges Oel in einer Ausbeute von 79,4 % der eingesetzten Menge erhalten wird.

| | | | |
|---|---|---|---|
| Analyse: | berechnet | 73,26 % C | 9,54 % H |
| | gefunden | 73,51 % C | 9,38 % H |

Beispiel 5: Thiodiethylenglykol bis[2-methyl-3-(3',5'-di-t-butyl-4'-hydroxyphenyl)-propionat]

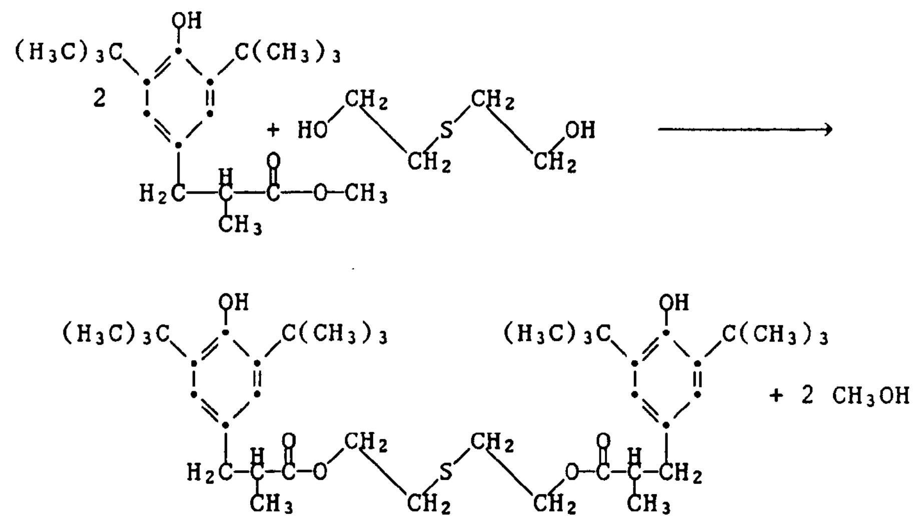

91,9 g (0,3 Mol) Methyl-2-methyl-3-(3',5'-di-t-butyl-4'-hydroxyphenyl)-propionat und 18,3 g (0,15 Mol) Thiodiethylenglykol werden in einem 4-Hals-Glaskolben vorgelegt und auf 100°C aufgeheizt. Man gibt 0,4 g Dibutylzinnoxyd ≙ 0,5 Mol-% zu und erwärmt bis auf 180°C, wobei sich Methanol abspaltet. Man hält die Temperatur während 6 Stunden bei 180°C.

Nach dem Waschen mit Wasser und einer Hochvakuumtrocknung werden 96,3 g ≙ 95,7 % d.Th. eines braunen Oeles erhalten.

Mittels Säulenchromatographie wird das braune Oel nachgereinigt, wobei 72,4 % der eingesetzten Menge in Form eines leicht braunen viskosen Oeles erhalten werden.

| | | | | |
|---|---|---|---|---|
| Analyse: | berechnet | 71,60 % C | 9,31 % H | 4,78 % S |
| | gefunden | 71,66 % C | 9,08 % H | 4,90 % S |

Beispiel 6: Dodecyl-2-methyl-3-(3',5'-di-t-butyl-4'-hydroxyphenyl)-propionat

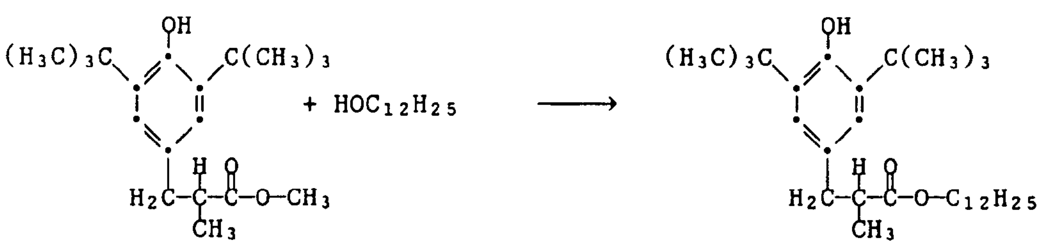

30,6 g (0,1 Mol) Methyl-2-methyl-3-(3',5'-di-t-butyl-4'-hydroxyphenyl)-propionat und 18,6 g (0,1 Mol) 1-Dodecanol werden in einem 4-Hals-Glaskolben vorgelegt und auf 100°C aufgeheizt, dann 0,25 g ≙ 1 Mol-% Dibutylzinnoxyd zugegeben und auf 180°C weiter erhitzt, wobei die Methanolabspaltung einsetzt. Das Reaktionsgemisch wird während 6 Stunden bei 180°C gehalten.

Es schliesst sich eine Hochvakuumtrocknung an. Man erhält 45,8 g ≙ 99,3 % d.Th. eines leicht gelblichen Oeles.

| | | |
|---|---|---|
| Analyse: berechnet | 78,20 % C | 11,38 % H |
| gefunden | 78,32 % C | 11,27 % H |

Beispiel 7: 2-Ethylhexyl-[2-methyl-3-(3',5'-di-t-butyl-4'-hydroxyphenyl)-propionat]

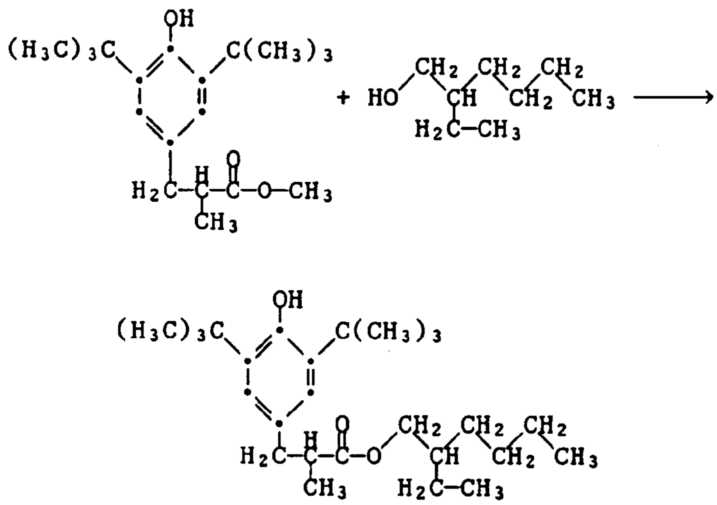

61,3 g (0,2 Mol) Methyl-2-methyl-3-(3',5'-di-t-butyl-4'-hydroxyphenyl)-propionat und 26 g (0,2 Mol) 2-Ethyl-1-hexanol werden in einem 4-Hals-Glaskolben vorgelegt und auf 100°C aufgeheizt. Dann wird 1 g (2 Mol-%) Dibutylzinnoxyd zugegeben und die Temperatur auf 180°C gesteigert. Es spaltet sich Methanol ab. Das Reaktionsgemisch wird während 9 Stunden bei 180°C gehalten. Das Endprodukt wird ohne Aufarbeitung, rein, in einer Ausbeute von 79,4 g ≙ 98,2 % d.Th., als leicht gelbliches Harz erhalten.

| | | |
|---|---|---|
| Analyse: berechnet | 77,18 % C | 10,96 % H |
| gefunden | 77,05 % C | 11,03 % H |

Beispiel 8: 1,6-Hexandiol bis[2-methyl-3-(3',5'-di-t-butyl-4'-hydroxyphenyl)-propionat]

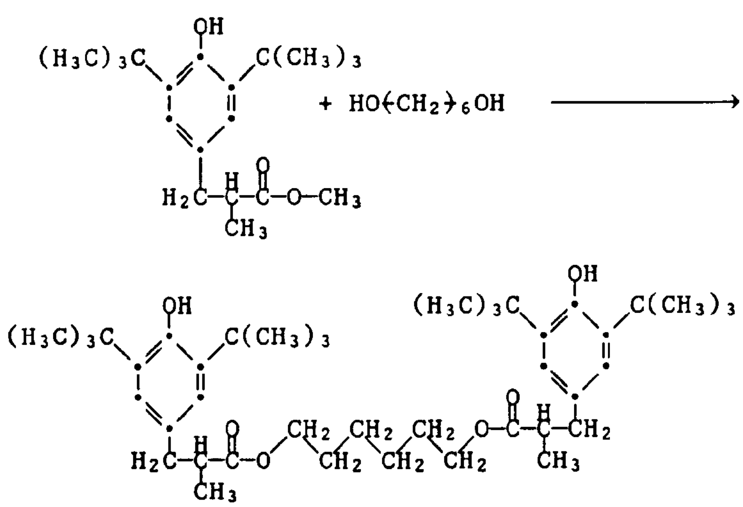

46 g (0,15 Mol) Methyl-2-methyl-3-(3',5'-di-t-butyl-4'-hydroxyphenyl)-propionat und 8,9 g (0,075 Mol) 1,6-Hexandiol werden in einem 200 ml 4-Hals-Glaskolben vorgelegt und auf 100°C aufgeheizt. 0,38 g ≙ 2 Mol-%

Dibutylzinnoxyd werden dann zugegeben und das Reaktionsgemisch wird anschliessend auf 180°C aufgeheizt. Es spaltet sich dabei Methanol ab. Die Reaktion wird 6 Stunden bei 180°C gehalten. Die anschliessende Aufarbeitung erfolgt durch Kristallisieren des Ansatzes aus 100 ml eines Gemisches aus Isopropanol und Wasser (7:3), Abnutschen und Waschen mit 2 x 30 ml kaltem Isopropanol/Wasser-Gemisch (7:3), sowie Trocknen im Ofen bei 50°C.

Die Ausbeute beträgt 24,7 g ≙ 49,4 % d.Th. eines leicht beigen pulverförmigen Produktes.

| | | | |
|---|---|---|---|
| Analyse: | berechnet: | 75,63 % C | 9,97 % H |
| | gefunden | 75,85 % C | 9,97 % H |

Beispiel 9:

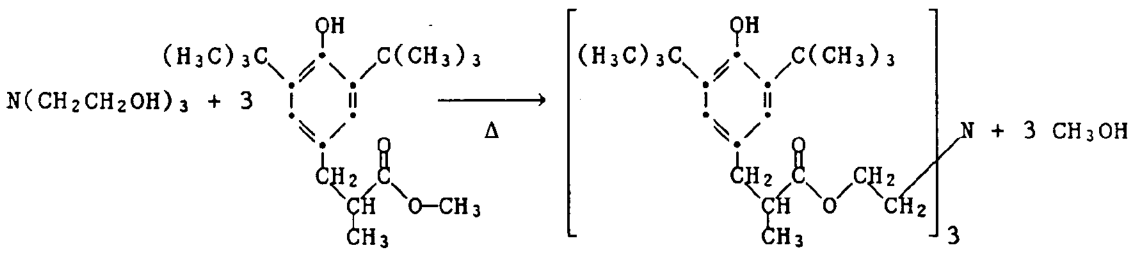


4,5 g N-Triäthanolamin und 30,6 g Methyl-2-methyl-3-(3′,5′-di-tert-butyl-4′-hydroxyphenyl)-propionat werden in einem 100 ml Sulfierkolben mit absteigendem "Liebig"-Kühler unter leichtem Stickstoffstrom vorgelegt. Es wird auf 120°C aufgeheizt und 0,25 g Dibutylzinnoxyd zugegeben und anschliessend die Temperatur bis 160°C erhöht, wobei MeOH abgespaltet wird. Die Reaktionstemperatur wird während 6 h bei 160°C gehalten.

Der Ansatz wird auf 80°C abgekühlt, dann in 60 ml Toluol aufgenommen und am Rotationsverdampfer eingeengt. Man erhält 26,8 g eines leicht braunen Oeles.

Nach Reinigung mittels "Flash"-Chromatographie mit Toluol:Essigester = 9:1 als Lösungsmittel, erhält man 23,6 g ≙ 81,1 d. Th. eines leicht braunen Oeles.

| Analyse: | gefunden | berechnet | |
|---|---|---|---|
| | 74,32 | 74,11 | % C |
| | 9,65 | 9,64 | % H |
| | 1,52 | 1,44 | % N |

Beispiel 10:

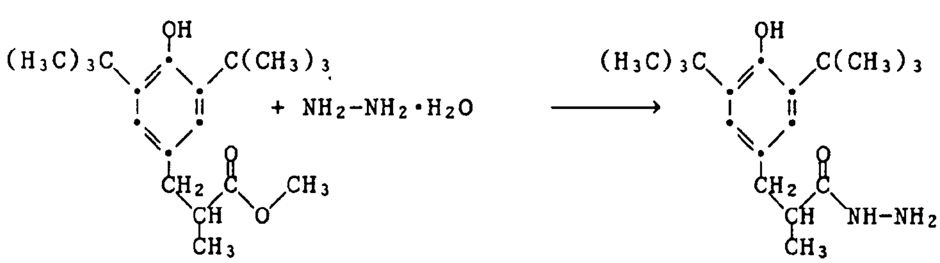

46 g Methyl-2-methyl-3-(3′,5′-di-tert-butyl-4′-hydroxyphenyl)-propionat werden in 100 ml Ethanol vorgelegt und bei Raumtemperatur zur klaren, leicht gelblichen Lösung von 7,5 g Hydrazinhydrat innerhalb 30 Minuten zugetropft. Die Temperatur wird erhöht, der Ethanol abdestilliert und dann die Reaktionstemperatur bis auf 100°C gesteigert, wobei ein unrührbarer dicker Brei entsteht. Nach Abkühlen auf Raumtemperatur und Mischen mit Hexan wird abgesaugt und im Ofen bei 60° getrocknet.

Man erhält 39,8 g ≙ 86,5 % d. Th. eines weissen Pulvers mit einem Schmelzpunkt von 128°C

| Analyse: | gefunden | berechnet | |
|---|---|---|---|
| | 70,72 | 70,55 | % C |
| | 10,03 | 9,87 | % H |
| | 9,03 | 9,14 | % N |

Beispiel 11:

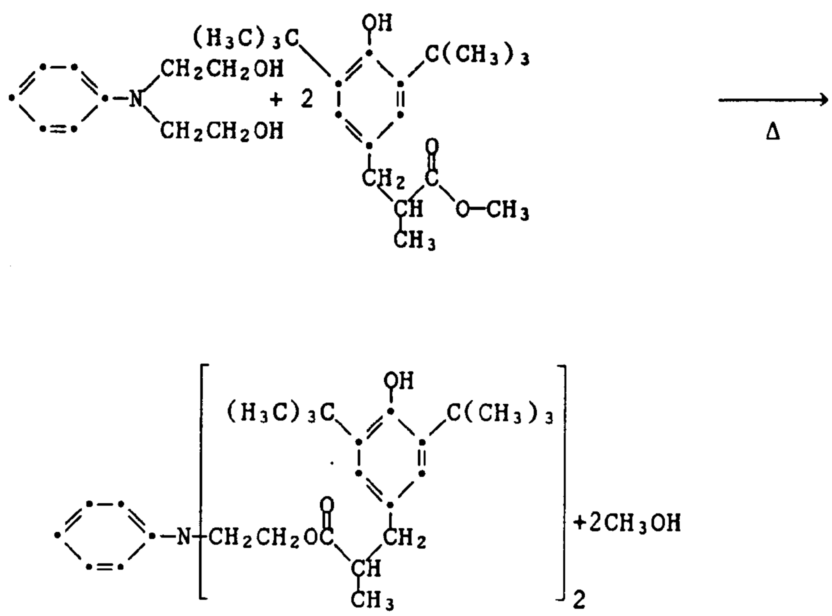


18,1 g N-Phenyldiäthanolamin und 61,3 g Methyl-2-methyl-3-(3′,5′-di-tert-butyl-4′-hydroxyphenyl)-propionat werden in einem 350 ml Sulfierkolben mit absteigendem "Liebig"-Kühler unter leichtem Stickstoffstrom vorgelegt. Das Reaktionsgemisch wird auf 120°C aufgeheizt, 0,5 g Dibutylzinnoxyd zugegeben und die Temperatur auf 160°C erhöht, wobei sich MeOH abspaltet. Bei 150°C wird 8 h ausreagieren gelassen. Der Ansatz wird auf 80°C abgekühlt und in 100 ml Toluol aufgenommen. Am Rotationsverdampfer wird eingeengt. Man erhält 74 g eines braunen Oeles.

Die Reinigung erfolgt mittels "Flash"-Chromatographie mit Toluol:Essigester 9:1 als Lösungsmittel.

Man erhält 59,5 g ≙ 81,5 % d. Th. eines orangen Oeles.

| Analyse: | gefunden | berechnet | |
|---|---|---|---|
| | 75,68 | 75,83 | % C |
| | 9,25 | 9,26 | % H |
| | 1,92 | 1,87 | % N |

Beispiel 12:

Verschiedene der Verbindungen nach den Beispielen 1-11 werden auf ihre Eignung als Stabilisatoren gegen oxidativen Abbau eines Schmieroeles geprüft.

Ein handelsübliches mineralisches Schmieroel (Mobil 15 SS 4) wird mit 0,05 Gew.-% eines handelsüblichen Korrosionsinhibitors des Types Alkenylsuccinylsäurehalbester versetzt.

Diesem vorbereiteten Oel werden nun die einzelnen Stabilisatoren, entsprechend den vorstehenden Beispielen, in einer Menge von 0,25 Gew.-%, bezogen auf das Oel, zugemischt und diese stabilisierten Oele einem Oxidationstest unterzogen.

Das Testverfahren ist bekannt als TOST-Test, (Oxidationscharakteristika von Mineraloel Mobil 15 SS 4, ASTM D 934/DIN 51587/IP 157).

Das zu testende Oel wird in Gegenwart von Wasser, Sauerstoff, einem Eisen-Kupferkatalysator und dem Stabilisator während 500 Stunden auf 95°C erwärmt. Danach wird der Säurewert TAN (in mg KOH-Verbrauch pro g Testoel) sowie der Schlamm (Sludge) (in mg Rückstand pro Ansatz) bestimmt. Die Resultate sind in Tabelle 1 zusammengestellt.

| Stabilisator gemäss | 500 Std TOST TAN (mg KOH/g Oel) | SLUDGE (mg) Schlamm |
|---|---|---|
| Beispiel 1 | 0,11 | 22 |
| Beispiel 2 | 0,20 | 103 |
| Beispiel 3 | 0,30 | 76 |
| Beispiel 4 | 0,06 | 30 |
| Beispiel 7 | 0,09 | 72 |
| Beispiel 8 | 0,07 | 33 |
| ohne | >2,0 | >1000 |

## Patentansprüche

### Patentansprüche für folgende Vertragsstaaten : BE, DE, FR, GB, IT, NL

1. Zusammensetzung, enthaltend
   a) mindestens ein dem oxidativen, thermischen oder actinischen Abbau unterworfenes organisches Material und
   b) mindestens eine Verbindung der allgemeinen Formel I

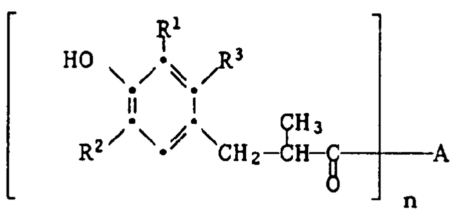

(I),

wobei
$R^1$ = Alkyl mit 1 bis 18 C-Atomen, Cycloalkyl mit 5 bis 12 C-Atomen, Phenyl oder Aralkyl mit 7 bis 9 C-Atomen,
$R^2$ = -H, Alkyl mit 1 bis 18 C-Atomen, Cycloalkyl mit 5 bis 12 C-Atomen, Phenyl oder Aralkyl mit 7 bis 9 C-Atomen und
$R^3$ = -H oder $CH_3$ bedeutet und

n eine Zahl von 1 bis 4 oder 6 bedeutet, wobei,
wenn n = 1 ist,
A = $-OR^4$ oder

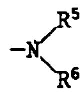

ist, und

$R^4$ die Bedeutung von -H, Alkyl mit 1 bis 45 C-Atomen, Cycloalkyl mit 5 bis 12 C-Atomen, Alkenyl mit 2 bis 18 C-Atomen,

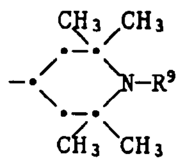

oder $-CH_2CH_2-XR^{5a}$ hat, und

$R^5$ und $R^6$, unabhängig voneinander, -H, Alkyl mit 1 bis 20 C-Atomen, Phenyl, Cycloalkyl mit 5 bis 12 C-Atomen, $C_1$-$C_4$ alkylsubstituiertes Cycloalkyl mit 5 bis 12 C-Atomen, Alkenyl mit 3 bis 8 C-Atomen, Aralkyl mit 7 bis 10 C-Atomen oder

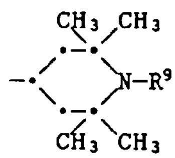

bedeuten, und

$R^5$ zusätzlich $-NH_2$ bedeutet,

$R^9$ = -H, Alkyl mit 1 bis 8 C-Atomen,

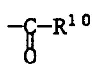 ,

-O· oder $-OR^{9\prime}$ ist, wobei

$R^{9\prime}$ = -H, Alkyl mit 1-25 C-Atomen oder

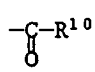

darstellt,

X = -O, -S- oder

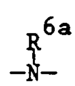

ist,

$R^{5a}$ =

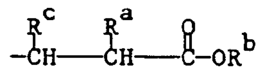 , 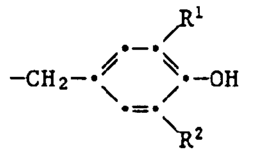 ,

-H, Alkyl mit 1-24 C-Atomen, Phenyl, Cycloalkyl mit 5 bis 12 C-Atomen oder

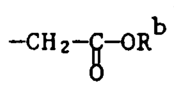

ist,
und

$R^{10}$ = Alkyl mit 1 bis 20 C-Atomen ist,
$R^a$ = -H oder $-CH_3$,
$R^b$ = -H oder Alkyl mit 1 bis 24 C-Atomen und
$R^c$ = -H oder $-CH_3$ sind, mit der Massgabe, dass $R^a$ und $R^c$ nicht gleichzeitig $-CH_3$ sind,

und

$R^{6a}$ = Alkyl mit 1 bis 18 C-Atomen, Phenyl, mit einer oder mehreren Alkylgruppen mit insgesamt 1 bis 24 C-Atomen substituiertes Phenyl oder $C_5$-$C_8$-Cycloalkyl ist, oder,
wenn n = 2 ist,
A =

$-O-C_xH_{2x}-O-$,
$-O-(CH_2CH_2O)_aCH_2CH_2O-$ ,
$-O-CH_2-CH_2-B-CH_2-CH_2-O-$ ,

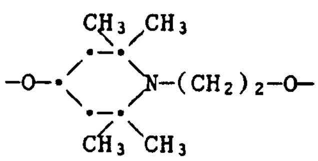 , 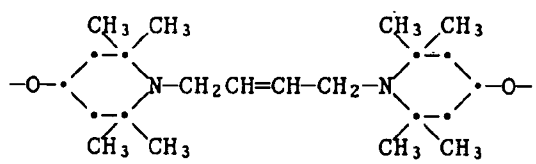 ,

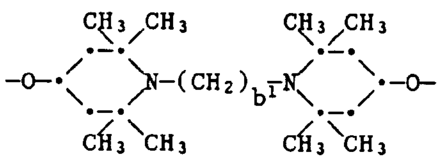 ,

$-O-CH_2CH{=}CHCH_2-O-$ , $-O-CH_2C{\equiv}CCH_2-O-$ , 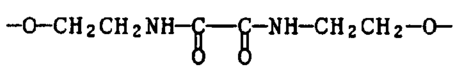 ,

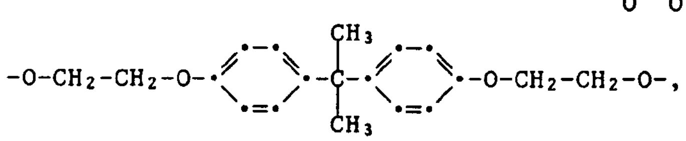

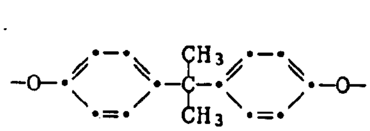 , 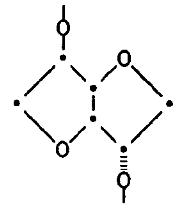 oder 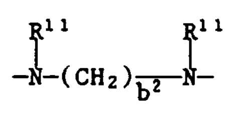 ist,

wobei
a eine Zahl von 1 bis 30 ist,
x eine Zahl von 2 bis 20 ist,
B = -S-,

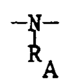 oder 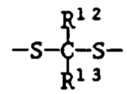

ist,

$R_A$ = Alkyl mit 1 bis 20 C-Atomen, Phenyl oder mit einer oder mehreren Alkylgruppen mit insgesamt 1 bis 20 C-Atomen substituiertes Phenyl, Cyclohexyl oder

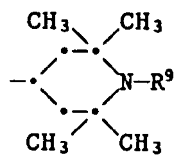

ist, und

$R^{11}$, $R^{12}$ und $R^{13}$, unabhängig voneinander, -H, Alkyl mit 1 bis 12 C-Atomen oder Phenyl sind oder $R^{12}$ und $R^{13}$, mit dem C-Atom, an das sie gebunden sind, einen Cycloalkylring mit 5 bis 12 C-Atomen bilden,

$b^1$ eine Zahl von 2 bis 10 ist, und

$b^2$ eine Zahl von 0 bis 6 darstellt,

oder wenn n = 3 ist,

A =

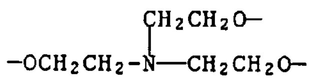 oder 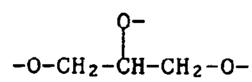

bedeutet,

oder wenn n = 4 ist

A =

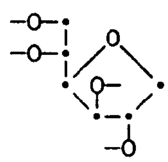 oder 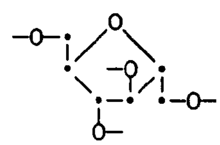

bedeutet,

oder wenn n = 6 ist,

A =

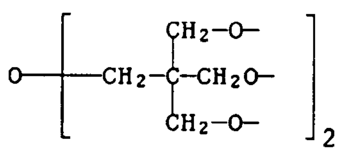

bedeutet.

**2.** Zusammensetzungen nach Anspruch 1, enthaltend mindestens eine Verbindung der Formel I, wobei

$R^1$ = Alkyl mit 1 bis 18 C-Atomen, Cycloalkyl mit 5 bis 12 C-Atomen, Phenyl oder Benzyl,

$R^2$ = -H, Alkyl mit 1 bis 18 C-Atomen, Cycloalkyl mit 5 bis 12 C-Atomen, Phenyl oder Benzyl und

$R^3$ = -H bedeuten und

n eine Zahl von 1 bis 4 oder 6 bedeutet, wobei,

wenn n = 1 ist,

A = $-OR^4$ oder

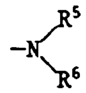

ist, und

$R^4$ die Bedeutung von -H, Alkyl mit 1 bis 20 C-Atomen, Cyclohexyl, Alkenyl mit 2 bis 18 C-Atomen oder $-CH_2CH_2-XR^{5a}$ hat, und

$R^5$ -H, Alkyl mit 1 bis 12 C-Atomen, Phenyl, Cyclohexyl, $C_1$-$C_4$ alkylsubstituiertes Cycloalkyl mit 5 bis 12 C-Atomen, Alkenyl mit 3 bis 8 C-Atomen Benzyl oder $-NH_2$ bedeutet, und

$R^6$ -H oder Alkyl mit 1 bis 12 C-Atomen bedeutet,

X = -O-, -S- oder

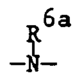

ist,

$R^{5a}$ = -H, Alkyl mit 1-12 C-Atomen, Phenyl, Cyclohexyl oder

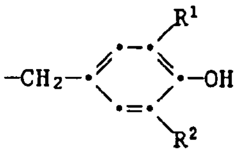

ist,

und

$R^{6a}$ = Alkyl mit 1 bis 12 C-Atomen, Phenyl oder mit einer oder mehreren Alkylgruppen mit insgesamt 1 bis 18 C-Atomen substituiertes Phenyl ist, oder,

wenn n = 2 ist,

A =

$$-O-C_xH_{2x}-O-,$$
$$-O-(CH_2CH_2O)_aCH_2CH_2O-,$$
$$-O-CH_2-CH_2-B-CH_2-CH_2O-,$$

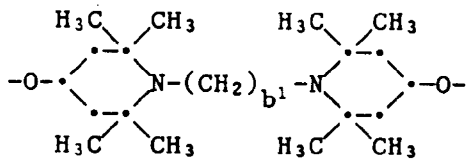

$-O-CH_2CH=CHCH_2-O-$ , 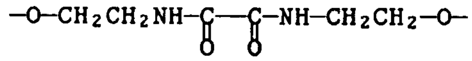 ,

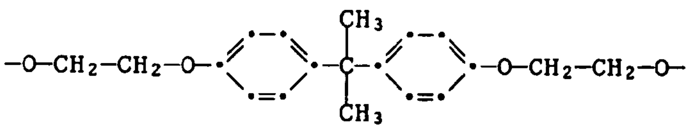 ,

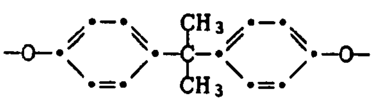 , 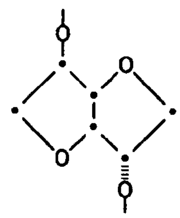 oder 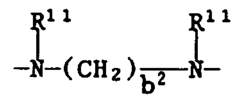

ist,

wobei

a eine Zahl von 1 bis 12 ist,

x eine Zahl von 2 bis 12 ist,

B = -S- oder

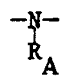

ist,

$R_A$ = Alkyl mit 1 bis 12 C-Atomen, Phenyl oder mit einer oder mehreren Alkylgruppen mit insgesamt 1 bis 18 C-Atomen substituiertes Phenyl, oder Cyclohexyl ist, und

$R^{11}$, -H, Alkyl mit 1 bis 12 C-Atomen oder Phenyl ist und

$b^1$ eine Zahl von 2 bis 6 ist, und

$b^2$ eine Zahl von 0 bis 6 darstellt,

oder wenn n = 3 ist,

A =

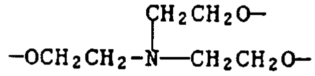 oder 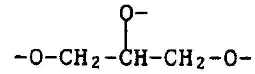

bedeutet,

oder wenn n = 4 ist

A =

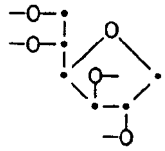 oder 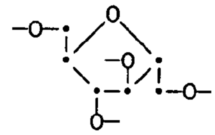

bedeutet,

oder wenn n = 6 ist,
A =

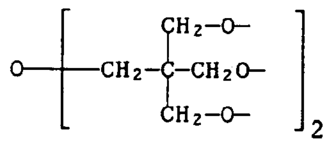

bedeutet.

3. Zusammensetzungen nach Anspruch 1, enthaltend mindestens eine Verbindung der Formel I, wobei
$R^1$ = Alkyl mit 1 bis 8 C-Atomen, Cyclohexyl oder Phenyl,
$R^2$ = -H, Alkyl mit 1 bis 8 C-Atomen, Cyclohexyl oder Phenyl und
$R^3$ = -H darstellen und
n = 1 ist und
A = $-OR^4$ oder

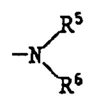

darstellt, wobei
$R^4$ = -H, Alkyl mit 1 bis 18 C-Atomen, Cyclohexyl, $-CH_2CH=CH_2$, $-(CH_2)_7-CH=CH-(CH_2)_7CH_3$,

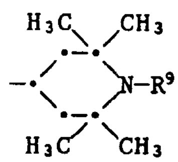

oder $-CH_2CH_2XR^{5a}$ bedeutet, wobei
$R^9$ = -H, Alkyl mit 1 bis 4 C-Atomen, -O·, -O-Alkyl mit 1 bis 4 C-Atomen oder Cyclohexyl ist,
x = -O-, -S- oder

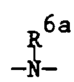

ist,
$R^{5a}$ = -H, Alkyl mit 1 bis 18 C-Atomen, Phenyl,

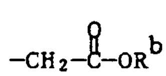,

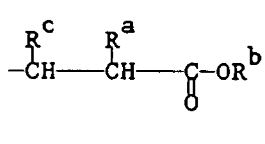 oder 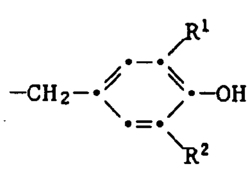

ist, wobei
$R^b$ =Alkyl mit 1 bis 24 C-Atomen ist,
$R^a$ = -H oder $-CH_3$ ist,
$R^c$ = -H ist und

$R^{6a}$ = Alkyl mit 1 bis 12 C-Atomen oder Phenyl ist, und
$R^5$ und $R^6$, unabhängig voneinander, -H, Alkyl mit 1 bis 12 C-Atomen, Phenyl
oder

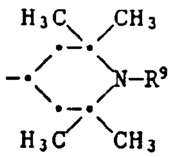

sind,
wobei $R^9$ die oben angegebene Bedeutung hat, oder
$R^5$ $-NH_2$ und $R^6$ -H bedeutet,
oder,
n gleich 2 ist, wobei
A = $-O-C_xH_{2x}-O-$ bedeutet und
x = 2 bis 8 ist.

**4.** Zusammensetzung gemäss Anspruch 1, enthaltend mindestens eine Verbindung der Formel I, wobei
$R^1$ = tert.-Butyl
$R^2$ = -H, Methyl oder tert.-Butyl und
$R^3$ = -H darstellen und
n gleich 1 ist, wobei
A = $-OR^4$ oder

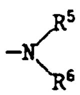

darstellt, und
$R^4$ = Alkyl mit 1 bis 18 C-Atomen, $-(CH_2)_7-CH=CH-(CH_2)_7CH_3$,

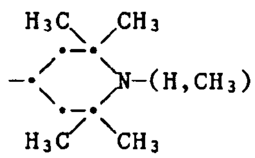

oder
$-CH_2CH_2-SR^{5a}$ ist, wobei
$R^{5a}$ = -H oder

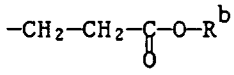

bedeutet, wobei
$R^b$ = n-$C_4H_9$ bis n-$C_8H_{17}$ oder tert-$C_4C_9$ bis tert. $C_8H_{17}$ ist,
oder
n gleich 2 ist, wobei
A = $-O-C_xH_{2x}-O-$ und
x = 2 bis 8 ist, oder
A = $-O-(CH_2-CH_2-O-)_a-CH_2-CH_2-O-$ und
a = 1 bis 4 ist, oder
A = $-O-CH_2-CH_2-B-CH_2-CH_2-O-$ und
B = -S- ,

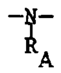 oder 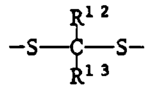

ist, wobei

$R_A$ = $C_4$-$C_8$-Alkyl oder Phenyl,
$R^{12}$ = H, $C_1$-$C_8$-Alkyl und
$R^{13}$ = H, $C_1$-$C_8$-Alkyl oder Phenyl darstellen, oder
A =

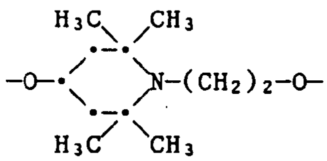 , 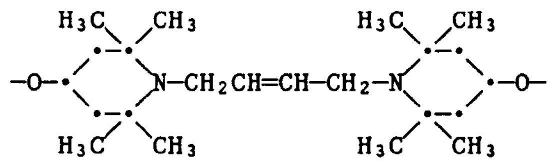 oder

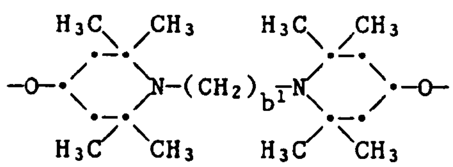 ist, wobei

$b^1$ = 2 bis 6 ist, oder
A =

-O-$CH_2$-CH=CH-$CH_2$-O- ,
-O-$CH_2$-C≡C-$CH_2$-O- ,

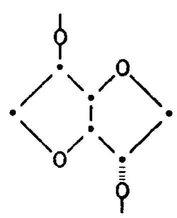 , 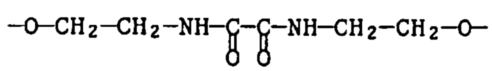 oder 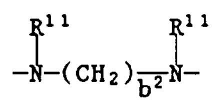

ist,
wobei
$b^2$ = 0 bis 6 und
$R^{11}$ = -H oder $C_1$-$C_8$-Alkyl darstellen.

5. Zusammensetzungen nach Anspruch 1, enthaltend mindestens eine Verbindung der Formel I, wobei
$R^1$ = tert.-Butyl
$R^2$ = -H, $-CH_3$ oder tert.-Butyl und
$R^3$ = -H darstellen und
n = 1 ist und dabei
A = $-OR^4$ bedeutet und
$R^4$ = $C_1$-$C_{18}$-Alkyl ist.

6. Zusammensetzungen nach Anspruch 1, enthaltend mindestens eine Verbindung der Formel I, wobei
$R^1$ = tert.-Butyl
$R^2$ = -H, $-CH_3$ oder tert.-Butyl und
$R^3$ = -H darstellen und
n = 2 ist und dabei
A = -O-$C_xH_{2x}$-O- und
x = 2 bis 6 ist, oder
A = -O-$(CH_2-CH_2-O)_a$-$CH_2$-$CH_2$-O- und

a = 1, 2 oder 3 ist,
oder
A = O-$CH_2$-$CH_2$-B-$CH_2$-$CH_2$-O- und
B = -S- oder

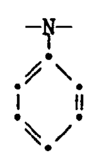

ist,
oder
A =

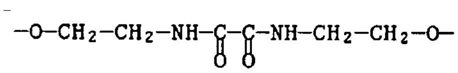

oder
A = -NH-NH- ist.

**7.** Zusammensetzung nach Anspruch 1, enthaltend mindestens eine Verbindung der Formel I, wobei
$R^1$ = tert.-Butyl,
$R^2$ = -H, -$CH_3$ oder tert.-Butyl
$R^3$ = -H darstellen,
n = 1 ist und dabei
A = -$OR^4$ darstellt, wobei
$R^4$ = $C_1$-$C_{18}$-Alkyl ist, oder
n = 2 ist und dabei
A = -O-$C_xH_{2x}$-O- und x = 2-6,
-$OCH_2CH_2$-S-$CH_2CH_2O$- oder
-$O(CH_2CH_2O)_aCH_2CH_2$-O- und
a = 1 bis 4 sind.

**8.** Zusammensetzung nach Anspruch 7, enthaltend mindestens eine Verbindung der Formel I, wobei a = 1 oder 2 ist.

**9.** Zusammensetzung nach Anspruch 1, enthaltend mindestens eine Verbindung der Formel I, wobei
$R^1$ = tert.-Butyl
$R^2$ = -H, -$CH_3$, tert.-Butyl und
$R^3$ = -H darstellen und
n gleich 3 ist und dabei
A =

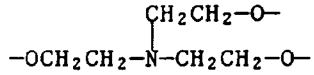

darstellt,
oder
n = 4 ist und dabei
A =

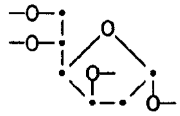

darstellt,

oder

n = 6 ist und dabei

A =

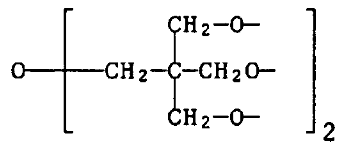

bedeutet.

**10.** Zusammensetzung nach Anspruch 1, enthaltend mindestens eine Verbindung aus der Reihe der Formeln

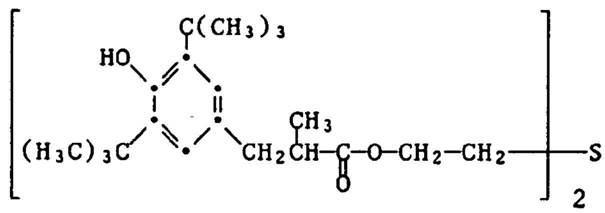

,

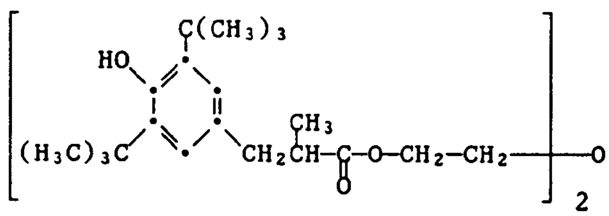

,

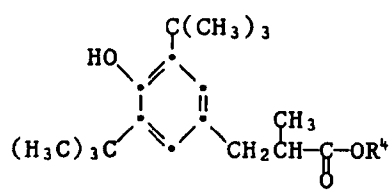

,

wobei

$R^4$ die Bedeutung von $C_1$-$C_{18}$-Alkyl hat,

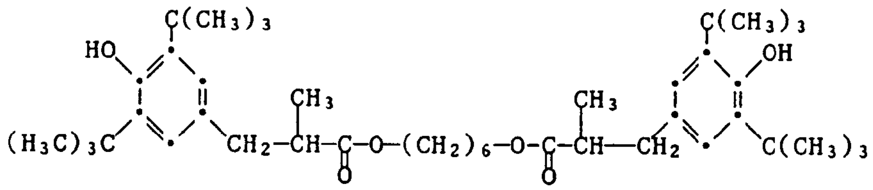

und

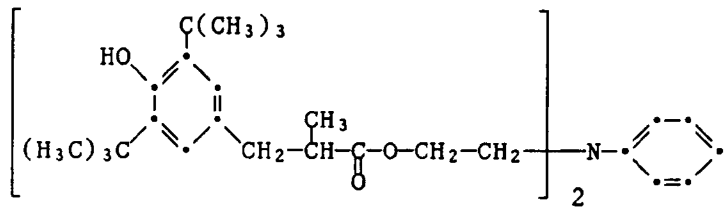

.

**11.** Zusammensetzung nach Anspruch 1, enthaltend
a) eine funktionelle Flüssigkeit aus der Reihe der Schmierstoffe, der Hydraulikflüssigkeiten und der Metallbearbeitungsflüssigkeiten oder ein synthetisches Polymer und
b) wenigstens eine Verbindung der Formel I nach Anspruch 1.

**12.** Zusammensetzung nach Anspruch 1, enthaltend
a) einen Schmierstoff aus der Reihe der Mineralöle, der synthetischen Oele oder einer Mischung davon und
b) wenigstens eine Verbindung der Formel I nach Anspruch 1.

**13.** Zusammensetzung nach Anspruch 1, enthaltend wenigstens eine Verbindung nach Anspruch 10.

**14.** Zusammensetzung nach Anspruch 1, enthaltend
a) ein synthetisches Polymer und
b) wenigstens eine Verbindung der Formel I nach Anspruch 1.

**15.** Zusammensetzung nach Anspruch 14, enthaltend
a) ein Polyolefin und
b) wenigstens eine Verbindung der Formel I nach Anspruch 1.

**16.** Verbindungen der in Anspruch 1 definierten Formel I mit der Maßgabe, daß $R^4$ nicht $C_1$-$C_4$-Alkyl bedeutet.

**17.** Verbindungen nach Anspruch 16, wobei $R^1$, $R^2$, $R^3$, A und n die in Anspruch 2 angegebenen Bedeutungen haben.

**18.** Verbindungen nach Anspruch 16, wobei $R^1$, $R^2$, $R^3$, A und n die in Anspruch 3 angegebenen Bedeutungen haben.

**19.** Verbindungen nach Anspruch 16, wobei $R^1$, $R^2$, $R^3$, A und n die in Anspruch 4 angegebenen Bedeutungen haben.

**20.** Verbindungen nach Anspruch 16, wobei $R^1$, $R^2$, $R^3$, A und n die in Anspruch 5 angegebenen Bedeutungen haben.

**21.** Verbindungen nach Anspruch 16, wobei $R^1$, $R^2$, $R^3$, A und n die in Anspruch 6 angegebenen Bedeutungen haben.

**22.** Verbindungen nach Anspruch 16, wobei $R^1$, $R^2$, $R^3$, A und n die in Anspruch 7 angegebenen Bedeutungen haben.

**23.** Verbindungen nach Anspruch 16, wobei $R^1$, $R^2$, $R^3$, A und n die in Anspruch 8 angegebenen Bedeutungen haben.

24. Verbindungen nach Anspruch 16, wobei $R^1$, $R^2$, $R^3$, A und n die in Anspruch 9 angegebenen Bedeutungen haben.

25. Verbindungen nach Anspruch 16, der in Anspruch 10 angegebenen Formeln.

26. Verbindungen nach Anspruch 16, wobei $R^4$ Alkyl mit 9 bis 18 C-Atomen ist.

27. Verwendung der Verbindungen gemäß Anspruch 16 als Antioxidantien in organischen, gegen oxidativen, thermischen oder actinischen Abbau empfindlichen, Materialien.

28. Verwendung gemäß Anspruch 27 in Schmierstoffen.

**Patentansprüche für folgenden Vertragsstaat : ES**

1. Zusammensetzung, enthaltend
   a) mindestens ein dem oxidativen, thermischen oder actinischen Abbau unterworfenes organisches Material und
   b) mindestens eine Verbindung der allgemeinen Formel I

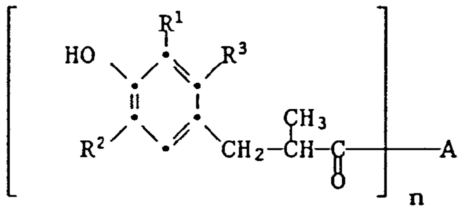

(I),

wobei

$R^1$ = Alkyl mit 1 bis 18 C-Atomen, Cycloalkyl mit 5 bis 12 C-Atomen, Phenyl oder Aralkyl mit 7 bis 9 C-Atomen,

$R^2$ = -H, Alkyl mit 1 bis 18 C-Atomen, Cycloalkyl mit 5 bis 12 C-Atomen, Phenyl oder Aralkyl mit 7 bis 9 C-Atomen und

$R^3$ = -H oder $CH_3$ bedeutet und

n eine Zahl von 1 bis 4 oder 6 bedeutet, wobei,

wenn n = 1 ist,

A = $-OR^4$ oder

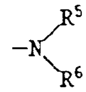

ist, und

$R^4$ die Bedeutung von -H, Alkyl mit 1 bis 45 C-Atomen, Cycloalkyl mit 5 bis 12 C-Atomen, Alkenyl mit 2 bis 18 C-Atomen,

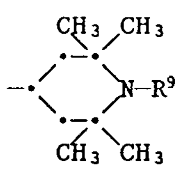

oder $-CH_2CH_2-XR^{5a}$ hat, und

$R^5$ und $R^6$, unabhängig voneinander, -H, Alkyl mit 1 bis 20 C-Atomen, Phenyl, Cycloalkyl mit 5 bis 12 C-Atomen, $C_1$-$C_4$ alkylsubstituiertes Cycloalkyl mit 5 bis 12 C-Atomen, Alkenyl mit 3 bis 8 C-Atomen, Aralkyl mit 7 bis 10 C-Atomen oder

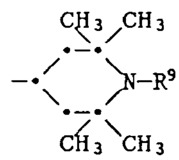

bedeuten, und

$R^5$ zusätzlich $-NH_2$ bedeutet,

$R^9$ = -H, Alkyl mit 1 bis 8 C-Atomen,

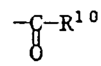 ,

-O· oder $-OR^{9}$ 'ist, wobei

$R^{9}$ ' = -H, Alkyl mit 1-25 C-Atomen oder

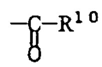

darstellt,

X = -O, -S- oder

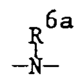

ist,

$R^{5a}$ =

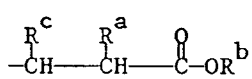 , 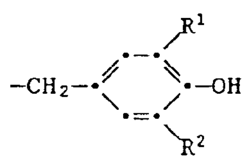 ,

-H, Alkyl mit 1-24 C-Atomen, Phenyl, Cycloalkyl mit 5 bis 12 C-Atomen oder

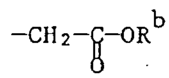

ist,

und

$R^{10}$ = Alkyl mit 1 bis 20 C-Atomen ist,

$R^a$ = -H oder $-CH_3$,

$R^b$ = -H oder Alkyl mit 1 bis 24 C-Atomen und

$R^c$ = -H oder $-CH_3$ sind, mit der Massgabe, dass $R^a$ und $R^c$ nicht gleichzeitig $-CH_3$ sind,

und

$R^{6a}$ = Alkyl mit 1 bis 18 C-Atomen, Phenyl, mit einer oder mehreren Alkylgruppen mit insgesamt 1 bis 24 C-Atomen substituiertes Phenyl oder $C_5$-$C_8$-Cycloalkyl ist, oder,

wenn n = 2 ist,

A =

$$-O-C_xH_{2x}-O-,$$
$$-O-(CH_2CH_2O)_aCH_2CH_2O- ,$$
$$-O-CH_2-CH_2-B-CH_2-CH_2-O- ,$$

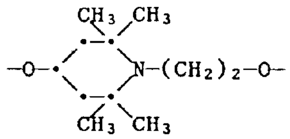 , 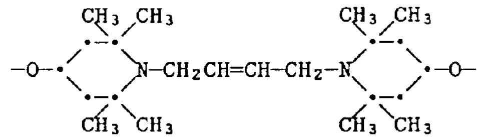 ,

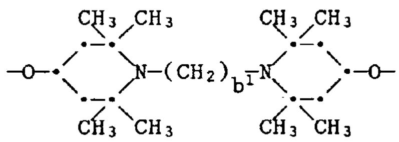 ,

$-O-CH_2CH=CHCH_2-O-$ , $-O-CH_2C\equiv CCH_2-O-$ , 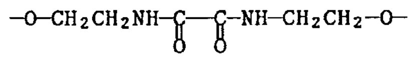 ,

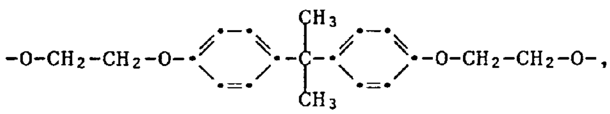

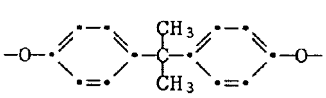 , 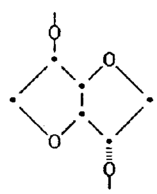 oder 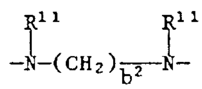 ist,

wobei

a eine Zahl von 1 bis 30 ist,

x eine Zahl von 2 bis 20 ist,

B = -S-,

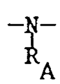 oder 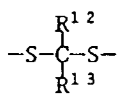

ist,

$R_A$ = Alkyl mit 1 bis 20 C-Atomen, Phenyl oder mit einer oder mehreren Alkylgruppen mit insgesamt 1 bis 20 C-Atomen substituiertes Phenyl, Cyclohexyl oder

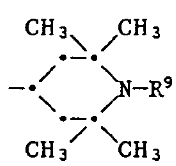

ist, und

$R^{11}$, $R^{12}$ und $R^{13}$, unabhängig voneinander, -H, Alkyl mit 1 bis 12 C-Atomen oder Phenyl sind oder

$R^{12}$ und $R^{13}$, mit dem C-Atom, an das sie gebunden sind, einen Cycloalkylring mit 5 bis 12 C-Atomen bilden,

$b^1$ eine Zahl von 2 bis 10 ist, und

$b^2$ eine Zahl von 0 bis 6 darstellt,

oder wenn n = 3 ist,

A =

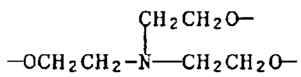 oder 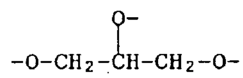

bedeutet,

oder wenn n = 4 ist

A =

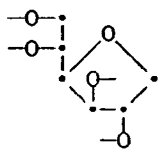 oder 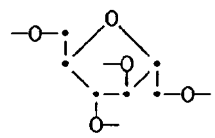

bedeutet,

oder wenn n = 6 ist,

A =

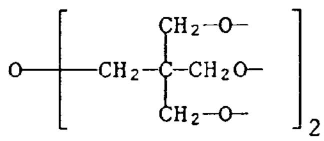

bedeutet.

**2.** Zusammensetzungen nach Anspruch 1, enthaltend mindestens eine Verbindung der Formel I, wobei

$R^1$ = Alkyl mit 1 bis 18 C-Atomen, Cycloalkyl mit 5 bis 12 C-Atomen, Phenyl oder Benzyl,

$R^2$ = -H, Alkyl mit 1 bis 18 C-Atomen, Cycloalkyl mit 5 bis 12 C-Atomen, Phenyl oder Benzyl und

$R^3$ = -H bedeuten und

n eine Zahl von 1 bis 4 oder 6 bedeutet, wobei,

wenn n = 1 ist,

A = $-OR^4$ oder

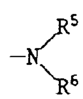

ist, und

$R^4$ die Bedeutung von -H, Alkyl mit 1 bis 20 C-Atomen, Cyclohexyl, Alkenyl mit 2 bis 18 C-Atomen oder $-CH_2CH_2-XR^{5a}$ hat, und

$R^5$ -H, Alkyl mit 1 bis 12 C-Atomen, Phenyl, Cyclohexyl, $C_1$-$C_4$ alkylsubstituiertes Cycloalkyl mit 5 bis 12 C-Atomen, Alkenyl mit 3 bis 8 C-Atomen Benzyl oder $-NH_2$ bedeutet, und

$R^6$ -H oder Alkyl mit 1 bis 12 C-Atomen bedeutet,

X = -O-, -S- oder

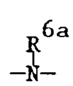

ist,

$R^{5a}$ = -H, Alkyl mit 1-12 C-Atomen, Phenyl, Cyclohexyl oder

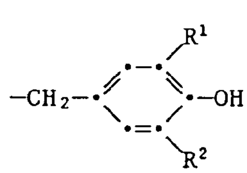

ist,
und

$R^{6a}$ = Alkyl mit 1 bis 12 C-Atomen, Phenyl oder mit einer oder mehreren Alkylgruppen mit insgesamt 1 bis 18 C-Atomen substituiertes Phenyl ist, oder,

wenn n = 2 ist,

A =

$$-O-C_xH_{2x}-O-,$$
$$-O-(CH_2CH_2O)_aCH_2CH_2O-\ ,$$
$$-O-CH_2-CH_2-B-CH_2-CH_2O-\ ,$$

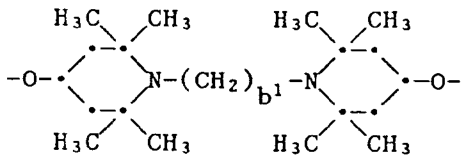

$-O-CH_2CH{=}CHCH_2-O-$ , 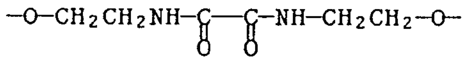 ,

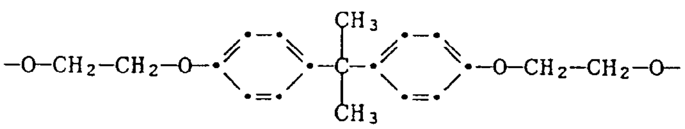 ,

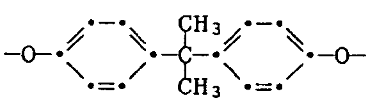 , 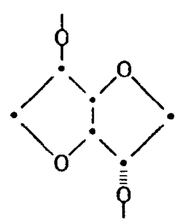 oder 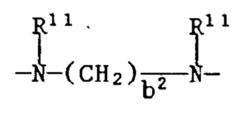

ist,
wobei

a eine Zahl von 1 bis 12 ist,

x eine Zahl von 2 bis 12 ist,

B = -S- oder

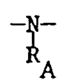

ist,

$R_A$ = Alkyl mit 1 bis 12 C-Atomen, Phenyl oder mit einer oder mehreren Alkylgruppen mit insgesamt 1 bis 18 C-Atomen substituiertes Phenyl, oder Cyclohexyl ist, und

$R^{11}$, -H, Alkyl mit 1 bis 12 C-Atomen oder Phenyl ist und

$b^1$ eine Zahl von 2 bis 6 ist, und

$b^2$ eine Zahl von 0 bis 6 darstellt,

oder wenn n = 3 ist,

A =

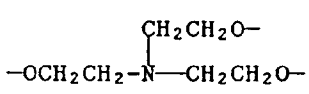 oder 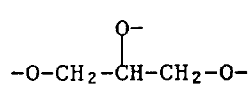

bedeutet,

oder wenn n = 4 ist

A =

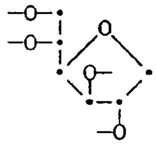 oder 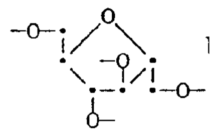

bedeutet,

oder wenn n = 6 ist,

A =

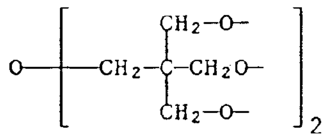

bedeutet.

3. Zusammensetzungen nach Anspruch 1, enthaltend mindestens eine Verbindung der Formel I, wobei

$R^1$ = Alkyl mit 1 bis 8 C-Atomen, Cyclohexyl oder Phenyl,

$R^2$ = -H, Alkyl mit 1 bis 8 C-Atomen, Cyclohexyl oder Phenyl und

$R^3$ = -H darstellen und

n = 1 ist und

A = $-OR^4$ oder

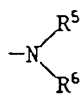

darstellt, wobei

$R^4$ = -H, Alkyl mit 1 bis 18 C-Atomen, Cyclohexyl, $-CH_2CH{=}CH_2$, $-(CH_2)_7-CH{=}CH-(CH_2)_7CH_3$,

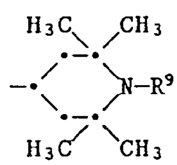

oder $-CH_2CH_2XR^{5a}$ bedeutet, wobei

$R^9$ = -H, Alkyl mit 1 bis 4 C-Atomen, -O·, -O-Alkyl mit 1 bis 4 C-Atomen oder Cyclohexyl ist,

X = -O-, -S- oder

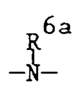

ist,

$R^{5a}$ = -H, Alkyl mit 1 bis 18 C-Atomen, Phenyl,

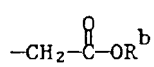,

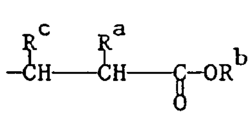 oder 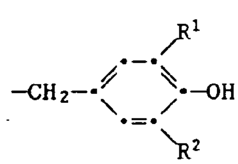

ist, wobei
$R^b$ = Alkyl mit 1 bis 24 C-Atomen ist,
$R^a$ = -H oder $-CH_3$ ist,
$R^c$ = -H ist und
$R^{6a}$ = Alkyl mit 1 bis 12 C-Atomen oder Phenyl ist, und
$R^5$ und $R^6$, unabhängig voneinander, -H, Alkyl mit 1 bis 12 C-Atomen, Phenyl oder

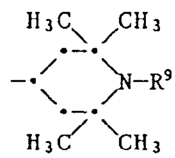

sind,
wobei $R^9$ die oben angegebene Bedeutung hat, oder
$R^5$ $-NH_2$ und $R^6$ -H bedeutet,
oder,
n gleich 2 ist, wobei
A = $-O-C_xH_{2x}-O-$ bedeutet und
x = 2 bis 8 ist.

**4.** Zusammensetzung gemäss Anspruch 1, enthaltend mindestens eine Verbindung der Formel I, wobei
$R^1$ = tert.-Butyl
$R^2$ = -H, Methyl oder tert.-Butyl und
$R^3$ = -H darstellen und
n gleich 1 ist, wobei
A = $-OR^4$ oder

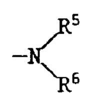

darstellt, und
$R^4$ = Alkyl mit 1 bis 18 C-Atomen, $-(CH_2)_7-CH=CH-(CH_2)_7CH_3$,

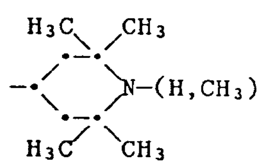

oder
$-CH_2CH_2-SR^{5a}$ ist, wobei
$R^{5a}$ = -H oder

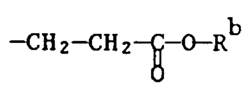

bedeutet, wobei
$R^b$ = $n-C_4H_9$ bis $n-C_8H_{17}$ oder $tert-C_4H_9$ bis tert. $C_8H_{17}$ ist, oder
n gleich 2 ist, wobei
A = $-O-C_xH_{2x}-O-$ und
x = 2 bis 8 ist, oder
A = $-O-(CH_2-CH_2-O-)_a-CH_2-CH_2-O-$ und

a = 1 bis 4 ist, oder
A = $-O-CH_2-CH_2-B-CH_2-CH_2-O-$ und
B = -S-,

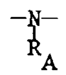 oder 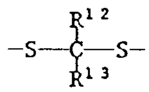

ist, wobei
$R_A$ = $C_4$-$C_8$-Alkyl oder Phenyl,
$R^{12}$ = H, $C_1$-$C_8$-Alkyl und
$R^{13}$ = H, $C_1$-$C_8$-Alkyl oder Phenyl darstellen, oder
A =

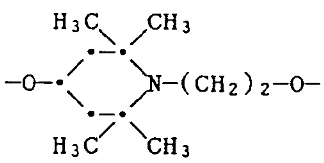 , 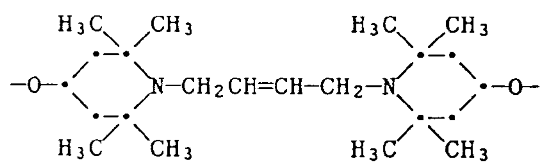 oder

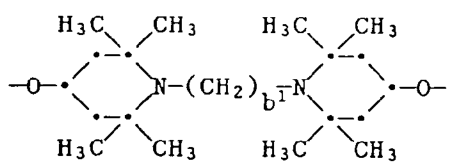 ist, wobei

$b^1$ = 2 bis 6 ist, oder
A =

$-O-CH_2-CH=CH-CH_2-O-$ ,
$-O-CH_2-C{\equiv}C-CH_2-O-$ ,

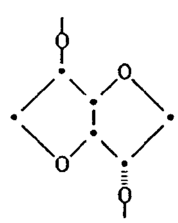 , 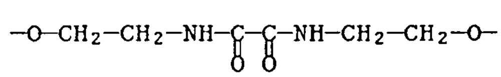 oder 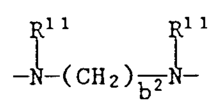

ist, wobei
$b^2$ = 0 bis 6 und
$R^{11}$ = -H oder $C_1$-$C_8$-Alkyl darstellen.

5. Zusammensetzungen nach Anspruch 1, enthaltend mindestens eine Verbindung der Formel I, wobei
$R^1$ = tert.-Butyl
$R^2$ = -H, $-CH_3$ oder tert.-Butyl und
$R^3$ = -H darstellen und
n = 1 ist und dabei
A = $-OR^4$ bedeutet und
$R^4$ = $C_1$-$C_{18}$-Alkyl ist.

6. Zusammensetzungen nach Anspruch 1, enthaltend mindestens eine Verbindung der Formel I, wobei
$R^1$ = tert.-Butyl
$R^2$ = -H, $-CH_3$ oder tert.-Butyl und
$R^3$ = -H darstellen und

n = 2 ist und dabei
A = $-O-C_xH_{2x}-O-$ und
x = 2 bis 6 ist, oder
A = $-O-(CH_2-CH_2-O)_a-CH_2-CH_2-O-$ und
a = 1, 2 oder 3 ist,
oder
A = $-O-CH_2-CH_2-B-CH_2-CH_2-O-$ und
B = -S- oder

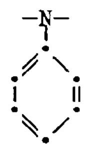

ist,
oder
A =

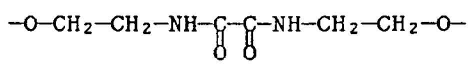

oder
A = -NH-NH- ist.

7. Zusammensetzung nach Anspruch 1, enthaltend mindestens eine Verbindung der Formel I, wobei
$R^1$ = tert.-Butyl,
$R^2$ = -H, $-CH_3$ oder tert.-Butyl
$R^3$ = -H darstellen,
n = 1 ist und dabei
A = $-OR^4$ darstellt, wobei
$R^4$ = $C_1$-$C_{18}$-Alkyl ist, oder
n = 2 ist und dabei
A = $-O-C_xH_{2x}-O-$ und x = 2-6,
$-OCH_2CH_2-S-CH_2CH_2O-$ oder
$-O(CH_2CH_2O)_aCH_2CH_2-O-$ und
a = 1 bis 4 sind.

8. Zusammensetzung nach Anspruch 7, enthaltend mindestens eine Verbindung der Formel I, wobei a = 1 oder 2 ist.

9. Zusammensetzung nach Anspruch 1, enthaltend mindestens eine Verbindung der Formel I, wobei
$R^1$ = tert.-Butyl
$R^2$ = -H, $-CH_3$, tert.-Butyl und
$R^3$ = -H darstellen und
n gleich 3 ist und dabei
A =

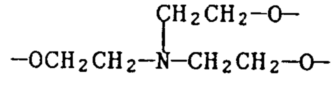

darstellt,
oder
n = 4 ist und dabei
A =

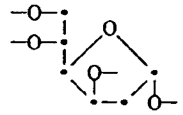

darstellt,

oder

n = 6 ist und dabei

A =

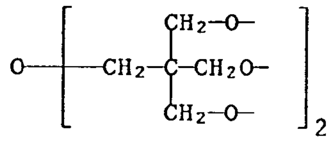

bedeutet.

**10.** Zusammensetzung nach Anspruch 1, enthaltend mindestens eine Verbindung aus der Reihe der Formeln

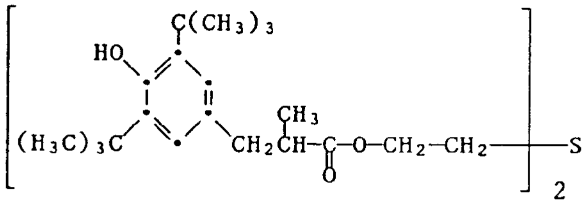

,

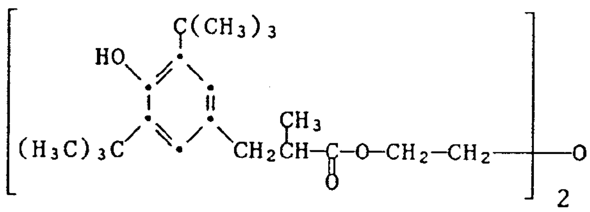

,

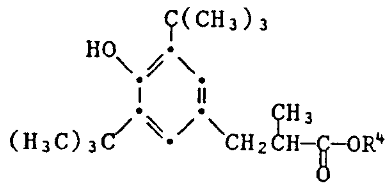

,

wobei

$R^4$ die Bedeutung von $C_1$-$C_{18}$-Alkyl hat,

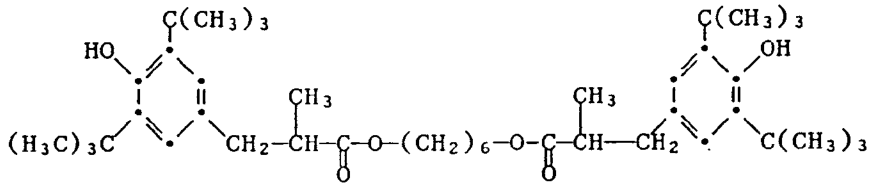

und

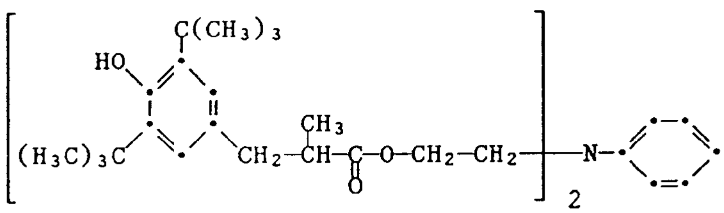

.

**11.** Zusammensetzung nach Anspruch 1, enthaltend
a) eine funktionelle Flüssigkeit aus der Reihe der Schmierstoffe, der Hydraulikflüssigkeiten und der Metallbearbeitungsflüssigkeiten oder ein synthetisches Polymer und
b) wenigstens eine Verbindung der Formel I nach Anspruch 1.

**12.** Zusammensetzung nach Anspruch 1, enthaltend
a) einen Schmierstoff aus der Reihe der Mineralöle, der synthetischen Oele oder einer Mischung davon und
b) wenigstens eine Verbindung der Formel I nach Anspruch 1.

**13.** Zusammensetzung nach Anspruch 1, enthaltend wenigstens eine Verbindung nach Anspruch 10.

**14.** Zusammensetzung nach Anspruch 1, enthaltend
a) ein synthetisches Polymer und
b) wenigstens eine Verbindung der Formel I nach Anspruch 1.

**15.** Zusammensetzung nach Anspruch 14, enthaltend
a) ein Polyolefin und
b) wenigstens eine Verbindung der Formel I nach Anspruch 1.

**16.** Verwendung der Verbindungen der Formel I gemäss Anspruch 1 als Antioxidantien in organischen, gegen oxidativen, thermischen oder actinischen Abbau empfindlichen, Materialien.

**17.** Verwendung der Verbindungen der Formel I gemäss Anspruch 1 als Antioxidantien in Schmierstoffen.

## Claims

### Claims for the following Contracting States : BE, DE, FR, GB, IT, NL

**1.** A composition comprising
a) at least one organic material subject to oxidative, thermal or actinic degradation and
b) at least one compound of general formula I

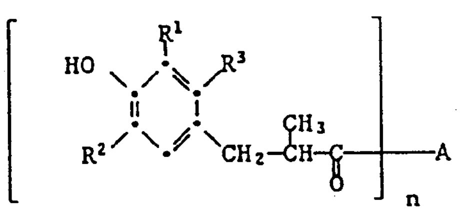

(I),

wherein

$R^1$ is alkyl having from 1 to 18 carbon atoms, cycloalkyl having from 5 to 12 carbon atoms, phenyl or aralkyl having from 7 to 9 carbon atoms,

$R^2$ is -H, alkyl having from 1 to 18 carbon atoms, cycloalkyl having from 5 to 12 carbon atoms, phenyl or aralkyl having from 7 to 9 carbon atoms, and

$R^3$ is -H or $CH_3$, and

n is a number from 1 to 4 or 6, and,

when n is 1,

A is $-OR^4$ or

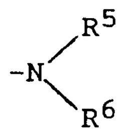,

and

$R^4$ is -H, alkyl having from 1 to 45 carbon atoms, cycloalkyl having from 5 to 12 carbon atoms, alkenyl having from 2 to 18 carbon atoms,

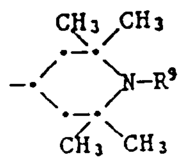

or $-CH_2CH_2-XR^{5a}$, and

each of $R^5$ and $R^6$, independently of the other, is -H, alkyl having from 1 to 20 carbon atoms, phenyl, cycloalkyl having from 5 to 12 carbon atoms,

$C_1$-$C_4$alkyl-substituted cycloalkyl having from 5 to 12 carbon atoms, alkenyl having from 3 to 8 carbon atoms, aralkyl having from 7 to 10 carbon atoms or

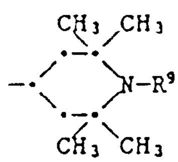,

and

$R^5$ may additionally be $-NH_2$,

$R^9$ is -H, alkyl having from 1 to 8 carbon atoms,

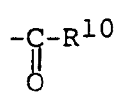,

-O· or $-OR^{9'}$, wherein

$R^{9'}$ is -H, alkyl having from 1 to 25 carbon atoms or

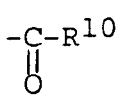,

X is -O-, -S- or

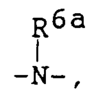

$R^{5a}$ is

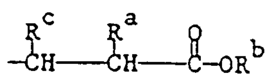 , 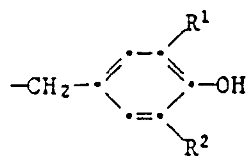 ,

-H, alkyl having from 1 to 24 carbon atoms, phenyl, cycloalkyl having from 5 to 12 carbon atoms or

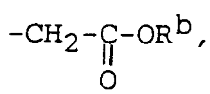

and

$R^{10}$ is alkyl having from 1 to 20 carbon atoms,

$R^a$ is -H or $-CH_3$,

$R^b$ is -H or alkyl having from 1 to 24 carbon atoms and

$R^c$ is -H or $-CH_3$, with the proviso that $R^a$ and $R^c$ are not $-CH_3$ at the same time,

and

$R^{6a}$ is alkyl having from 1 to 18 carbon atoms, phenyl, phenyl substituted by one or more alkyl groups having a total of from 1 to 24 carbon atoms, or $C_5$-$C_8$cycloalkyl, or,

when n is 2,

A is

$$-O-C_xH_{2x}-O-,$$
$$-O-(CH_2CH_2O)_aCH_2CH_2O-,$$
$$-O-CH_2-CH_2-B-CH_2-CH_2-O-,$$

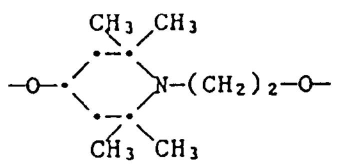 , 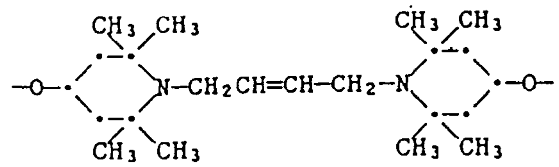 ,

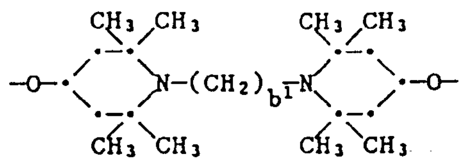 ,

$-O-CH_2CH=CHCH_2-O-$ , $-O-CH_2C\equiv CCH_2-O-$ , 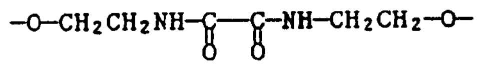 ,

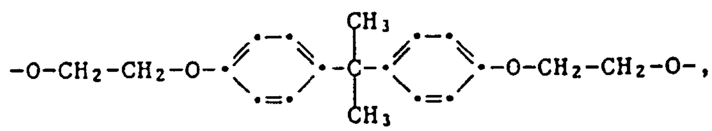

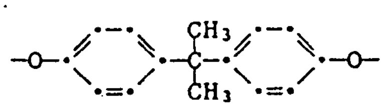 , 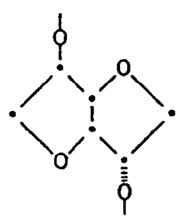 or 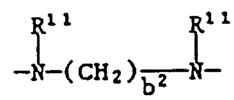 ,

wherein

a is a number from 1 to 30,

x is a number from 2 to 20,

B is -S- ,

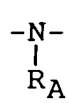 or 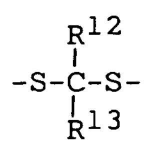 ,

$R_A$ is alkyl having from 1 to 20 carbon atoms, phenyl, phenyl substituted by one or more alkyl groups having a total of from 1 to 20 carbon atoms, cyclohexyl or

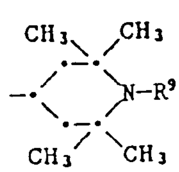 ,

and

each of $R^{11}$, $R^{12}$ and $R^{13}$, independently of the others, is -H, alkyl having from 1 to 12 carbon atoms or phenyl, or

$R^{12}$ and $R^{13}$ together with the carbon atom to which they are bonded form a cycloalkyl ring having from 5 to 12 carbon atoms,

$b^1$ is a number from 2 to 10 and

$b^2$ is a number from 0 to 6, or,

when n is 3,

A is

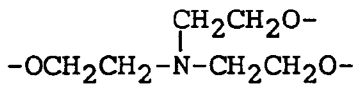 or 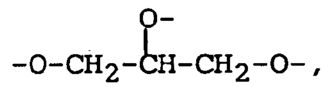,

or,

when n is 4,

A is

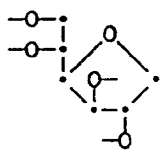 or 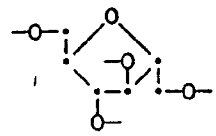,

or, when n is 6,

A is

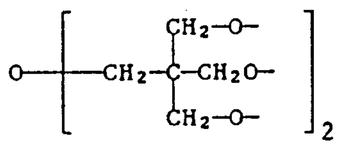.

2. A composition according to claim 1, comprising at least one compound of formula I wherein

$R^1$ is alkyl having from 1 to 18 carbon atoms, cycloalkyl having from 5 to 12 carbon atoms, phenyl or benzyl,

$R^2$ is -H, alkyl having from 1 to 18 carbon atoms, cycloalkyl having from 5 to 12 carbon atoms, phenyl or benzyl, and

$R^3$ is -H, and

n is a number from 1 to 4 or 6, wherein,

when n is 1,

A is $-OR^4$ or

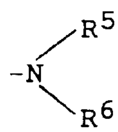,

and

$R^4$ is -H, alkyl having from 1 to 20 carbon atoms, cyclohexyl, alkenyl having from 2 to 18 carbon atoms or $-CH_2CH_2-XR^{5a}$, and

$R^5$ is -H, alkyl having from 1 to 12 carbon atoms, phenyl, cyclohexyl, $C_1$-$C_4$alkyl-substituted cycloalkyl having from 5 to 12 carbon atoms, alkenyl having from 3 to 8 carbon atoms, benzyl or $-NH_2$, and

$R^6$ is -H or alkyl having from 1 to 12 carbon atoms,

X is -O-, -S- or

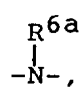,

$R^{5a}$ is -H, alkyl having from 1 to 12 carbon atoms, phenyl, cyclohexyl or

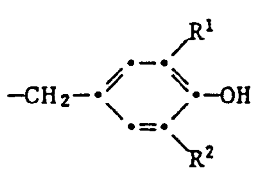,

and

$R^{6a}$ is alkyl having from 1 to 12 carbon atoms, phenyl, or phenyl substituted by one or more alkyl groups having a total of from 1 to 18 carbon atoms, or,

when n is 2,

A is

$$-O-C_xH_{2x}-O-,$$
$$-O-(CH_2CH_2O)_aCH_2CH_2O-,$$
$$-O-CH_2-CH_2-B-CH_2-CH_2O-,$$

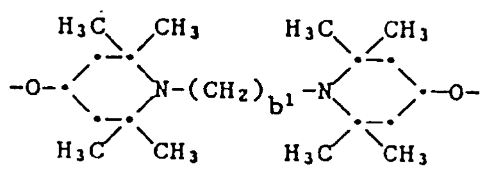

$-O-CH_2CH=CHCH_2-O-$ , 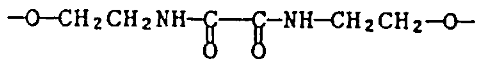 ,

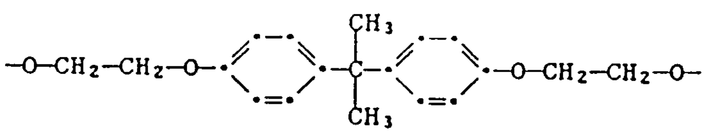 ,

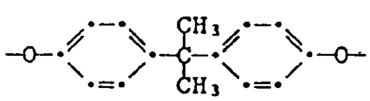 , 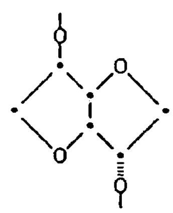 or 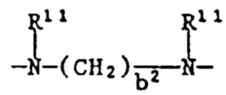 ,

wherein

a is a number from 1 to 12,

x is a number from 2 to 12,

B is -S- or

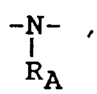 ,

$R_A$ is alkyl having from 1 to 12 carbon atoms, phenyl, phenyl substituted by one or more alkyl groups having a total of from 1 to 18 carbon atoms or cyclohexyl, and

$R^{11}$ is -H, alkyl having from 1 to 12 carbon atoms or phenyl, and

$b^1$ is a number from 2 to 6 and

$b^2$ is a number from 0 to 6, or,

when n is 3,

A is

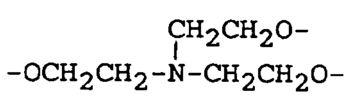 or 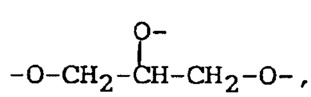 ,

or,

when n is 4,

A is

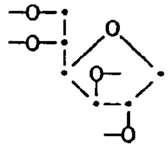 or 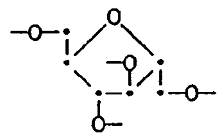 ,

or, when n is 6,
A is

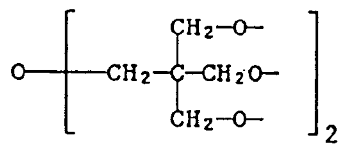 .

**3.** A composition according to claim 1, comprising at least one compound of formula I wherein
$R^1$ is alkyl having from 1 to 8 carbon atoms, cyclohexyl or phenyl,
$R^2$ is -H, alkyl having from 1 to 8 carbon atoms, cyclohexyl or phenyl, and
$R^3$ is -H, and
n is 1 and
A is $-OR^4$ or

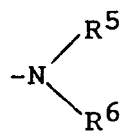 ,

wherein
$R^4$ is -H, alkyl having from 1 to 18 carbon atoms, cyclohexyl, $-CH_2CH=CH_2$, $-(CH_2)_7-CH=CH-(CH_2)_7CH_3$,

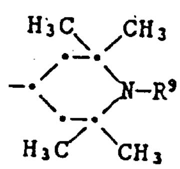

or $-CH_2CH_2XR^{5a}$, wherein
$R^9$ is -H, alkyl having from 1 to 4 carbon atoms, -O·, -O-alkyl having from 1 to 4 carbon atoms or cyclohexyl,
X is -O-, -S- or

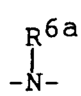 ,

$R^{5a}$ is -H, alkyl having from 1 to 18 carbon atoms, phenyl,

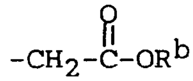 ,

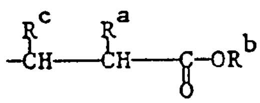 or 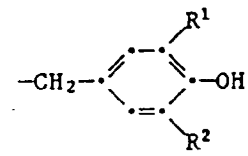 ,

wherein

$R^b$ is alkyl having from 1 to 24 carbon atoms,

$R^a$ is -H or -$CH_3$,

$R^c$ is -H and

$R^{6a}$ is alkyl having from 1 to 12 carbon atoms or phenyl, and

each of $R^5$ and $R^6$, independently of the other, is -H, alkyl having from 1 to 12 carbon atoms, phenyl

or

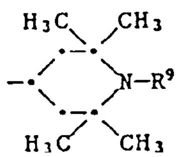 ,

wherein $R^9$ is as defined above, or

$R^5$ is -$NH_2$ and

$R^6$ is -H, or

n is 2, in which case

A is -O-$C_xH_{2x}$-O- and

x is 2 to 8.

4. A composition according to claim 1, comprising at least one compound of formula I wherein

$R^1$ is tert-butyl,

$R^2$ is -H, methyl or tert-butyl, and

$R^3$ is -H, and

n is 1, in which case

A is -$OR^4$ or

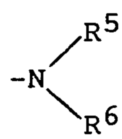 ,

and

$R^4$ is alkyl having from 1 to 18 carbon atoms, -$(CH_2)_7$-CH=CH-$(CH_2)_7CH_3$,

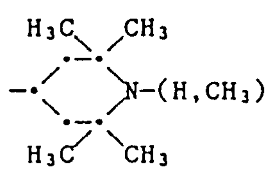

or -$CH_2CH_2$-$SR^{5a}$ wherein

$R^{5a}$ is -H- or

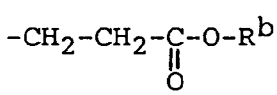 ,

wherein

$R^b$ is n-$C_4H_9$ to n-$C_8H_{17}$ or tert-$C_4H_9$ to tert-$C_8H_{17}$, or

n is 2, in which case

A is $-O-C_xH_{2x}-O-$ and
x is 2 to 8, or
A is $-O-(CH_2-CH_2-O-)_a-CH_2-CH_2-O-$ and
a is 1 to 4, or
A is $-O-CH_2-CH_2-B-CH_2-O-$ and
B is -S- ,

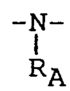 or 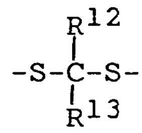 ,

wherein
$R_A$ is $C_4$-$C_8$alkyl or phenyl,
$R^{12}$ is H or $C_1$-$C_8$alkyl and
$R^{13}$ is H, $C_1$-$C_8$alkyl or phenyl, or
A is

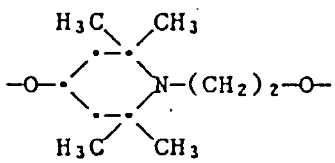 , 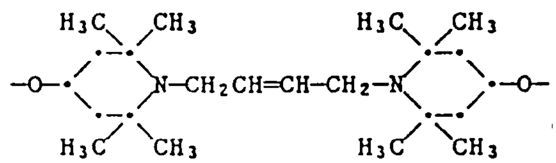

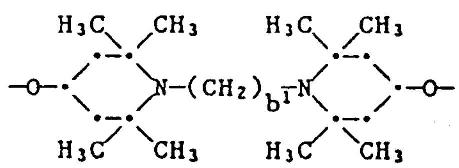 ,

wherein
$b^1$ is 2 to 6, or
A is

$-O-CH_2-CH=CH-CH_2-O-$,
$-O-CH_2-C{\equiv}C-CH_2-O-$,

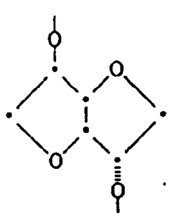 , 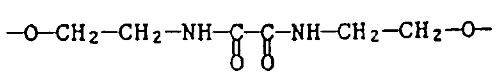 or 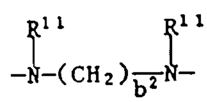 ,

wherein
$b^2$ is 0 to 6 and
$R^{11}$ is -H or $C_1$-$C_8$alkyl.

5. A composition according to claim 1, comprising at least one compound of formula I wherein
$R^1$ is tert-butyl,
$R^2$ is -H, $-CH_3$ or tert-butyl, and
$R^3$ is -H and
n is 1 and
A is $-OR^4$ and
$R^4$ is $C_1$-$C_{18}$alkyl.

6. A composition according to claim 1, comprising at least one compound of formula I wherein
$R^1$ is tert-butyl,

$R^2$ is -H, $-CH_3$ or tert-butyl, and
$R^3$ is -H, and
n is 2 and
A is $-O-C_xH_{2x}-O-$ and
x is 2 to 6, or
A is $-O-(CH_2-CH_2-O)_a-CH_2-CH_2-O-$ and
a is 1, 2 or 3, or
A is $-O-CH_2-CH_2-B-CH_2-CH_2-O-$ and
B is -S- or

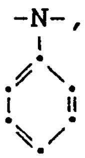

or
A is

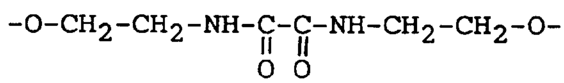

or
A is -NH-NH-.

7. A composition according to claim 1, comprising at least one compound of formula I wherein
$R^1$ is tert-butyl,
$R^2$ is -H, $-CH_3$ or tert-butyl,
$R^3$ is -H,
n is 1 and
A is $-OR^4$, wherein
$R^4$ is $C_1$-$C_{18}$alkyl, or
n is 2 and
A is $-O-C_xH_{2x}-O-$, and x is 2 to 6,
$-OCH_2CH_2-S-CH_2CH_2O-$ or
$-O(CH_2CH_2O)_aCH_2CH_2-O-$ and
a is 1 to 4.

8. A composition according to claim 7, comprising at least one compound of formula I wherein a is 1 or 2.

9. A composition according to claim 1, comprising at least one compound of formula I wherein
$R^1$ is tert-butyl,
$R^2$ is -H, $-CH_3$, tert-butyl, and
$R^3$ is -H, and
n is 3 and
A is

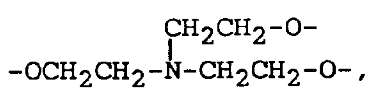

or
n is 4 and
A is

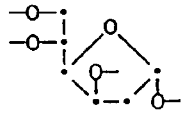,

or
n is 6 and
A is

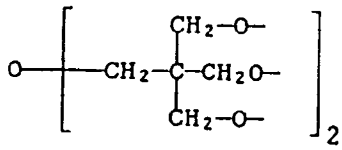.

**10.** A composition according to claim 1, comprising at least one compound from the series of formulae

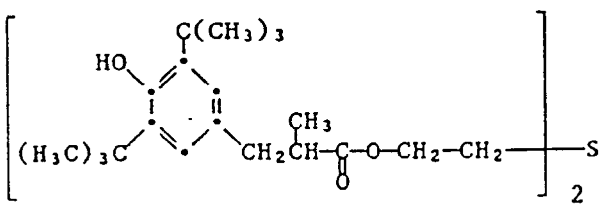,

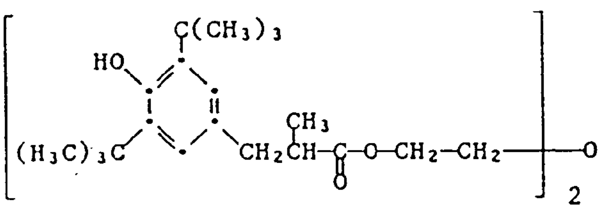,

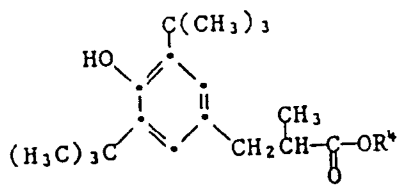,

wherein $R^4$ is $C_1$-$C_{18}$alkyl,

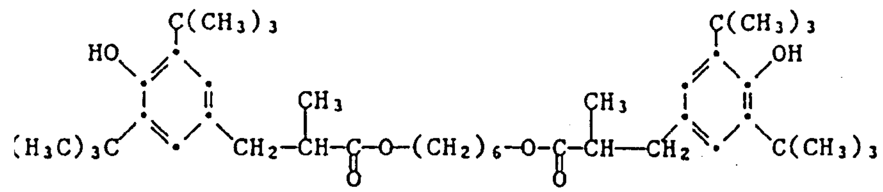

and

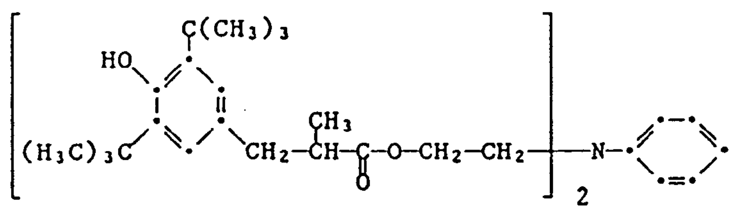

.

**11.** A composition according to claim 1, comprising
a) a functional fluid from the group consisting of lubricants, hydraulic fluids and metal working fluids or a synthetic polymer and
b) at least one compound of formula I according to claim 1.

**12.** A composition according to claim 1, comprising
a) a lubricant from the group consisting of mineral oils, synthetic oils or a mixture thereof and
b) at least one compound of formula I according to claim 1.

**13.** A composition according to claim 1, comprising at least one compound according to claim 10.

**14.** A composition according to claim 1, comprising
a) a synthetic polymer and
b) at least one compound of formula I according to claim 1.

**15.** A composition according to claim 14, comprising
a) a polyolefin and
b) at least one compound of formula I according to claim 1.

**16.** A compound of formula I defined in claim 1 with the proviso that $R^4$ is not $C_1$-$C_4$alkyl.

**17.** A compound according to claim 16, wherein $R^1$, $R^2$, $R^3$, A and n are as defined in claim 2.

**18.** A compound according to claim 16, wherein $R^1$, $R^2$, $R^3$, A and n are as defined in claim 3.

**19.** A compound according to claim 16, wherein $R^1$, $R^2$, $R^3$, A and n are as defined in claim 4.

**20.** A compound according to claim 16, wherein $R^1$, $R^2$, $R^3$, A and n are as defined in claim 5.

**21.** A compound according to claim 16, wherein $R^1$, $R^2$, $R^3$, A and n are as defined in claim 6.

**22.** A compound according to claim 16, wherein $R^1$, $R^2$, $R^3$, A and n are as defined in claim 7.

**23.** A compound according to claim 16, wherein $R^1$, $R^2$, $R^3$, A and n are as defined in claim 8.

**24.** A compound according to claim 16, wherein $R^1$, $R^2$, $R^3$, A and n are as defined in claim 9.

**25.** A compound according to claim 16 of a formula given in claim 10.

**26.** A compound according to claim 16, wherein $R^4$ is alkyl having from 9 to 18 carbon atoms.

**27.** The use of a compound according to claim 16 as an antioxidant in organic materials that are sensitive to oxidative, thermal or actinic degradation.

**28.** The use according to claim 27 in lubricants.

**Claims for the following Contracting State : ES**

**1.** A composition comprising

a) at least one organic material subject to oxidative, thermal or actinic degradation and

b) at least one compound of general formula I

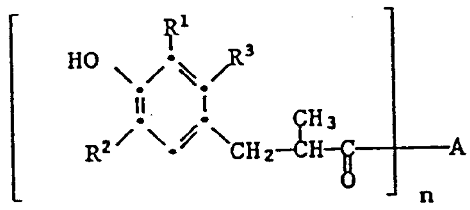

(I),

wherein

$R^1$ is alkyl having from 1 to 18 carbon atoms, cycloalkyl having from 5 to 12 carbon atoms, phenyl or aralkyl having from 7 to 9 carbon atoms,

$R^2$ is -H, alkyl having from 1 to 18 carbon atoms, cycloalkyl having from 5 to 12 carbon atoms, phenyl or aralkyl having from 7 to 9 carbon atoms, and

$R^3$ is -H or $CH_3$, and

n is a number from 1 to 4 or 6, and,

when n is 1,

A is $-OR^4$ or

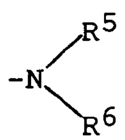

,

and

$R^4$ is -H, alkyl having from 1 to 45 carbon atoms, cycloalkyl having from 5 to 12 carbon atoms, alkenyl having from 2 to 18 carbon atoms,

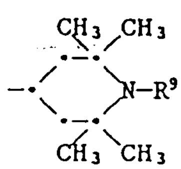

or $-CH_2CH_2-XR^{5a}$, and

each of $R^5$ and $R^6$, independently of the other, is -H, alkyl having from 1 to 20 carbon atoms, phenyl, cycloalkyl having from 5 to 12 carbon atoms, $C_1$-$C_4$alkyl-substituted cycloalkyl having from 5 to 12 carbon atoms, alkenyl having from 3 to 8 carbon atoms, aralkyl having from 7 to 10 carbon atoms or

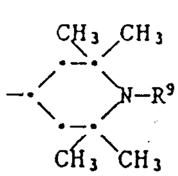

,

and

$R^5$ may additionally be $-NH_2$,

$R^9$ is -H, alkyl having from 1 to 8 carbon atoms,

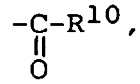

-O· or -OR$^{9}$ ', wherein

R$^{9}$ ' is -H, alkyl having from 1 to 25 carbon atoms or

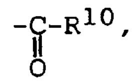

X is -O-, -S- or

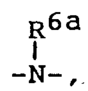

R$^{5a}$ is

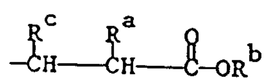 , 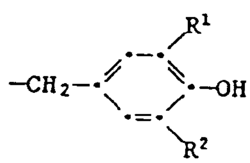 ,

-H, alkyl having from 1 to 24 carbon atoms, phenyl, cycloalkyl having from 5 to 12 carbon atoms or

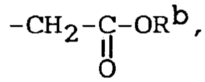

and

R$^{10}$ is alkyl having from 1 to 20 carbon atoms,

R$^{a}$ is -H or -$CH_3$,

R$^{b}$ is -H or alkyl having from 1 to 24 carbon atoms and

R$^{c}$ is -H or -$CH_3$, with the proviso that R$^{a}$ and R$^{c}$ are not -$CH_3$ at the same time,

and

R$^{6a}$ is alkyl having from 1 to 18 carbon atoms, phenyl, phenyl substituted by one or more alkyl groups having a total of from 1 to 24 carbon atoms, or $C_5$-$C_8$cycloalkyl, or,

when n is 2,

A is

$$-O-C_xH_{2x}-O-,$$
$$-O-(CH_2CH_2O)_aCH_2CH_2O-,$$
$$-O-CH_2-CH_2-B-CH_2-CH_2-O-,$$

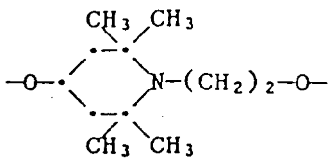 , 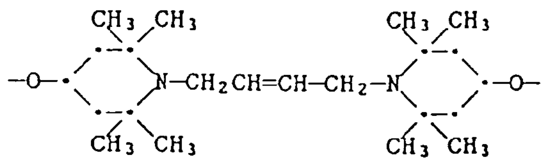 ,

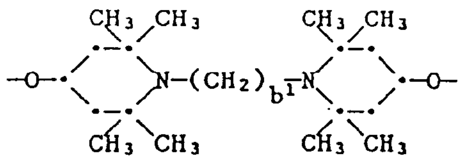 ,

$-O-CH_2CH=CHCH_2-O-$ , $-O-CH_2C\equiv CCH_2-O-$ , 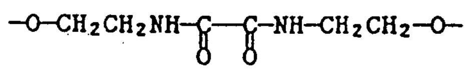 ,

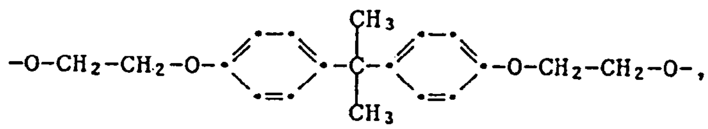

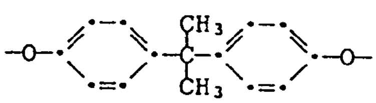 , 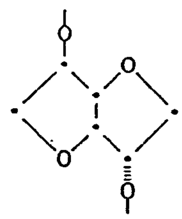 or 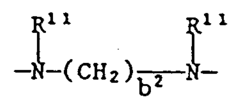 ,

wherein

a is a number from 1 to 30,

x is a number from 2 to 20,

B is -S- ,

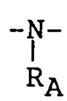 or 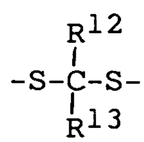 ,

$R_A$ is alkyl having from 1 to 20 carbon atoms, phenyl, phenyl substituted by one or more alkyl groups having a total of from 1 to 20 carbon atoms, cyclohexyl or

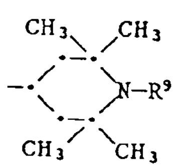 ,

and

each of $R^{11}$, $R^{12}$ and $R^{13}$, independently of the others, is -H, alkyl having from 1 to 12 carbon atoms or phenyl, or

$R^{12}$ and $R^{13}$ together with the carbon atom to which they are bonded form a cycloalkyl ring having from 5 to 12 carbon atoms,

b¹ is a number from 2 to 10 and

b² is a number from 0 to 6, or,

when n is 3,

A is

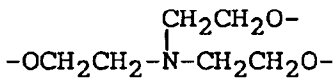 or 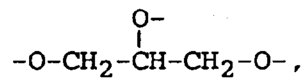

or,

when n is 4,

A is

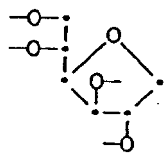 or 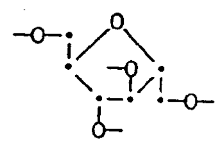 ,

or, when n is 6,

A is

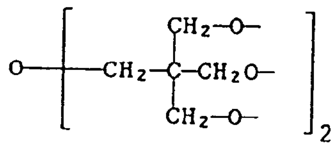 .

2. A composition according to claim 1, comprising at least one compound of formula I wherein

$R^1$ is alkyl having from 1 to 18 carbon atoms, cycloalkyl having from 5 to 12 carbon atoms, phenyl or benzyl,

$R^2$ is -H, alkyl having from 1 to 18 carbon atoms, cycloalkyl having from 5 to 12 carbon atoms, phenyl or benzyl, and

$R^3$ is -H, and

n is a number from 1 to 4 or 6, wherein,

when n is 1,

A is $-OR^4$ or

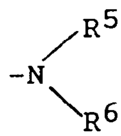 ,

and

$R^4$ is -H, alkyl having from 1 to 20 carbon atoms, cyclohexyl, alkenyl having from 2 to 18 carbon atoms or $-CH_2CH_2-XR^{5a}$, and

$R^5$ is -H, alkyl having from 1 to 12 carbon atoms, phenyl, cyclohexyl, $C_1$-$C_4$alkyl-substituted cycloalkyl having from 5 to 12 carbon atoms, alkenyl having from 3 to 8 carbon atoms, benzyl or $-NH_2$, and

$R^6$ is -H or alkyl having from 1 to 12 carbon atoms,

X is -O-, -S- or

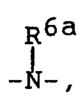

$R^{5a}$ is -H, alkyl having from 1 to 12 carbon atoms, phenyl, cyclohexyl or

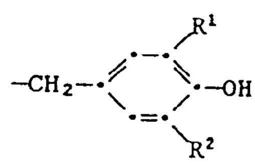 ,

and

$R^{6a}$ is alkyl having from 1 to 12 carbon atoms, phenyl, or phenyl substituted by one or more alkyl groups having a total of from 1 to 18 carbon atoms, or,

when n is 2,

A is

$$-O-C_xH_{2x}-O-,$$
$$-O-(CH_2CH_2O)_aCH_2CH_2O-,$$
$$-O-CH_2-CH_2-B-CH_2-CH_2O-,$$

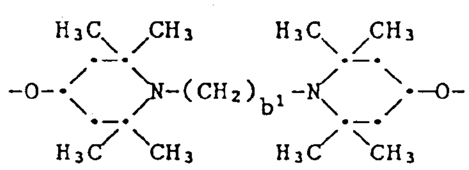

$-O-CH_2CH=CHCH_2-O-$ , 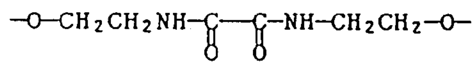 ,

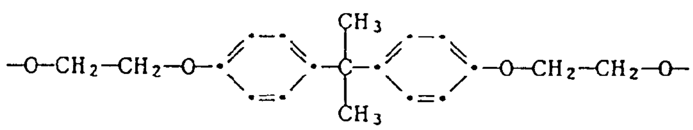 ,

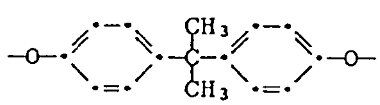 , 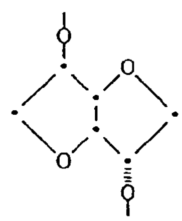 or 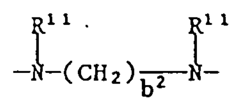 ,

wherein

a is a number from 1 to 12,

x is a number from 2 to 12,

B is -S- or

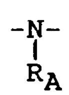 ,

$R_A$ is alkyl having from 1 to 12 carbon atoms, phenyl, phenyl substituted by one or more alkyl groups having a total of from 1 to 18 carbon atoms or cyclohexyl, and

$R^{11}$ is -H, alkyl having from 1 to 12 carbon atoms or phenyl, and

$b^1$ is a number from 2 to 6 and

$b^2$ is a number from 0 to 6, or,

when n is 3,

A is

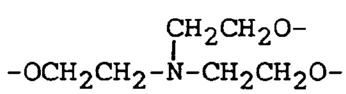 or 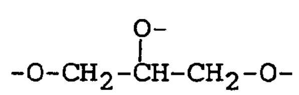 ,

or,

when n is 4,

A is

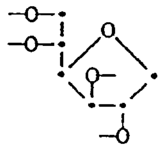

or

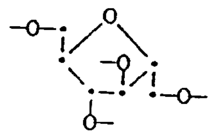

,

or, when n is 6,
A is

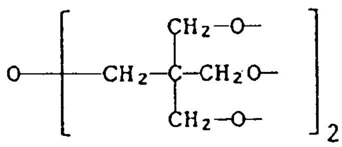

.

3. A composition according to claim 1, comprising at least one compound of formula I wherein
$R^1$ is alkyl having from 1 to 8 carbon atoms, cyclohexyl or phenyl,
$R^2$ is -H, alkyl having from 1 to 8 carbon atoms, cyclohexyl or phenyl, and
$R^3$ is -H, and
n is 1 and
A is $-OR^4$ or

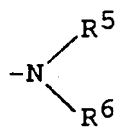

,

wherein
$R^4$ is -H, alkyl having from 1 to 18 carbon atoms, cyclohexyl, $-CH_2CH=CH_2$, $-(CH_2)_7-CH=CH-(CH_2)_7CH_3$,

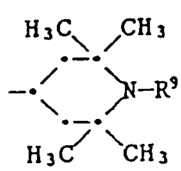

or $-CH_2CH_2XR^{5a}$, wherein
$R^9$ is -H, alkyl having from 1 to 4 carbon atoms, -O·, -O-alkyl having from 1 to 4 carbon atoms or cyclohexyl,
X is -O-, -S- or

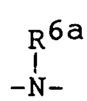

,

$R^{5a}$ is -H, alkyl having from 1 to 18 carbon atoms, phenyl,

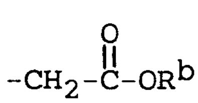

,

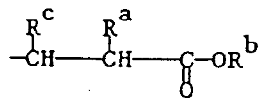 or 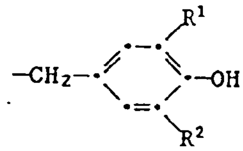 ,

wherein

$R^b$ is alkyl having from 1 to 24 carbon atoms,
$R^a$ is -H or $-CH_3$,
$R^c$ is -H and
$R^{6a}$ is alkyl having from 1 to 12 carbon atoms or phenyl, and
each of $R^5$ and $R^6$, independently of the other, is -H, alkyl having from 1 to 12 carbon atoms, phenyl

or

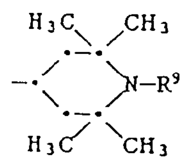 ,

wherein $R^9$ is as defined above, or
$R^5$ is $-NH_2$ and
$R^6$ is -H, or
n is 2, in which case
A is $-O-C_xH_{2x}-O-$ and
x is 2 to 8.

4. A composition according to claim 1, comprising at least one compound of formula I wherein
$R^1$ is tert-butyl,
$R^2$ is -H, methyl or tert-butyl, and
$R^3$ is -H, and
n is 1, in which case
A is $-OR^4$ or

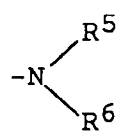 ,

and

$R^4$ is alkyl having from 1 to 18 carbon atoms, $-(CH_2)_7-CH=CH-(CH_2)_7CH_3$,

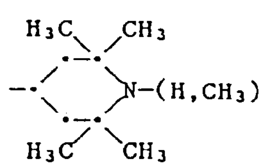

or $-CH_2CH_2-SR^{5a}$ wherein
$R^{5a}$ is -H or

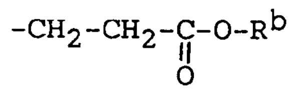 ,

wherein

$R^b$ is $n-C_4H_9$ to $n-C_8H_{17}$ or $tert-C_4H_9$ to $tert-C_8H_{17}$, or

$\underline{n}$ is 2, in which case
A is $-O-C_xH_{2x}-O-$ and
$\underline{x}$ is 2 to 8, or
A is $-O-(CH_2-CH_2-O-)_a-CH_2-CH_2-O-$ and
$\underline{a}$ is 1 to 4, or
A is $-O-CH_2-CH_2-B-CH_2-CH_2-O-$ and
B is -S- ,

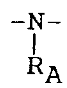 or 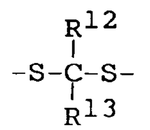 ,

wherein
$R_A$ is $C_4$-$C_8$alkyl or phenyl,
$R^{12}$ is H or $C_1$-$C_8$alkyl and
$R^{13}$ is H, $C_1$-$C_8$alkyl or phenyl, or
A is

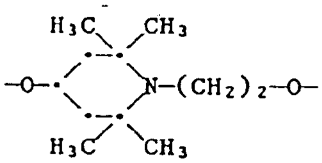 , 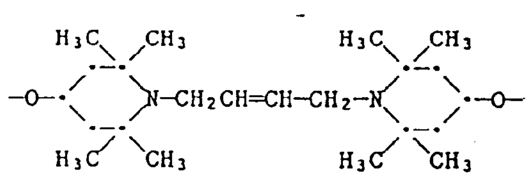 or

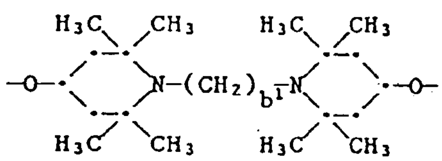

wherein
$\underline{b}^1$ is 2 to 6, or
A is

$-O-CH_2-CH=CH-CH_2-O-$,
$-O-CH_2-C{\equiv}C-CH_2-O-$,

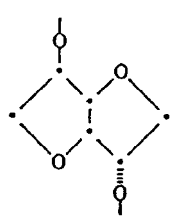 , 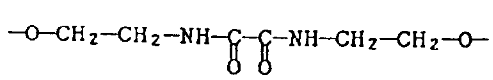 or 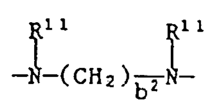 ,

wherein
$\underline{b}^2$ is 0 to 6 and
$R^{11}$ is -H or $C_1$-$C_8$alkyl.

5. A composition according to claim 1, comprising at least one compound of formula I wherein
$R^1$ is tert-butyl,
$R^2$ is -H, $-CH_3$ or tert-butyl, and
$R^3$ is -H and
$\underline{n}$ is 1 and
A is $-OR^4$ and
$R^4$ is $C_1$-$C_{18}$alkyl.

6. A composition according to claim 1, comprising at least one compound of formula I wherein
$R^1$ is tert-butyl,
$R^2$ is -H, $-CH_3$ or tert-butyl, and
$R^3$ is -H, and
n is 2 and
A is $-O-C_xH_{2x}-O-$ and
x is 2 to 6, or
A is $-O-(CH_2-CH_2-O)_a-CH_2-CH_2-O-$ and
a is 1, 2 or 3, or
A is $-O-CH_2-CH_2-B-CH_2-CH_2-O-$ and
B is -S-or

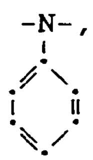

or
A is

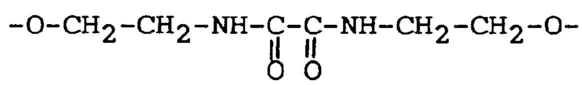

or
A is-NH-NH-.

7. A composition according to claim 1, comprising at least one compound of formula I wherein
$R^1$ is tert-butyl,
$R^2$ is -H, $-CH_3$ or tert-butyl,
$R^3$ is -H,
n is 1 and
A is $-OR^4$, wherein
$R^4$ is $C_1$-$C_{18}$alkyl, or
n is 2 and
A is $-O-C_xH_{2x}-O-$, and x is 2 to 6,
$-OCH_2CH_2-S-CH_2CH_2O-$ or
$-O(CH_2CH_2O)_aCH_2CH_2-O-$ and
a is 1 to 4.

8. A composition according to claim 7, comprising at least one compound of formula I wherein a is 1 or 2.

9. A composition according to claim 1, comprising at least one compound of formula I wherein
$R^1$ is tert-butyl,
$R^2$ is -H, $-CH_3$, tert-butyl, and
$R^3$ is -H, and
n is 3 and
A is

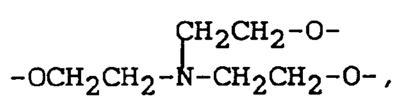

or
n is 4 and
A is

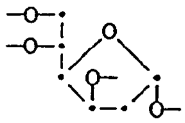

,

or
n is 6 and
A is

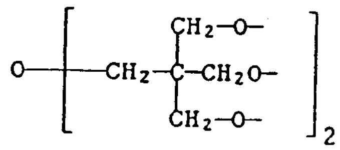

.

10. A composition according to claim 1, comprising at least one compound from the series of formulae

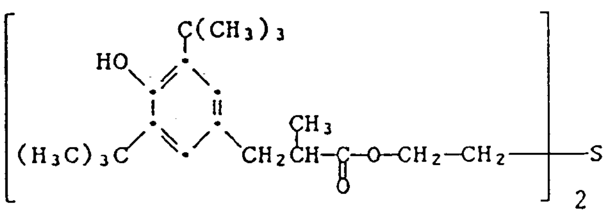

,

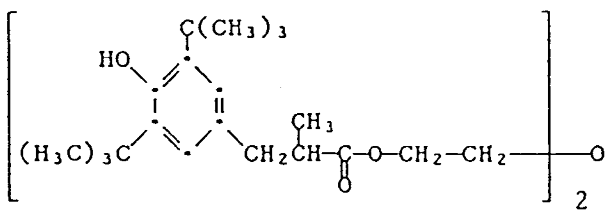

,

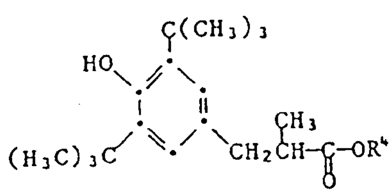

,

wherein $R^4$ is $C_1$-$C_{18}$alkyl,

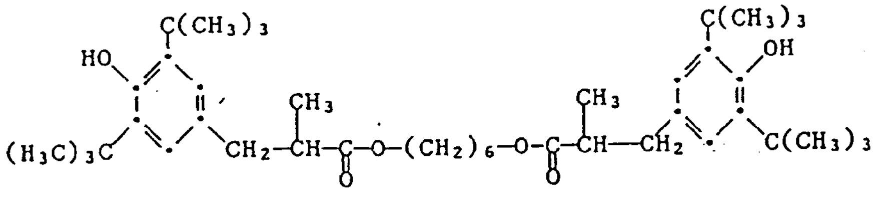

and

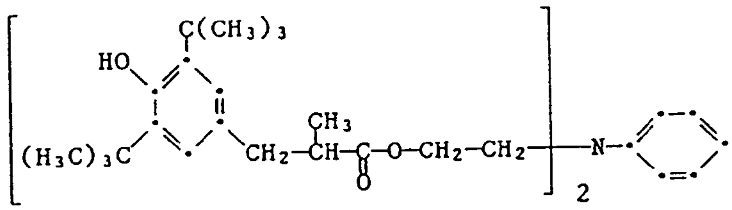

.

**11.** A composition according to claim 1, comprising
a) a functional fluid from the group consisting of lubricants, hydraulic fluids and metal working fluids or a synthetic polymer and
b) at least one compound of formula I according to claim 1.

**12.** A composition according to claim 1, comprising
a) a lubricant from the group consisting of mineral oils, synthetic oils or a mixture thereof and
b) at least one compound of formula I according to claim 1.

**13.** A composition according to claim 1, comprising at least one compound according to claim 10.

**14.** A composition according to claim 1, comprising
a) a synthetic polymer and
b) at least one compound of formula I according to claim 1.

**15.** A composition according to claim 14, comprising
a) a polyolefin and
b) at least one compound of formula I according to claim 1.

**16.** The use of a compound of formula I according to claim 1 as an antioxidant in organic materials that are sensitive to oxidative, thermal or actinic degradation.

**17.** The use of a compound of formula I according to claim 1 as an antioxidant in lubricants.

**Revendications**

**Revendications pour les Etats contractants suivants : BE, DE, FR, GB, IT, NL,**

**1.** Composition, contenant :
(a) au moins une matière organique soumise ou sujette à une dégradation oxydante, thermique ou actinique, et
(b) au moins un composé de formule générale I

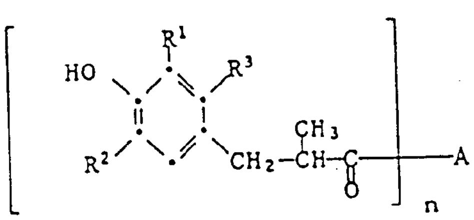

(I),

dans laquelle,

$R^1$ représente un groupe alkyle ayant 1 à 18 atomes de carbone, cycloalkyle ayant 5 à 12 atomes de carbone, phényle ou aralkyle comportant de 7 à 9 atomes de carbone,

$R^2$ représente un atome d'hydrogène, un groupe alkyle ayant 1 à 18 atomes de carbone, cycloalkyle ayant 5 à 12 atomes de carbone, phényle ou aralkyle ayant 7 à 9 atomes de carbone, et

$R^3$ représente -H ou $CH_3$, et

n est un nombre valant 1 à 4 ou 6, et

lorsque n vaut 1,

A représente $OR^4$ ou

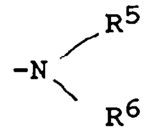

,

et

$R^4$ représente -H, un groupe alkyle ayant 1 à 45 atomes de carbone, cyclo alkyle ayant 5 à 12 atomes de carbone, alcényle ayant 2 à 18 atomes de carbone,

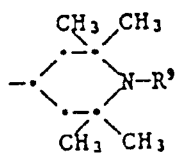

ou $-CH_2CH_2-XR^{5a}$, et

$R^4$ et $R^5$ représentent chacun, indépendamment l'un de l'autre, un atome de H, un groupe alkyle ayant 1 à 20 atomes de carbone, un groupe phényle, cycloalkyle ayant 5 à 12 atomes de carbone, cycloalkyle comportant 5 à 12 atomes de carbone et portant un substituant alkyle en $C_1$ à $C_4$, un groupe alcényle ayant 3 à 8 atomes de carbone, aralkyle ayant 7 à 10 atomes de carbone ou

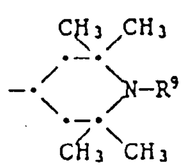

et,,

$R^5$ représente en outre $-NH_2$,

$R^9$ représente -H, un groupe alkyle ayant 1 à 8 atomes de carbone,

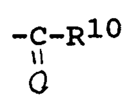

,

-O' ou $-OR^{9\prime}$, et

$R^{9\prime}$ représente -H, un groupe alkyle ayant 1 à 25 atomes de carbone ou

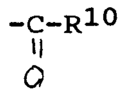

,

X représente -O-, -S-, ou

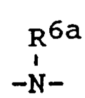

,

$R^{5a}$ représente

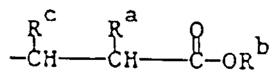 , 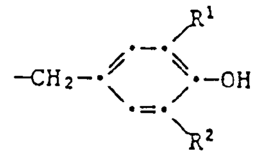 ,

-H, un groupe alkyle ayant 1 à 24 atomes de carbone, phényle, cycloalkyle ayant 5 à 12 atomes de carbone ou

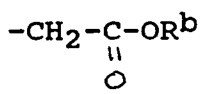 ,

et

$R^{10}$ représente un groupe alkyle ayant 1 à 20 atomes de carbone ,

$R^a$ représente -H ou $-CH_3$,

$R^b$ représente -H ou un groupe alkyle ayant 1 à 24 atomes de carbone et

$R^c$ représente -H ou $-CH_3$, avec la réserve que $R^a$ et $R^c$ ne représentent pas simultanément chacun $-CH_3$,

et

$R^{6a}$ représente un groupe alkyle ayant 1 à 18 atomes de carbone , phényle , phényle (substitué par un ou plusieurs groupes alkyles ayant au total 1 à 24 atomes de carbone ) ou cycloalkyle en $C_5$ à $C_8$, ou bien, lorsque n vaut 2,

A représente

$-O-C_xH_{2x}-O-$,

$-O-(CH_2CH_2O)_aCH_2CH_2O-$ ,

$-O-CH_2-CH_2-B-CH_2-CH_2-O-$ ,

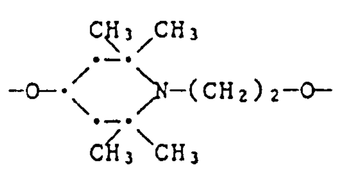 , 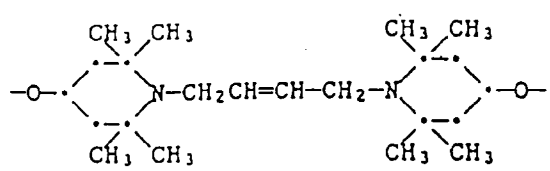 ,

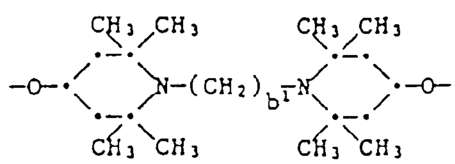 ,

$-O-CH_2CH=CHCH_2-O-$ , $-O-CH_2C\equiv CCH_2-O-$ , 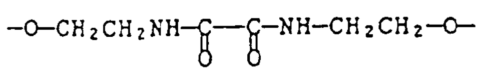 ,

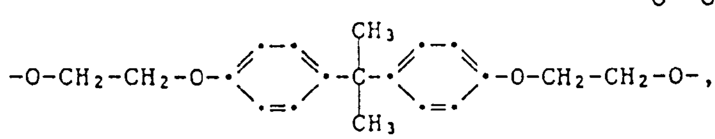

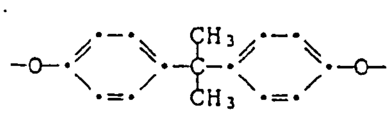 , 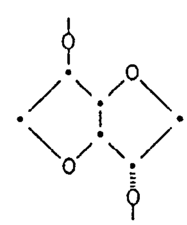 ou 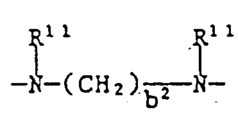

formules dans lesquelles :

a est un nombre valant 1 à 30,

x est un nombre valant 2 à 20,

B représente -S- ,

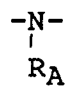 ou 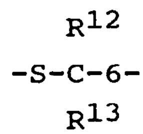 ,

$R_A$ représente un groupe alkyle ayant 1 à 20 atomes de carbone, phényle ou phényle (substitué par un ou plusieurs groupes alkyles ayant au total 1 à 20 atomes de C), cyclohexyle ou

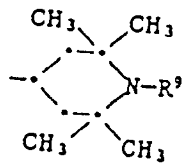

et,

$R^{11}$, $R^{12}$ et $R^{13}$ représentent chacun, indépendamment l'un de l'autre, un atome de H, un groupe alkyle ayant 1 à 12 atomes de carbone ou phényle, ou bien

$R^{12}$ et $R^{13}$, pris avec l'atome de C auquel ils sont fixés, forment un noyau cycloalkyle ayant 5 à 12 atomes de carbone,

$b^1$ est un nombre valant 2 à 10 et,

$b^2$ est un nombre valant 2 à 6 ou bien , lorsque n vaut 3,

A représente

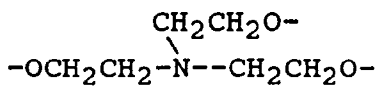 ou 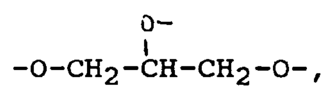,

ou bien lorsque n vaut 4 ,

A représente

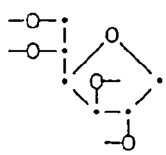 ou 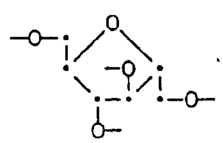

ou, lorsque n vaut 6,

A représente

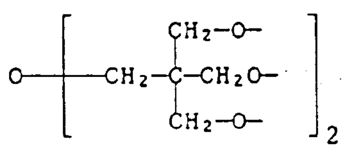

2. Compositions selon la revendication 1, contenant au moins un composé de formule I, dans laquelle :

$R^1$ représente un groupe alkyle ayant 1 à 18 atomes de carbone , cycloalkyle ayant 5 à 12 atomes de carbone , phényle ou benzyle,

$R^2$ représente -H, un groupe alkyle ayant 1 à 18 atomes de carbone, cycloalkyle ayant 5 à 12 atomes de carbone , phényle ou benzyle, et,

$R^3$ représente -H, et

n est un nombre valant 1 à 4 ou 6, et

lorque n vaut 1,

A représente $-OR^4$ ou

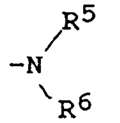

,

et

$R^4$ représente -H, un groupe alkyle ayant 1 à 20 atomes de carbone, cyclohexyle, alcényle ayant 2 à 18 atomes de carbone, ou $-CH_2CH_2-XR^{5a}$, et

$R^5$ représente -H, un groupe alkyle ayant 1 à 12 atomes de carbone, phényle, cyclohexyle, cycloalkyle ayant 5 à 12 atomes de carbone (substitué par un ou des groupes alkyles en $C_1$ à $C_4$), alcényle ayant 3 à 8 atomes de carbone, benzyle ou $-NH_2$ et

$R^6$ représente -H ou un groupe alkyle ayant 1 à 12 atomes de carbone,

X représente -O-, -S- ou

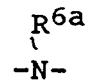

,

$R^{5a}$ représente -H, un groupe alkyle ayant 1 à 12 atomes de C, un groupe phényle, cyclohexyle ou

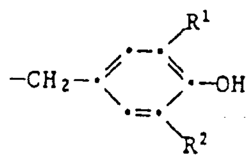

et,

$R^{6a}$ représente un groupe alkyle ayant 1 à 12 atomes de carbone, phényle ou phényle(substitué par un ou plusieurs groupes alkyles ayant au total 1 à 18 atomes de carbone), ou bien,

lorsque n vaut 2,

A représente

$$-O-C_xH_{2x}-O-,$$
$$-O-(CH_2CH_2O)_aCH_2CH_2O-,$$
$$-O-CH_2-CH_2-B-CH_2-CH_2O-,$$

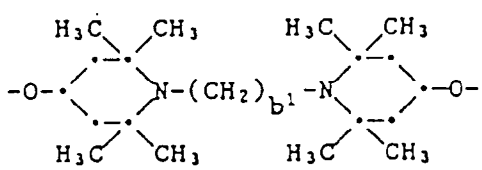

$-O-CH_2CH=CHCH_2-O-$ , 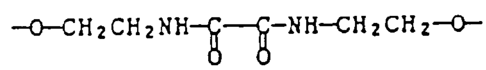 ,

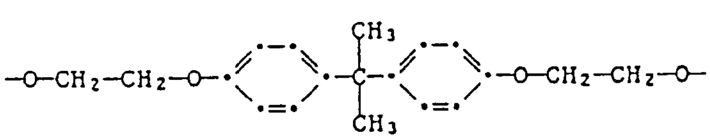 ,

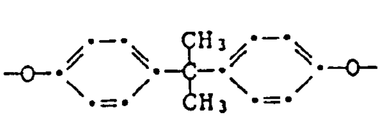 , 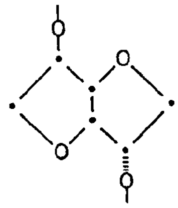 ou 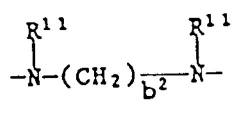

formules dans lesquelles :
a est un nombre valant 1 à 12,
x est un nombre valant 2 à 12,
B représente -S- ou

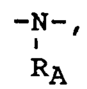

$R_A$ représente un groupe alkyle ayant 1 à 12 atomes de carbone, phényle ou phényle(substitué par un ou plusieurs groupes alkyles ayant au total 1 à 18 atomes de carbone) ou cyclohexyle, et
$R^{11}$ représente -H, un groupe alkyle ayant 1 à 12 atomes de carbone ou phényle, et
$b^1$ est un nombre valant 2 à 6,
$b^2$ est un nombre valant 0 à 6,
ou bien lorsque n vaut 3,
A représente

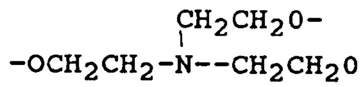 ou 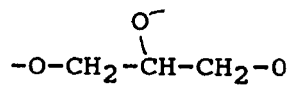 ,

ou bien, lorsque n vaut 4
A représente

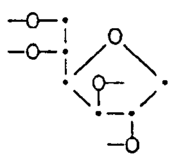 ou 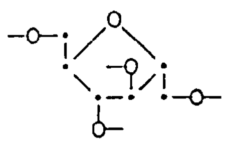

ou bien , lorsque n vaut 6
A représente

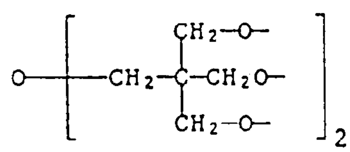

**3.** Compositions selon la revendication 1,contenant au moins un composé de formule I, dans laquelle
$R^1$ représente un groupe alkyle ayant 1 à 8 atomes de carbone , cyclohexyle ou phényle,
$R^2$ représente -H, un groupe alkyle ayant 1 à 8 atomes de carbone, cyclochexyle ou phényle et,
$R^3$ représente -H, et
n vaut 1, et
A représente $-OR^4$ ou

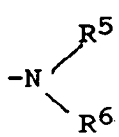 ,

$R^4$ représente -H, un groupe alkyle ayant 1 à 18 atomes de carbone, cyclohexyle $-CH_2CH=CH_2$, $-(CH_2)_7-CH=CH-(CH_2)_7CH_3$,

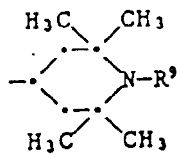

ou $-CH_2CH_2XR^{5a}$ et,dans ce cas

$R^9$ représente -H, un groupe alkyle ayant 1 à 4 atomes de carbone, -O', -0-alkyle ayant 1 à 4 atomes de carbone ou cyclohexyle,

X représente -O-, -S- ou

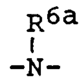 ,

$R^{5a}$ représente -H, un groupe alkyle ayant 1 à 18 atomes de carbone , phényle,

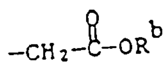 ,

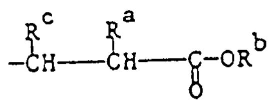 ou 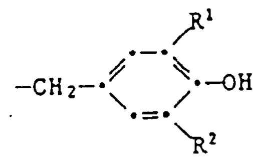

et,dans ce cas

$R^b$ représente un groupe alkyle ayant 1 à 24 atomes de carbone,

$R^a$ représente -H ou $-CH_3$,

$R^c$ représente -H, et

$R^{6a}$ représente un groupe alkyle ayant 1 à 12 atomes de carbone ou un groupe phényle, et

$R^5$ et $R^6$ représentent chacun, indépendamment l'un de l'autre, -H, un groupe alkyle ayant 1 à 12 atomes de carbone , un groupe phényle, ou

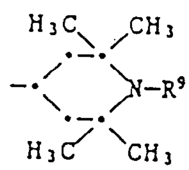

formule dans laquelle, $R^9$ a le sens précité, ou bien

$R^5$ représente $-NH_2$ et $R^6$ reprsente -H,

ou, lorsque n vaut 2, et

A représente $-O-C_xH_{2x}-O-$ , et

x vaut 2 à 8.

4. Composition selon la revendication 1, contenant au moins un composé de formule I, dans laquelle :

$R^1$ représente un groupe tertiobutyle,

$R^2$ représente -H, un groupe méthyle ou tert.-butyle, et

$R^3$ représente -H, et

n vaut 1, et dans ce cas

A représente $-OR^4$ ou

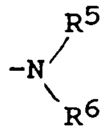

et,

$R^4$ représente un groupe alkyle ayant 1 à 18 atomes de carbone,$-(CH_2)_7-CH=CH(CH_2)_7CH_3$, ou $R^4$ représente

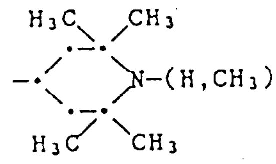

ou

$-CH_2CH_2-SR^{5a}$ et ,
$R^{5a}$ représente -H ou un groupe

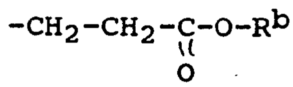

,

et
$R^b$ représente un groupe n-$C_4H_9$ à n-$C_8H_{17}$ ou tert.-$C_4H_9$ à tert.-$C_8H_{17}$, ou bien ,
n vaut 2 et, dans ce cas
A représente alors $-O-C_xH_{2x}-O-$ , et
x vaut 2 à 8, ou bien
A représente $-O-(CH_2-CH_2-O-)_a-CH_2-CH^2-O-$, et
a vaut 1 à 4,
ou bien
A représente $-O-CH_2-CH_2-B-CH_2-CH_2-O-$ , et
B représente -S- ,

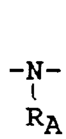 ou 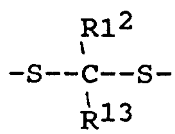,

et

$R_A$ représente un groupe alkyle en $C_4$ à $C_8$ ou phényle,
$R^{12}$ représente H, un groupe alkyle en $C_1$ à $C_8$ et,
$R^{13}$ représente H, un groupe alkyle en $C_1$ à $C_8$ ou phényle,
ou bien

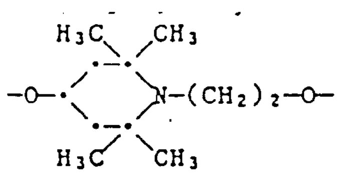 , 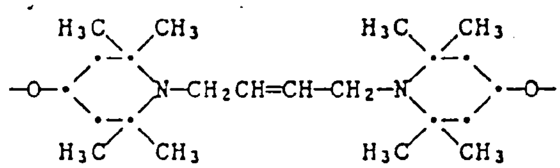 ou

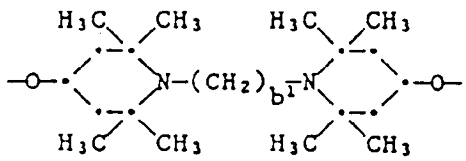

et,

$b^1$ vaut 2 à 6, ou bien

A représente

$-O-CH_2-CH=CH-CH_2-O-$ ,

$-O-CH_2-C{\equiv}C-CH_2-O-$ ,

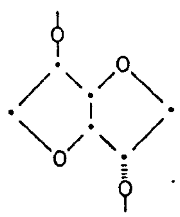 , 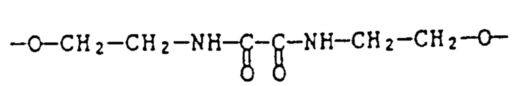 ou 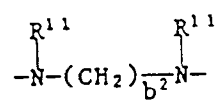

et $b^2$ vaut 0 à 6 , et

$R^{11}$ représente -H ou un groupe alkyle en $C_1$ à $C_8$.

5. Compositions selon la revendication 1, contenant au moins un composé de formule I, dans laquelle $R^1$ représente un groupe tert.-butyle,

$R^2$ représente -H, $-CH_3$ ou un groupe tert.- butyle, et

$R^3$ représente -H, et

n vaut 1 et, dans ce cas,

A représente un groupe $-OR^4$, et

R4 représente un groupe alkyle en $C_1$ à $C_{18}$.

6. Compositions selon la revendication 1, contenant au moins un composé de formule I, dans laquelle

$R^1$ représente un groupe tert.-butyl,

$R^2$ représente -H, $-CH_3$ ou un groupe tert.- butyle, et

$R^3$ représente -H, et

n vaut 2 et, dans ce cas ,

A représente $-O-c_xH_{2x}-O-$, et

x vaut 2 à 6, ou bien

A représente $-O-(CH_2-CH_2-O)_a-CH_2-CH_2-O-$ et,

a vaut 1, 2 ou 3 ou bien,

A représente $-O-CH_2-CH_2-B-CH_2-CH_2-O-$ et

B représente -S- ou

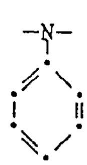

ou bien

A représente

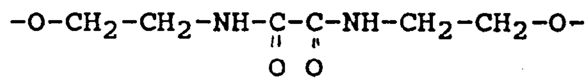

ou
A représente -NH-NH- .

7. Composition selon la revendication 1 contenant au moins un composé de formule I dans laquelle,
$R^1$ représente un groupe tertiobutyle,
$R^2$ représente -H, $-CH_3$ ou un groupe tert.- butyle,
$R^3$ représente -H,
n vaut 1 et, dans ce cas,
A représente $-OR^4$
$R^4$ représente un groupe cycloalkyle en $C_1$ à $C_{18}$, ou bien
n vaut 2 et, dans ce cas,
A représente $-O-C_xH_{2x}-O-$ et x vaut 2 à 6,
$-OCH_2CH_2-s-CH_2CH_2O-$ ou
$-O(CH_2CH_2O)_aCH_2CH_2-O-$ et
a vaut 1 à 4.

8. Compositoon selon la revendication 7, contenant au moins un composé de formule I, dans laquelle a vaut 1 ou 2.

9. Composition selon la revendication 1, contenant au moins un composé de formule I, dans laquelle
$R^1$ représente un groupe tert.-butyle,
$R^2$ représente -H, $-CH_3$, un groupe tert.-butyle et
$R^3$ représente -H,
et
n vaut 3 et , dans ce cas,
A représente

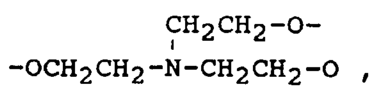

ou,
n vaut 4, et dans ce cas,
A représente

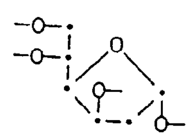

ou
n vaut 6 et, dans ce cas,
A représente

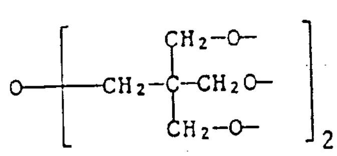

10. Composition selon la revendication 1, contenant au moins un composé appartenant à la série des formules

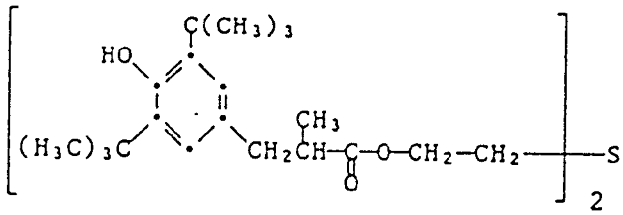

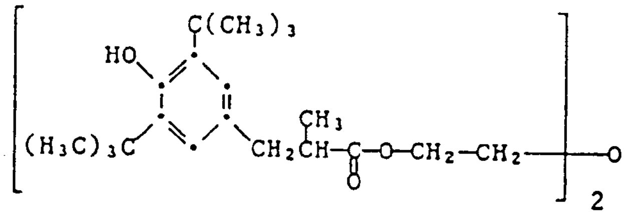

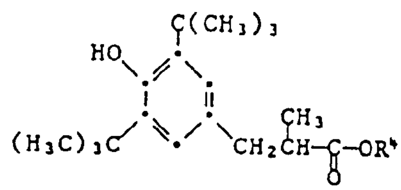

dans lesquelles
$R^4$ représente un groupe alkyle en $C_1$ à $C_{18}$,

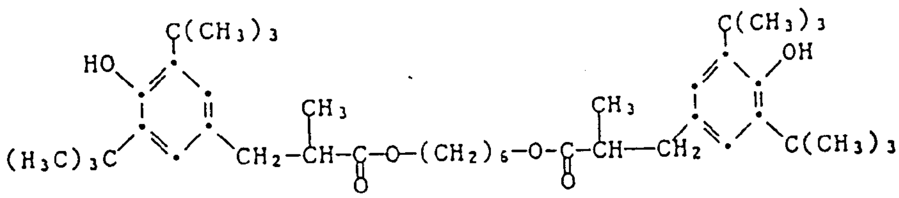

et

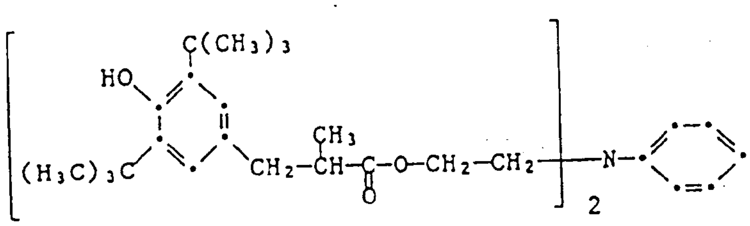

.

**11.** Composition selon la revendication 1, contenant
a) un liquide fonctionnel choisi parmi des lubrifiants, des liquides hydrauliques et des liquides pour le travail des métaux ou un polymère synthétique, et
(b) au moins un composé de formule I selon la revendication 1.

**12.** Composition selon la revendication 1, contrenant
(a) un lubrifiant choisi parmi les huiles minérales, les huiles synthétiques et un de leurs mélanges, et
(b) au moins un composé de formule I, selon la revendication 1.

**13.** Composition selon la revendication 1, contenant au moins un composé selon la revendication 10.

**14.** Composition selon la revendication 1, contenant :
(a) un polymère synthétique, et

(b) au moins un composé de formule I selon la revendication 1.

**15.** Composition selon la revendication 14, contenant :
(a) une polyoléfine, et
(b) au moins un composé de formule I selon la revendication 1.

**16.** Composés répondant à la formule définie à la revendication 1, à la condition que $R^4$ ne représente pas un groupe alkyle en $C_1$ à $C_4$.

**17.** Composés selon la revendication 16, dans lesquels $R^1$, $R^2$, $R^3$, A et n ont le sens indiqué à la revendication 2.

**18.** Composés selon la revendication 16, dans lesquels $R^1$, $R^2$, $R^3$, A et n ont les sens indiqués à la revendication 3.

**19.** Composés selon la revendication 16, dans lesquels $R^1$, $R^2$, $R^3$, A et n ont les sens indiqués à la revendication 4.

**20.** Composés selon la revendication 16, dans lesquels $R^1$, $R^2$, $R^3$, A et n ont les sens indiqués à la revendication 5.

**21.** Composés selon la revendication 16, dans lesquels $R^1$, $R^2$, $R^3$, A et n ont les sens indiqués à la revendication 6.

**22.** Composés selon la revendication 16, dans lesquels $R^1$, $R^2$, $R^3$, A et n ont les sens indiqués à la revendication 7.

**23.** Composés selon la revendication 16, dans lesquels $R^1$, $R^2$, $R^3$, A et n ont les sens indiqués à la revendication 8.

**24.** Composés selon la revendication 16, dans lesquels $R^1$, $R^2$, $R^3$, A et n ont les sens indiqués à la revendication 9.

**25.** Composés selon la revendication 16, répondant aux formules présentées à la revendication 10.

**26.** Composés selon la revendication 16, dans lesquels $R^4$ représente un groupe alkyle ayant 9 à 18 atomes de carbone.

**27.** Utilisation des composés selon la revendication 16, à titre d'anti-oxydants dans des matières organiques sensibles à une dégradation par un ou des effets d'oxydation, par des effets thermiques ou actiniques.

**28.** Utilisation selon la revendication 27 dans des lubrifiants.

**Revendications pour l'Etat contractant suivant : ES**

**1.** Composition, contenant :
(a) au moins une matière organique soumise ou sujette à une dégradation oxydante, thermique ou actinique, et
(b) au moins un composé de formule générale I

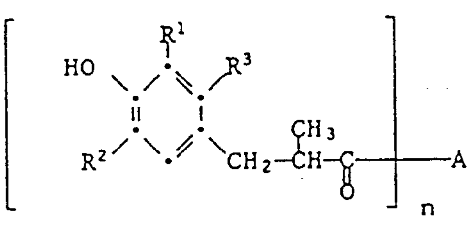

(I),

dans laquelle,

$R^1$ représente un groupe alkyle ayant 1 à 18 atomes de carbone, cycloalkyle ayant 5 à 12 atomes

de carbone, phényle ou aralkyle comportant de 7 à 9 atomes de carbone,

$R^2$ représente un atome d'hydrogène, un groupe alkyle ayant 1 à 18 atomes de carbone, cycloalkyle ayant 5 à 12 atomes de carbone, phényle ou aralkyle ayant 7 à 9 atomes de carbone, et

$R^3$ représente -H ou $CH_3$, et

n est un nombre valant 1 à 4 ou 6, et

lorsque n vaut 1,

A représente $OR^4$ ou

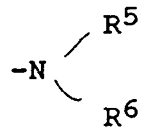 ,

et

$R^4$ représente -H, un groupe alkyle ayant 1 à 45 atomes de carbone, cyclo alkyle ayant 5 à 12 atomes de carbone, alcényle ayant 2 à 18 atomes de carbone,

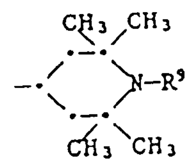

ou $-CH_2CH_2-XR^{5a}$, et

$R^4$ et $R^5$ représentent chacun, indépendamment l'un de l'autre, un atome de H, un groupe alkyle ayant 1 à 20 atomes de carbone, un groupe phényle, cycloalkyle ayant 5 à 12 atomes de carbone, cycloalkyle comportant 5 à 12 atomes de carbone et portant un substituant alkyle en $C_1$ à $C_4$, un groupe alcényle ayant 3 à 8 atomes de carbone, aralkyle ayant 7 à 10 atomes de carbone ou

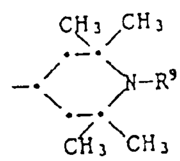

et,

$R^5$ représente en outre $-NH_2$,

$R^9$ représente -H, un groupe alkyle ayant 1 à 8 atomes de carbone,

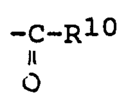 ,

-O' ou $-OR^{9\prime}$, et

$R^{9\prime}$ représente -H, un groupe alkyle ayant 1 à 25 atomes de carbone ou

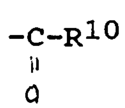 ,

X représente -O-, -S-, ou

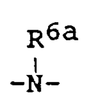 ,

$R^{5a}$ représente

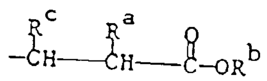 , 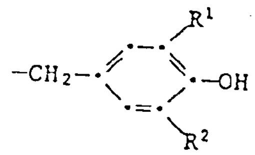 ,

-H, un groupe alkyle ayant 1 à 24 atomes de carbone, phényle, cycloalkyle ayant 5 à 12 atomes de carbone ou

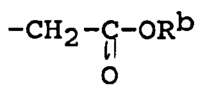 ,

et

$R^{10}$ représente un groupe alkyle ayant 1 à 20 atomes de carbone ,

$R^a$ représente -H ou $-CH_3$,

$R^b$ représente -H ou un groupe alkyle ayant 1 à 24 atomes de carbone et

$R^c$ représente -H ou $-CH_3$, avec la réserve que $R^a$ et $R^c$ ne représentent pas simultanément chacun $-CH_3$,

et

$R^{6a}$ représente un groupe alkyle ayant 1 à 18 atomes de carbone , phényle , phényle (substitué par un ou plusieurs groupes alkyles ayant au total 1 à 24 atomes de carbone ) ou cycloalkyle en $C_5$ à $C_8$, ou bien, lorsque n vaut 2,

A représente

$-O-C_xH_{2x}-O-$,

$-O-(CH_2CH_2O)_aCH_2CH_2O-$,

$-O-CH_2-CH_2-B-CH_2-CH_2-O-$,

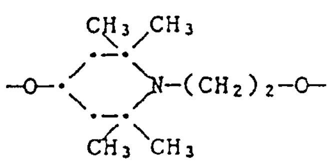 , 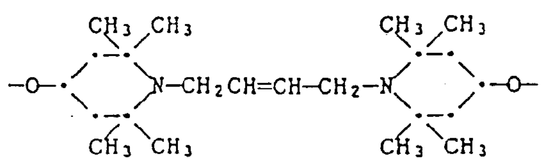 ,

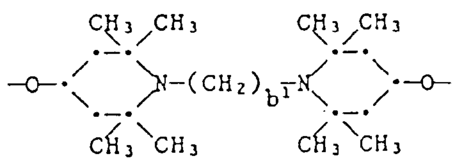 ,

$-O-CH_2CH=CHCH_2-O-$ , $-O-CH_2C\equiv CCH_2-O-$ , 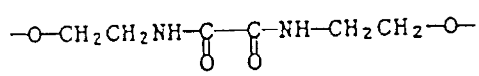 ,

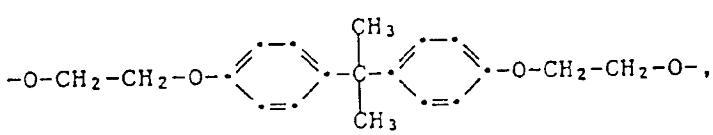,

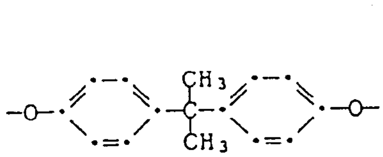 , 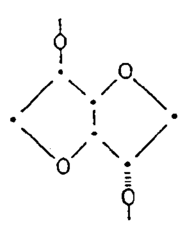 ou 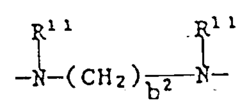

formules dans lesquelles :

a est un nombre valant 1 à 30,

x est un nombre valant 2 à 20,
B représente -S- ,

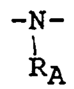 ou 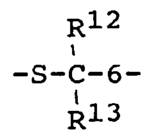 ,

$R_A$ représente un groupe alkyle ayant 1 à 20 atomes de carbone, phényle ou phényle (substitué par un ou plusieurs groupes alkyles ayant au total 1 à 20 atomes de C), cyclohexyle ou

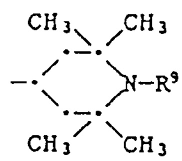

et,
$R^{11}$, $R^{12}$ et $R^{13}$ représentent chacun, indépendamment l'un de l'autre, un atome de H, un groupe alkyle ayant 1 à 12 atomes de carbone ou phényle, ou bien
$R^{12}$ et $R^{13}$, pris avec l'atome de C auquel ils sont fixés, forment un noyau cycloalkyle ayant 5 à 12 atomes de carbone,
$b^1$ est un nombre valant 2 à 10 et,
$b^2$ est un nombre valant 2 à 6 ou bien , lorsque n vaut 3,
A représente

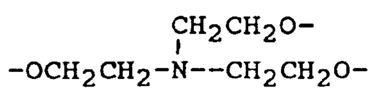 ou 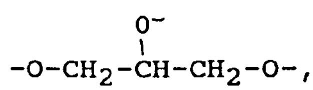

ou bien lorsque n vaut 4 ,
A représente

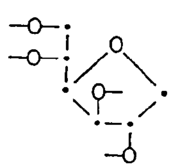 ou 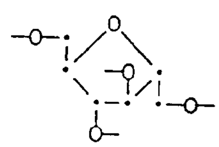

ou, lorsque n vaut 6,
A représente

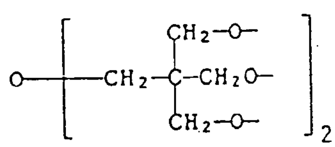

**2.** Compositions selon la revendication 1, contenant au moins un composé de formule I, dans laquelle :
$R^1$ représente un groupe alkyle ayant 1 à 18 atomes de carbone , cycloalkyle ayant 5 à 12 atomes de carbone , phényle ou benzyle,
$R^2$ représente -H, un groupe alkyle ayant 1 à 18 atomes de carbone, cycloalkyle ayant 5 à 12 atomes de carbone , phényle ou benzyle, et,
$R^3$ représente -H, et
n est un nombre valant 1 à 4 ou 6, et lorque n vaut 1,
A représente $-OR^4$ ou

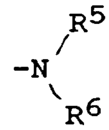

,

et

$R^4$ représente -H, un groupe alkyle ayant 1 à 20 atomes de carbone, cyclohexyle, alcényle ayant 2 à 18 atomes de carbone, ou $-CH_2CH_2-XR^{5a}$, et

$R^5$ représente -H, un groupe alkyle ayant 1 à 12 atomes de carbone, phényle, cyclohexyle, cycloalkyle ayant 5 à 12 atomes de carbone (substitué par un ou des groupes alkyles en $C_1$ à $C_4$), alcényle ayant 3 à 8 atomes de carbone, benzyle ou $-NH_2$ et

$R^6$ représente -H ou un groupe alkyle ayant 1 à 12 atomes de carbone,

X représente -O-, -S- ou

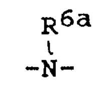

,

$R^{5a}$ représente -H, un groupe alkyle ayant 1 à 12 atomes de C, un groupe phényle, cyclohexyle ou

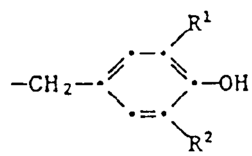

et,

$R^{6a}$ représente un groupe alkyle ayant 1 à 12 atomes de carbone, phényle ou phényle(substitué par un ou plusieurs groupes alkyles ayant au total 1 à 18 atomes de carbone), ou bien,

lorsque n vaut 2,

A représente

$-O-C_xH_{2x}-O-$,
$-O-(CH_2CH_2O)_aCH_2CH_2O-$,
$-O-CH_2-CH_2-B-CH_2-CH_2O$,

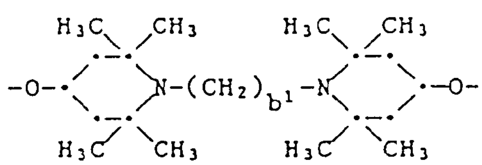

$-O-CH_2CH=CHCH_2-O-$ , 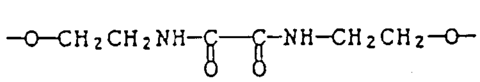 ,

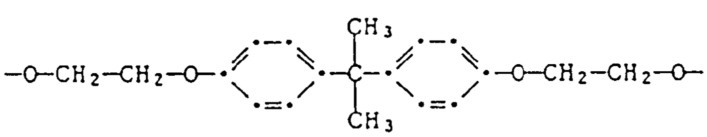 ,

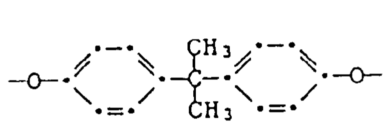 , 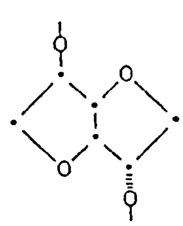 ou 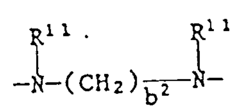

formules dans lesquelles :
a est un nombre valant 1 à 12,
x est un nombre valant 2 à 12,
B représente -S- ou

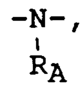

$R_A$ représente un groupe alkyle ayant 1 à 12 atomes de carbone, phényle ou phényle(substitué par un ou plusieurs groupes alkyles ayant au total 1 à 18 atomes de carbone) ou cyclohexyle, et
$R^{11}$ représente -H, un groupe alkyle ayant 1 à 12 atomes de carbone ou phényle, et
$b^1$ est un nombre valant 2 à 6,
$b^2$ est un nombre valant 0 à 6,
ou bien lorsque n vaut 3,
A représente

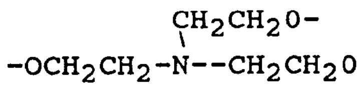 ou 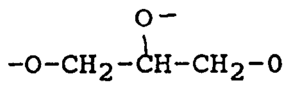 ,

ou bien, lorsque n vaut 4
A représente

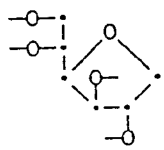 ou 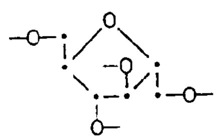

ou bien, lorsque n vaut 6
A représente

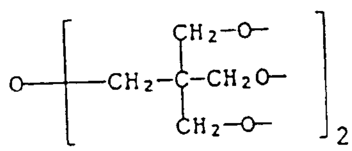 ,

3. Compositions selon la revendication 1, contenant au moins un composé de formule I, dans laquelle
$R^1$ représente un groupe alkyle ayant 1 à 8 atomes de carbone , cyclohexyle ou phényle,
$R^2$ représente -H, un groupe alkyle ayant 1 à 8 atomes de carbone, cyclochexyle ou phényle et,
$R^3$ représente -H, et
n vaut 1, et
A représente $-OR^4$ ou

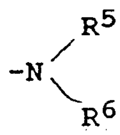 ,

$R^4$ représente -H, un groupe alkyle ayant 1 à 18 atomes de carbone, cyclohexyle $-CH_2CH=CH_2$, $-(CH_2)_7-CH=CH-(CH_2)_7CH_3$,

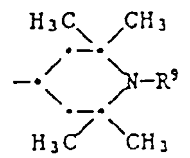

ou $-CH_2CH_2XR^{5a}$

et,dans ce cas

$R^9$ représente -H, un groupe alkyle ayant 1 à 4 atomes de carbone, -O′, -O-alkyle ayant 1 à 4 atomes de carbone ou cyclohexyle,

X représente -O-, -S- ou

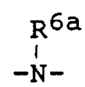 ,

$R^{5a}$ représente -H, un groupe alkyle ayant 1 à 18 atomes de carbone , phényle,

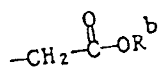 ,

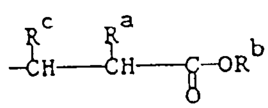 ou 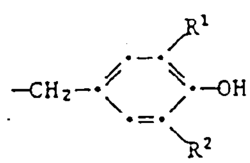

et, dans ce cas

$R^b$ représente un groupe alkyle ayant 1 à 24 atomes de carbone,

$R^a$ représente -H ou $-CH_3$,

$R^c$ représente -H, et

$R^{6a}$ représente un groupe alkyle ayant 1 à 12 atomes de carbone ou un groupe phényle, et

$R^5$ et $R^6$ représentent chacun, indépendamment l'un de l'autre, -H, un groupe alkyle ayant 1 à 12 atomes de carbone , un groupe phényle, ou

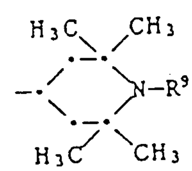

formule dans laquelle, $R^9$ a le sens précité, ou bien

$R^5$ représente $-NH_2$ et $R^6$ reprsente -H,

ou, lorsque n vaut 2, et

A représente $-O-C_xH_{2x}-O-$ , et

x vaut 2 à 8.

4. Composition selon la revendication 1, contenant au moins un composé de formule I, dans laquelle :

$R^1$ représente un groupe tertiobutyle,

$R^2$ représente -H, un groupe méthyle ou tert.-butyle, et

$R^3$ représente -H, et, dans ce cas

n vaut 1, et

A représente $-OR^4$ ou

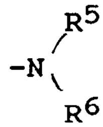

et,

$R^4$ représente un groupe alkyle ayant 1 à 18 atomes de carbone,$-(CH_2)_7-CH=CH(CH_2)_7CH_3$, ou $R^4$ représente

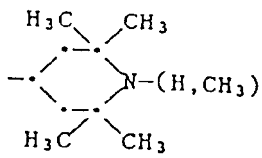

ou

$-CH_2CH_2-SR^{5a}$ et ,

$R^{5a}$ représente -H ou un groupe

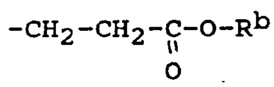 ,

et

$R^b$ représente un groupe n-$C_4H_9$ à n-$C_8H_{17}$ ou tert.-$C_4H_9$ à tert.-$C_8H_{17}$, ou bien ,
n vaut 2 et, dans ce cas,
A représente alors $-O-C_xH_{2x}-O-$ , et
x vaut 2 à 8, ou bien
A représente $-O-(CH_2-CH_2-O-)_a-CH_2-CH^2-O-$, et
a vaut 1 à 4,
ou bien
A représente $-O-CH_2-CH_2-B-CH_2-CH_2-O-$ , et
B représente -S- ,

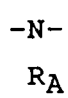 ou 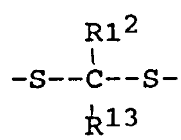 ,

et

$R_A$ représente un groupe alkyle en $C_4$ à $C_8$ ou phényle,
$R^{12}$ représente H, un groupe alkyle en $C_1$ à $C_8$ et,
$R^{13}$ représente H, un groupe alkyle en $C_1$ à $C_8$ ou phényle,
ou bien

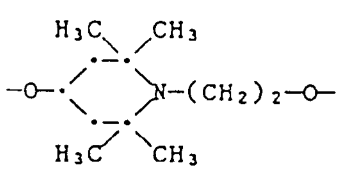 , 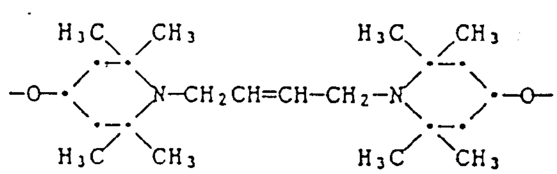 ou

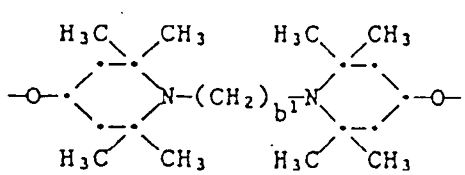

et,
$b^1$ vaut 2 à 6, ou bien
A représente

$-O-CH_2-CH=CH-CH_2-O-$ ,
$-O-CH_2-C=C-CH_2-O-$ ,

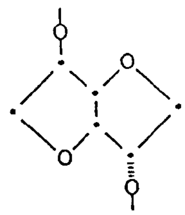 , 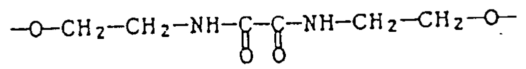 ;

ou

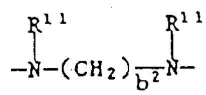

et $b^2$ vaut 0 à 6 , et
$R^{11}$ représente -H ou un groupe alkyle en $C_1$ à $C_8$.

5. Compositions selon la revendication 1, contenant au moins un composé de formule I, dans laquelle $R^1$ représente un groupe tert.-butyl,
$R^2$ représente -H, $-CH_3$ ou un groupe tert.- butyle, et
$R^3$ représente -H, et
n vaut 1 et, dans ce cas,
A représente un groupe $-OR^4$, et
R4 représente un groupe alkyle en $C_1$ à $C_{18}$.

6. Compositions selon la revendication 1, contenant au moins un composé de formule I, dans laquelle
$R^1$ représente un groupe tert.-butyle,
$R^2$ représente -H, $-CH_3$ ou un groupe tert.- butyle, et
$R^3$ représente -H, et
n vaut 2 et, dans ce cas ,
A représente $-O-c_xH_{2x}-O-$, et
x vaut 2 à 6, ou bien
A représente $-O-(CH_2-CH_2-O)_a-CH_2-CH_2-O-$ et,
a vaut 1, 2 ou 3 ou bien,
A représente $-O-CH_2-CH_2-B-CH_2-CH_2-O-$ et
B représente -S- ou

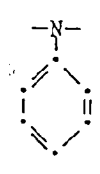 ,

ou bien
A représente

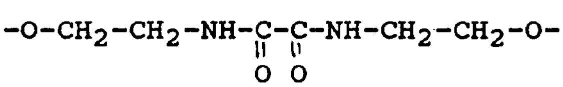

ou

A représente -NH-NH- .

**7.** Composition selon la revendication 1 contenant au moins un composé de formule I dans laquelle,
R[1] représente un groupe tertiobutyle,
R[2] représente -H, $-CH_3$ ou un groupe tert.- butyle,
R[3] représente -H,
n vaut 1 et, dans ce cas,
A représente $-OR^4$
R[4] représente un groupe cycloalkyle en $C_1$ à $C_{18}$, ou bien
n vaut 2 et, dans ce cas,
A représente $-O-C_xH_{2x}-O-$ et x vaut 2 à 6,
$-OCH_2CH_2-s-CH_2CH_2O-$ ou
$-O(CH_2CH_2O)_aCH_2CH_2-O-$ et
a vaut 1 à 4.

**8.** Composition selon la revendication 7, contenant au moins un composé de formule I, dans laquelle a vaut 1 ou 2.

**9.** Composition selon la revendication 1, contenant au moins un composé de formule I, dans laquelle
R[1] représente un groupe tert.-butyle,
R[2] représente -H, $-CH_3$, un groupe tert.-butyle
et
R[3] représente -H,
et
n vaut 3 et , dans ce cas,
A représente

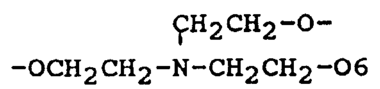 ,

ou,
n vaut 4, et dans ce cas,
A représente

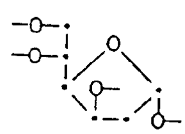

ou
n vaut 6 et, dans ce cas,
A représente

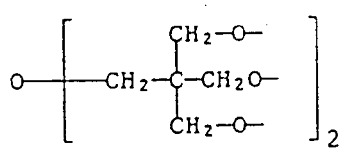 ,

**10.** Composition selon la revendication 1, contenant au moins un composé appartenant à la série des formules

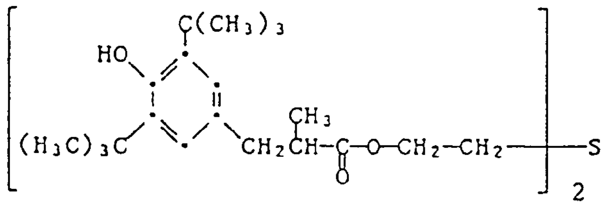,

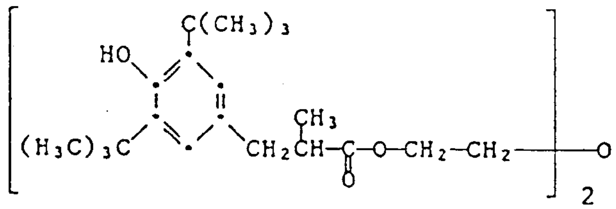,

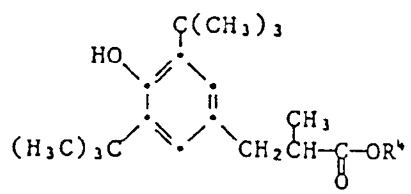,

dans lesquelles
$R^4$ représente un groupe alkyle en $C_1$ à $C_{18}$,

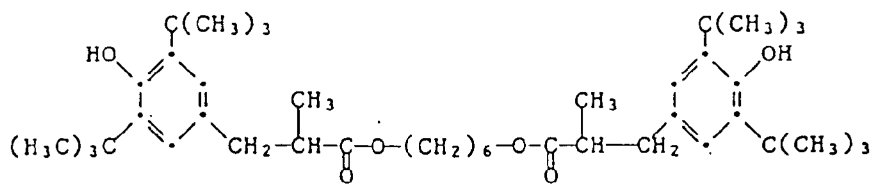 et

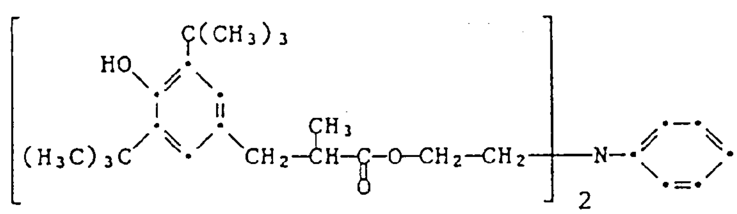

**11.** Composition selon la revendication 1, contenant
a) un liquide fonctionnel choisi parmi des lubrifiants, des liquides hydrauliques et des liquides pour le travail des métaux ou un polymère synthétique, et
(b) au moins un composé de formule I selon la revendication 1.

**12.** Composition selon la revendication 1, contrenant
(a) un lubrifiant choisi parmi les huiles minérales, les huiles synthétiques et un de leurs mélanges, et
(b) au moins un composé de formule I, selon la revendication 1.

**13.** Composition selon la revendication 1, contenant au moins un composé selon la revendication 10.

**14.** Composition selon la revendication 1, contenant :
(a) un polymère synthétique, et
(b) au moins un composé de formule I selon la revendication 1.

**15.** Composition selon la revendication 14, contenant :
(a) une polyoléfine, et
(b) au moins un composé de formule I selon la revendication 1.

**16.** Utilisation des composés de formule I selon la revendication 1, à titre d'anti-oxydants dans des matières organiques sensibles à une dégradation par oxydation, par voie thermique ou actinique.

**17.** Utilisation des composés de formule I selon la revendication 1 à titre d'anti-oxydants dans des lubrifiants.